(19) Europäisches Patentamt
European Patent Office
Office européen des brevets

(11) **EP 4 527 326 A1**

(12) **EUROPEAN PATENT APPLICATION**

(43) Date of publication:
**26.03.2025 Bulletin 2025/13**

(21) Application number: **24199491.2**

(22) Date of filing: **10.09.2024**

(51) International Patent Classification (IPC):
**A61B 18/02** (2006.01)  **A61B 18/00** (2006.01)
**A61B 1/015** (2006.01)  **A61B 90/00** (2016.01)

(52) Cooperative Patent Classification (CPC):
**A61B 18/02;** A61B 2018/00035; A61B 2018/00041;
A61B 2018/00577; A61B 2018/00642;
A61B 2018/00666; A61B 2018/00696;
A61B 2018/00702; A61B 2018/00744;
A61B 2018/00773; A61B 2018/00791;
A61B 2018/00863; A61B 2018/00898;
A61B 2018/0212; A61B 2018/0262;  (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **20.09.2023   US 202318471050**

(71) Applicant: **Varian Medical Systems, Inc.
Palo Alto, CA 94304 (US)**

(72) Inventors:
• **ZHANG, Hongxuan
Austin, 78750 (US)**
• **MEDEMA, Ryan
Georgetown, 78633 (US)**

(74) Representative: **Mathisen & Macara LLP
Charta House
30-38 Church Street
Staines-upon-Thames TW18 4EP (GB)**

(54) **APPARATUSES FOR CRYOABLATION SYSTEM DIAGNOSTICS AND PURGE**

(57)     A cryoablation apparatus includes a Dewar, a cryogen flow path fluidly coupled to the Dewar to supply cryogen to a cryoprobe, a first pressure sensor and a second pressure sensor positioned at a first portion of the cryogen flow path and at a second portion of the cryogen flow path, respectively, a first valve and a second valve each positioned along the cryogen flow path and each configured to operate in an open position and in a closed position, and a diagnostics control operatively coupled to the first pressure sensor, the second pressure sensor, the first valve, and the second valve. The diagnostics control is configured to determine whether a blockage or leak exists in the first portion and the second portion of the cryogen flow path. The apparatus also includes a gas assembly configured to supply a cryogen gas to purge the flow path.

FIG. 22

(52) Cooperative Patent Classification (CPC): (Cont.)
     A61B 2090/064; A61B 2090/0809

**Description**

FIELD

The present disclosure relates to apparatuses and methods for cryoablation system diagnostics and purge.

BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase Nitrogen, Argon, Helium, or the like) that may be passed through a probe that is in thermal contact with the target tissue. Heat from the tissue passes from the tissue, through the probe, and into the cryogen that removes heat from the targeted tissue. This removal of heat causes tissue to freeze, resulting in the destruction of the targeted tissue. When the tissue freezes, ice forms typically in an iceball. The iceball may be in the form a sphere, ellipsoid or other rounded shape. It is desirable to perform cryoablation treatments such that the target tissue is completely frozen and that the freezing of surrounding tissues and/or body structures is minimized.

Traditional or existing systems often include cryogen sources and delivery systems that operate to deliver the cryogen to the probe to create satisfactory and predictable temperatures at the target tissue. Existing systems, however, may include multiple separate systems, individual controls, independent measurement devices, and individual user interfaces that can lead to inefficiencies and treatments of reduced effectiveness. There exists a need, therefore, for improved apparatuses and systems for improved efficiency, lower treatment cost, and improved treatment effectiveness.

SUMMARY

This section provides a general summary of the disclosure, and is not a comprehensive disclosure of its full scope or all of its features.

In some embodiments of the present disclosure, a cryoablation apparatus may include a capacitance-based liquid level sensor positioned in the Dewar. The capacitance-based liquid level sensor may be coupled to a cryo-control that can determine a cryogen liquid level based on the capacitance of the liquid level sensor in the Dewar. The cryo-control may continuously determine a liquid level of the cryogen in the Dewar from the capacitance-based liquid level sensor. This real-time and/or continuous indication of the cryogen liquid level can be used to optimize the operation, performance and effectiveness of the cryoablation apparatus.

In some embodiments, a cryoablation apparatus may include a Dewar defining an internal volume to hold a cryogen and a capacitance-based cryogen liquid level sensor positioned in the Dewar configured to provide a cryogen liquid level of the cryogen in the Dewar. The cryoablation apparatus may also include a cryo-control configured to send a cryogen level warning if the cryogen liquid level is less than a predetermined level.

In one aspect, the capacitance-based cryogen liquid sensor may include a cryogen rod extending in the internal volume of the Dewar.

In another aspect, the cryogen rod may include a first capacitor surface and a second capacitor surface. The first capacitor surface may be positioned concentrically around the second capacitor surface and define an annular cavity therebetween.

In another aspect, the cryoablation apparatus may include a control circuit coupled to the capacitance-based cryogen liquid level sensor and the cryo-control. The cryo-control and the control circuit may be configured to continuously charge and discharge the capacitance-based cryogen liquid level sensor during a cryoablation cycle to continuously determine a cryogen liquid level in the Dewar.

In another aspect, the cryo-control may be further configured to determine the cryogen liquid level based on the capacitance of the capacitance based cryogen liquid level sensor and one or more operating conditions of the Dewar.

In another aspect, the one or more operating conditions of the Dewar may include a temperature and a pressure of the cryogen in the Dewar.

In another aspect, the one or more operating conditions of the Dewar may include a flow of the cryogen.

In another aspect, the capacitance-based liquid level sensor may include a cavity configured to supply cryogen from the Dewar to a cryoprobe.

In another aspect, the capacitance-based liquid level sensor may be configured to provide the cryogen liquid level for any level of cryogen along a length of the capacitance-based liquid level sensor.

In another aspect, the cryo-control may be configured to determine the cryogen liquid level based on a mapping of a capacitance of the capacitance-based liquid level sensor to a quantity of cryogen liquid in the Dewar.

In some embodiments of the present disclosure, a method of determining a cryogen liquid level is provided. The method may include obtaining a capacitance from a capacitance-based liquid level sensor in a Dewar and determining the cryogen liquid level based on the capacitance.

In one aspect, the step of obtaining the capacitance may include continuously charging and discharging the capacitance-based liquid level sensor during a cryoablation cycle.

In another aspect, the step of obtaining the capacitance may be performed while moving liquid cryogen through the capacitance-based liquid level sensor.

In another aspect, the step of determining the cryogen liquid level may be based on one or more operating conditions in the Dewar.

In another aspect, the one or more operating conditions in the Dewar may include a temperature and a pressure of the cryogen.

In another aspect, the one or more operating conditions in the Dewar may include a flow of the cryogen.

In another aspect, the method may include sending a cryogen level warning if the cryogen liquid level is less than a predetermined level.

In another aspect, the capacitance-based liquid level sensor may be implements as a cryogen rod extending in the Dewar.

In another aspect, the step of determining the cryogen liquid level may be performed using a trained machine learning model.

In some embodiments a cryo-control is provided. The cryo-control may include a processor and memory and the cryo-control may be configured to perform the methods of the present disclosure.

In some embodiments of the present disclosure, a cryoablation apparatus is provided that include a pressure control that can use a closed-loop feedback process to automatically monitor and adjust pressures in the Dewar and in the cryogen supply lines to provide efficient and effective delivery of the cryogen to one or more cryoprobes. The cryoablation apparatuses may include a heating assembly located in the Dewar and low pressure sensors in the Dewar to monitor a pressure of cryogen in the Dewar and activate and control the heating assembly to pressurize the Dewar to a desired low pressure condition. The cryoablation apparatus may also include a high pressure pump assembly that may be controlled and adjusted to provide a flow of high pressure cryogen to the one or more cryoprobes. The low pressure monitoring and control in the Dewar may be performed in parallel with the high pressure monitoring and control to provide the cryogen more efficiently and effectively that existing systems.

In some embodiments of the present disclosure, a cryoablation apparatus may include a Dewar defining an interior volume configured to retain a volume of cryogen and a supply tube extending into the interior volume of the Dewar. The cryoablation apparatus may also include a heating assembly positioned at a distal end of the supply tube and a pump assembly positioned proximate the heating assembly. The cryoablation apparatus may also include a lid assembly coupled to the Dewar at the proximate end of the supply tube opposite to the distal end of the supply tube.

In one aspect, the distal end of the supply tube may be positioned proximate a base of the Dewar.

In another aspect, the heating assembly may include a bubble barrier comprising at least one wall defining a heating boundary and a heater positioned inside the bubble barrier.

In another aspect, the distal end of the supply may be positioned at a top of the bubble barrier.

In another aspect, the apparatus may include at least one pressure sensor positioned inside the bubble barrier.

In another aspect, the heater may be configured to increase a pressure of the cryogen locally within the heating boundary to a pressure greater than a pressure of the cryogen outside the heating boundary.

In another aspect, the cryoablation apparatus may include a pressure control operably coupled to the heating assembly, the pump assembly, and the lid assembly. The pressure control may be configured to activate and deactivate the heater to maintain the cryogen supplied to the supply tube at a pressure within a first predetermined operating range.

In another aspect, the pressure control may be further configured to operate the pump assembly and the lid assembly to pressurize the cryogen at an outlet of a pump to a pressure within a second predetermined operating range.

In another aspect, the first predetermined operating range is a low pressure range and the second predetermined operating range is a high pressure range.

In another aspect, the cryoablation apparatus may include a valve assembly fluidly coupled to the interior volume of the Dewar. The pressure control may be operably coupled to the valve assembly and be further configured to open a valve of the valve assembly when the pressure of the cryogen in the Dewar is greater than an upper pressure level of the first predetermined operating range and to open the valve of the valve assembly when the pressure of the cryogen at the outlet of the pump is greater than an upper pressure level of the second predetermined operating range.

In some embodiments of the present disclosure, a method of pressuring cryogen includes obtaining Dewar pressure information characterizing a pressure of a cryogen in a Dewar and maintaining the pressure of the cryogen in the Dewar within a first pressure range using a heating assembly in the Dewar. The method may also include obtaining pump pressure information characterizing a pressure of the cryogen at an outlet of a pump and maintaining the pressure of the cryogen at the outlet of the pump within a second pressure range using the pump, wherein the step of maintaining the pressure of the cryogen in the Dewar and the step of maintaining the pressure of the cryogen at the outlet of the pump are performed simultaneously.

In another aspect, the first pressure range may be a low pressure range and the second pressure range may be a high pressure range.

In another aspect, the first pressure range may be a range of about 10 psi to about 15 psi.

In another aspect, the second pressure range may be a range of about 400 psi to about 600 psi.

In another aspect, the step of maintaining the pressure of the cryogen in the Dewar may be performed using the heating assembly that may include a bubble barrier comprising at least one wall defining a heating boundary and a heater positioned inside the bubble barrier.

In another aspect, the step of maintaining the pressure of the cryogen in the Dewar may include determining when the pressure of the cryogen in the Dewar is outside the first pressure range and modifying the operating parameters of a heater in Dewar.

In another aspect, the step of modifying the operating parameters of the heater may include determining a modification to the duty cycle delivered to the heater and the modification to the duty cycle may be determined using a trained machine learning model.

In another aspect, the step of maintaining the pressure of the cryogen at the outlet of the pump may include determining one or more modifications to the operating parameters of the pump.

In another aspect, the determining one or more modifications to the operating parameters of the pump may be determined using a trained machine learning model.

In some embodiments of the present disclosure, a pressure control is provided. The pressure control may include a processor and memory and the pressure control may be configured to perform any of the methods of the present disclosure.

In some embodiments of the present disclosure, a cryogen gas apparatus is provided. The cryogen gas apparatus may include a housing configured to retain a volume of liquid cryogen and a heater positioned in the housing and configured to heat the liquid cryogen to convert the liquid cryogen to cryogen gas. The cryogen gas apparatus may also include a flow conduit that includes a conduit heater. The flow conduit may be connected to the housing and configured to fluidly connect the housing to a cryogen flow path of a cryoablation system.

In another aspect, the cryogen gas apparatus may include a connector configured to fluidly connect the housing to a Dewar of a cryoablation system.

In another aspect, the housing may be positioned inside a Dewar of a cryoablation system.

In another aspect, the flow conduit may be positioned at a top of the housing to dispense cryogen gas from the housing to the cryogen flow path.

In another aspect, the cryogen gas apparatus may include a gas control coupled to the heater and the conduit heater. The gas control may be configured to activate the heater to achieve a predetermined pressure in the housing and active the conduit heater to achieve a predetermined temperature of cryogen gas.

In another aspect, the gas control is further configured to dispense the cryogen gas to the cryogen flow path to perform a purge of the cryogen flow path.

In some embodiments of the present disclosure, a cryoablation apparatus may include a Dewar and the cryogen gas apparatus positioned in the Dewar.

In some embodiments of the present disclosure a method of purging a cryogen flow path is provided. The method may include moving a portion of a volume of liquid cryogen from a Dewar to a housing of a cryogen gas apparatus, heating the portion of the volume of liquid cryogen to form cryogen gas in the housing, and moving the cryogen gas from the housing into the cryogen flow path to purge the cryogen flow path.

In another aspect, the housing defines an internal cavity inside the Dewar.

In another aspect, the housing may be positioned in the Dewar.

In accordance with a first aspect of the present invention, a cryoablation apparatus comprises a Dewar configured to retain a volume of cryogen, preferably nitrogen; a cryogen flow path fluidly coupled to the Dewar to supply the cryogen to a cryoprobe; a first pressure sensor and a second pressure sensor positioned at a first portion of the cryogen flow path and at a second portion of the cryogen flow path, respectively, a first valve and a second valve each positioned along the cryogen flow path, each configured to operate in an open position to allow the flow of cryogen and in a closed position to prevent the flow cryogen; and a diagnostics control operatively coupled to the first pressure sensor, the second pressure sensor, the first valve, and the second valve, the diagnostics control configured to: determine whether a blockage or leak exists in the first portion and the second portion of the cryogen flow path, optionally wherein the second portion of the cryogen flow path is positioned downstream of the first portion; and the diagnostics control is configured to determine whether a blockage or leak exists in the first portion before the second portion, wherein preferably the diagnostics control is further configured to cause a purge of the cryogen flow path to be performed when it determines a blockage exists.

In another aspect, the first portion and the second portion are positioned along a supply line of the cryogen flow path.

In another aspect, the cryoablation apparatus may include a return line fluidly coupled to the cryoprobe and to the supply line.

In another aspect, the cryoablation apparatus may also include a first connector valve fluidly connecting the supply line to the return line at a position upstream of the first valve.

In another aspect, the cryoablation apparatus may include a second connector valve fluidly connecting the supply line to the return line at a position downstream of the first valve and upstream of the second valve.

In another aspect, the return line may be fluidly coupled to vent allowing pressure in the return to be vented from the cryogen flow path.

In another aspect, the diagnostics control may determine whether a blockage or leak exists by comparing a pressure to a target pressure range and comparing a time to reach maximum pressure to target time range.

In another aspect, the diagnostics control may determine whether a blockage or leak exists using a trained machine learning model.

In another aspect, the diagnostics control may be configured to operate the first valve in the open position and the second valve in the closed position to determine whether a blockage or leak exists in the first portion before the diagnostics control operates the first valve in the open position and the second valve in the open position to determine whether a blockage or leak exists in the second portion.

The cryoablation apparatus may further comprise a housing configured to retain a second volume of liquid cryogen; a heater positioned in the housing and configured to heat the liquid cryogen to convert the liquid cryogen to cryogen gas; and a flow conduit comprising a conduit heater, the flow conduit fluidly connected to the housing and configured to fluidly connect the housing to a cryogen flow.

Preferably, the cryoablation apparatus further comprises a connector configured to fluidly connect the housing to a Dewar, optionally wherein the housing is positioned inside the Dewar.

Preferably, the flow conduit is positioned at a top of the housing to dispense cryogen gas from the housing to the cryogen flow path.

Preferably, the cryoablation apparatus further comprises a gas control coupled to the heater and the conduit heater, the gas control configured to: activate the heater to achieve a predetermined pressure in the housing; and active the conduit heater to achieve a predetermined temperature of cryogen gas.

Preferably, the gas control is further configured to dispense the cryogen gas to the cryogen flow path to perform a purge of the cryogen flow path.

In another aspect of the present invention, a method of diagnosing cryogen flow in a cryogen flow path comprises obtaining pressure information for a pressure of cryogen in a first portion of the cryogen flow path; comparing the pressure information to a target pressure range; and comparing a time to reach a maximum pressure in the first portion of the cryogen flow path to a target time range; and sending a flow alert if a flow issue is detected based on the pressure information and the time to reach the maximum pressure, the method optionally further comprising opening a first valve to allow cryogen to flow to the first portion of the cryogen flow path.

In another aspect, the method may further include opening a second valve to allow cryogen to flow to a second portion of the cryogen flow path, wherein the second portion of the cryogen flow path is downstream of the first portion, obtaining pressure information for a pressure of cryogen in the second portion of the cryogen flow path, comparing the pressure information to a second target pressure range, comparing a second time to reach a maximum pressure in the second portion of the cryogen flow path to a second target time range, and sending a flow alert if a flow issue is detected based on the pressure information in the second portion of the cryogen flow path and the second time to reach the maximum pressure.

In another aspect, the method may include venting pressure from the cryogen flow path if a flow issue is detected.

In another aspect, pressure is vented by opening a connector valve that fluidly connects the first portion of the cryogen flow path to a return line of the cryogen flow path.

Further areas of applicability will become apparent from the description provided herein. The description and specific examples in this summary are intended for purposes of illustration only and are not intended to limit the scope of the present disclosure.

DRAWINGS

The drawings described herein are for illustrative purposes only of selected embodiments and not all possible implementations, and are not intended to limit the scope of the present disclosure.

FIG. 1 is a diagram illustrating an example cryoablation apparatus in accordance with some embodiments of the present disclosure.

FIG. 2 is a diagram illustrating an example cryogen source with a cryogen capacitance level sensor in accordance with some embodiments of the present disclosure.

FIG. 3A is a cross-sectional view of an example rod sensor that may be used in the cryogen source of FIG. 2.

FIG. 3B is a cross-sectional view of another example rod sensor that may be used in the cryogen source of FIG. 2.

FIG. 4 is a diagram illustrating further aspects and an example control circuit that may be used with the example cryogen source and cryogen capacitance level sensor of FIG. 2.

FIG. 5 is a diagram illustrating aspects of signals and related processing that may occur during operation of the cryogen capacitance level sensors of the present disclosure.

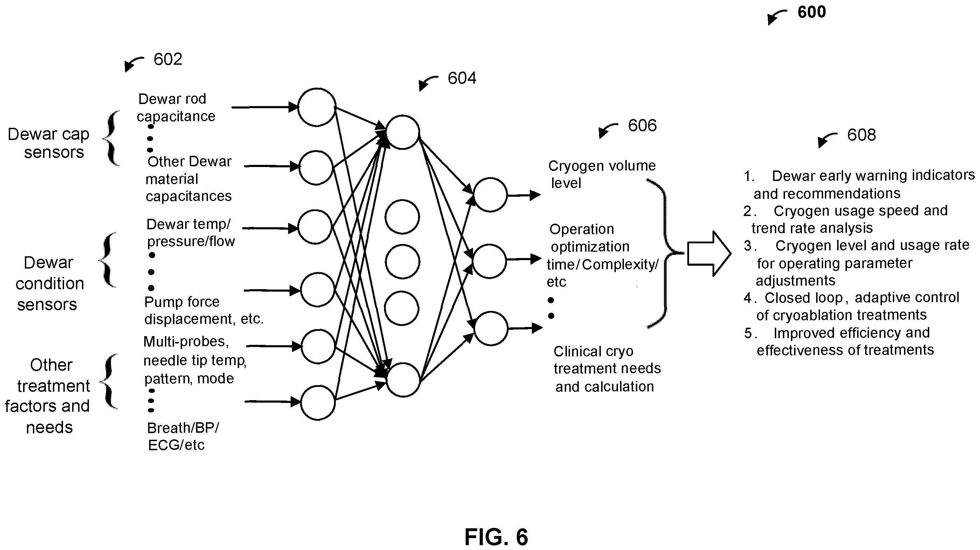

FIG. 6 is a diagram illustrating an example cryoablation machine learning model that may be trained and used to operate cryoablation apparatuses of the present disclosure.

FIG. 7 is a flow chart illustrating an example method of determining a cryogen level in a cryoablation apparatus in accordance with some embodiments of the present disclosure.

FIG. 8 is a flow chart illustrating another example method of determining a cryogen level in a cryoablation apparatus in accordance with some embodiments of the present disclosure.

FIG. 9 is a diagram illustrating an example computing device that can be used in one or more cryoablation systems of the present disclosure.

FIG. 10 is an illustration of an example cryoablation apparatus in accordance with some embodiments of the present disclosure.

FIG. 11 is an illustration of an example heating assembly that may be used in the cryoablation apparatus of FIG. 10.

FIG. 12 is an example cryoablation system that may include the cryoablation apparatus of FIG. 10 in accordance with some embodiments of the present disclosure.

FIG. 13 is another example cryoablation system in accordance with some embodiments of the present disclosure.

FIG. 14 is a diagram illustrating aspects of an example model that may be used in connection with some cryoablation systems of the present disclosure.

FIG. 15 is an example method of maintaining a pressure condition in accordance with some embodiments of the present disclosure.

FIG. 16 is another example method of maintaining a pressure condition in accordance with some embodiments of the present disclosure.

FIG. 17 is another example method of maintaining a pressure condition in accordance with some embodiments of the present disclosure.

FIG. 18 is an illustration of an example cryoablation apparatus that may include a cryogen gas assembly in accordance with some embodiments of the present disclosure.

FIG. 19 is an illustration of an example cryogen gas assembly in accordance with some embodiments of the present disclosure.

FIG. 20 is an example method of performing a purge process in accordance with some examples of the present disclosure.

FIG. 21 is another example method of performing a purge process in accordance with some examples of the present disclosure.

FIG. 22 is an illustration of an example cryoablation system in accordance with some embodiments of the present disclosure.

FIG. 23 is a chart showing example pressure outputs that may be observed in various circumstances using the cryoablation system of FIG. 22.

FIG. 24 is a diagram showing aspects of an example diagnostics model in accordance with some embodiments of the present disclosure.

FIG. 25 is an example diagnostics method in accordance with some embodiments of the present disclosure.

FIG. 26 is another example diagnostics method in accordance with some embodiments of the present disclosure.

Corresponding reference numerals indicate corresponding parts throughout the several views of the drawings.

DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Multiple different embodiments are described below that describe various functionality, apparatuses, systems and methods of a cryoablation system. Some functionality and apparatuses are described separately but it should be appreciated that the functionality and related elements may be included the same apparatuses to provide a cryoablation system with all the functionality and elements as described below.

The embodiments and aspects thereof that are further described below may be applied to various apparatuses and methods used for cryoablation treatments. While the disclosure below may be applied to various cryogens such as liquid nitrogen, liquid argon, liquid helium, liquid hydrogen, and liquid oxygen, for example, the present disclosure is particulaly applicable to liquid nitrogen. Liquid nitrogen presents challenges because it can be extremely difficult to work with in a cryogenic system and environment. The operating temperatures, pressures, and other conditions may stress common materials and present challenges that may not exist with other cryogens. Traditional and existing methods and appratuses may not work with the challenging properties of liquid nitrogen. In addition to the low temperatures, liquid nitrogen expands dramatically when it transitions from liquid to gas. Liquid nitrogen may expand 700 times in volume in this transition. Vapor lock is another challenge faced with liquid nitrogen systems. During such conditions, a gas pocket may become trapped in the cryogen flow path blocking the flow of liquid nitrogen. The apparatuses and methods of the present disclosure present improvement over existing systems that address and manage the challenges of working with liquid nitrogen.

## CAPACITANCE-BASED LIQUID LEVEL SENSOR

In some embodiments of the present disclosure, a cryoablation apparatus is provided that may include a cryogen capacitance level sensor that may operate to determine a level of cryogen in a cryogen source, such as a Dewar or other cryogen container. The cryogen capacitance level sensor may be positioned in the Dewar or be formed as part of the Dewar to allow a real-time cryogen level to be determined. Existing or traditional cryoablation systems may have sensors to determine discrete cryogen levels but such systems do not operate to deliver a real-time cryogen level and/or at poor at determining rates of cryogen use. Furthermore, existing systems are poor at anticipating when a cryogen source may need to be refilled and/or determine efficiencies that may be obtained by adjustment to operating parameters of the cryoablation apparatus.

The cryoablation apparatuses of the present disclosure and the included cryogen capacitance level sensors may be used to obtain many advantages over the short comings of existing systems and apparatuses. The real-time cryogen level that may be determined using the apparatuses of the present disclosure enable an actual cryogen level to be determined. The real-time cryogen level may be used to determine, for example, when a Dewar needs to be refilled.

Furthermore, the cryogen level may be used to anticipate when a Dewar will need to be refilled. The cryogen apparatuses of the present disclosure can be used to more efficiently use cryogen during cryoablation treatments to increase efficiency, reduce cost and to increase the likelihood of a successful and effective treatment.

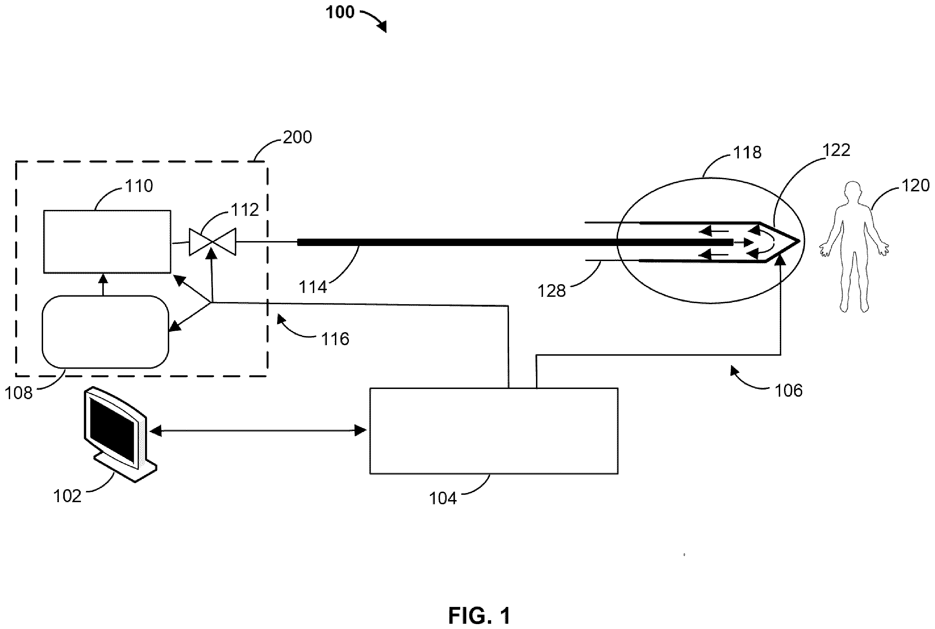

Referring now to FIG. 1, an example cryoablation system 100 is shown. The cryoablation system 100 may include a cryoablation computing device 102, a cryo-control 104, one or more probe information channels 106, a pump 108, a cryogen source 110, a cryogen valve 112, a cryogen supply 114, and a cryoprobe 128. The pump 108, the cryogen source 110, and the cryogen valve 112 may together form a cryogen apparatus 200. The cryogen apparatus 200 with the cryogen supply 114, and the cryoprobe 128 may operate to deliver a cryogen from the cryogen source 110 to the cryoprobe 128 to perform a cryoablation treatment. The cryogen (e.g., liquid nitrogen, argon, helium, or other) can be stored in the cryogen source 110, such as a Dewar or other suitable container, and then delivered to the cryoprobe 128 via the cryogen supply 114. The cryogen may exit the cryogen supply 114 at a tip 122 of the cryoprobe 128 and cool the tip 122 of the cryoprobe 128 to a temperature at which the target tissue of a patient 120 begins to freeze forming an iceball.

The cryoprobe 128 can be positioned at or near a target tissue (e.g., a tumor) in the patient 120. In this manner, the target tissue can be frozen destroying the target tissue. One or more freezing cycles can be performed in order to destroy the target tissue. An iceball 118 may form at the target tissue in the patient 120 during the freezing cycle. It is desirable to control and form the iceball 118 in a predetermined manner so the iceball 118 forms to a desired size, shape and rate so that the target tissue is frozen in the iceball 118 for a desired period of time. It is also desirable to form the iceball 118 with the desired size, shape and rate so that healthy tissue or body structures near the target tissue are not harmed by the freezing cycle. It can be desirable, for example, to limit a size of the iceball 118 so that it does not form and freeze healthy tissue.

A treatment plan can be determined prior to the performance of the cryoablation treatment. The treatment plan can detail and/or describe the various steps of the process and various aspects of the treatment such as the types of equipment to be used, a positioning of the cryoprobe, temperatures of the cryoprobe, duration of freezing (and thaw cycles) as well as a quantity of cycles. The treatment plan may also include a size, location, shape, growth rate and duration of an iceball. The treatment plan may be determined by a medical professional and/or by others. In some examples, the cryoablation computing device 102 may determine or recommend a treatment plan after health, patient, and other information is input into the cryoablation computing device 102 or such information is retrieved or otherwise obtained by the cryoablation computing device 102. The cryoablation computing device 102 may any suitable computing device such as a workstation, computer, laptop, tablet, server or the like.

As further shown, the cryoablation system 100 may include the cryo-control 104 that may be coupled to the cryoablation computing device 102, to the cryoprobe 128 and to the cryoablation apparatus 200. The cryo-control 104 may be any suitable controller, PLC, data acquisition unit, control unit or the like that can perform the operations described herein. In some examples, the cryo-control 104, the computing device 102, and/or aspects of the cryoablation apparatus 200 may be integrated into a cryoablation console. The cryoablation console may be sized to allow it to be easily moved and positioned in a treatment room near to the patient 120.

The cryo-control 104 may operate to obtain information regarding the operating conditions of the cryoablation apparatus 200 and/or the cryoprobe 128. One type of information that the cryo-control 104 may obtain is a cryogen liquid level in the cryogen source 110. The cryogen may be vaporized into cryogen gas to pressurize the cryogen source 110. The increased pressure in the cryogen source 110 may allow the liquid cryogen to be pumped to the cryoprobe 128 at extremely low temperatures so that the cryoprobe 128 can be cooled to form the iceball 118 at the target tissue. The pressure in the cryogen source 110 may be at critical pressure or near-critical pressure of the cryogen. As will be further described below, the cryoablation apparatus 200 may include a liquid level sensor that may operate to determine a liquid level of the cryogen in the cryogen source 110. When the cryogen in the cryogen source 110 has been depleted and/or when the cryogen has been vaporized to an extent that the cryogen in the cryogen source 110 is primarily in the form of cryogen gas, the cryogen source 110 may need to be refilled with liquid cryogen. The ablation process may need to be suspended when the cryogen source 110 is refilled. The cryo-control 104 may be coupled to the inlet valve 112 and the pump 108 to control the flow of cryogen to the cryoprobe 128.

For example, a cryogen Dewar may hold about 25 to 50 liters of liquid nitrogen. Such an example Dewar may be used to perform multiple cryoablation treatments in a range of about 5 treatments to about 8 treatments. The Dewar may be refilled when the Dewar reaches a predetermined liquid level. The predetermined liquid level may correspond to a suitable factor of safety to allow at least one cryoablation treatment to be performed. In one example, the predetermined liquid level may be about 10% of the total volume of the Dewar. In other examples, the predetermined liquid level may correspond to other amounts of liquid cryogen.

The cryo-control 104 may be coupled to the cryogen liquid sensor in the cryogen source 110. The cryo-control 104 may obtain liquid level information from the cryogen liquid sensor. The cryo-control 104 may then determine when the cryogen source 110 needs to be refilled and may alert an operator of the status of the liquid level in the cryogen source 110.

The cryo-control 104 may also operate to obtain other operating information of the cryogen apparatus 200 and/or the cryoprobe 128. Such operating information may include pump operating characteristics, cryogen flow characteristics, pressures, temperatures, and other information. The cryo-control 104 may provide the information to the cryoablation computing device 102. The cryoablation computing device 102 may then perform various operations to determine characteristics of the ice being formed during a cryoablation treatment, performance of the cryoablation apparatus 200, and/or characteristics of the cryogen source 110.

The cryoablation computing device 102 may take action to adjust, change, modify or otherwise control the one or more operating parameters of the cryoablation system 100. In some examples, the cryoablation computing device 102 may adjust the flow of the cryogen provided from the cryogen source 110 to the cryoprobe 128. The cryoablation computing device 102 may be coupled to the pump 108. The pump 108 can be an adjustable, programmable, or otherwise controllable pump. The pump 108 may allow for the flow rate, flow volume, flow speed, pressure or other characteristic of the flow of cryogen to be modified, controlled or customized. In some examples, the pump 108 can operate to deliver the cryogen to the cryoprobe 128 in a pulsed manner using pulse width modulation (PWM). In such examples, the pump 108 can be controlled to deliver a flow of cryogen at a desired frequency, pulse width, pulse amplitude or other desired flow characteristic.

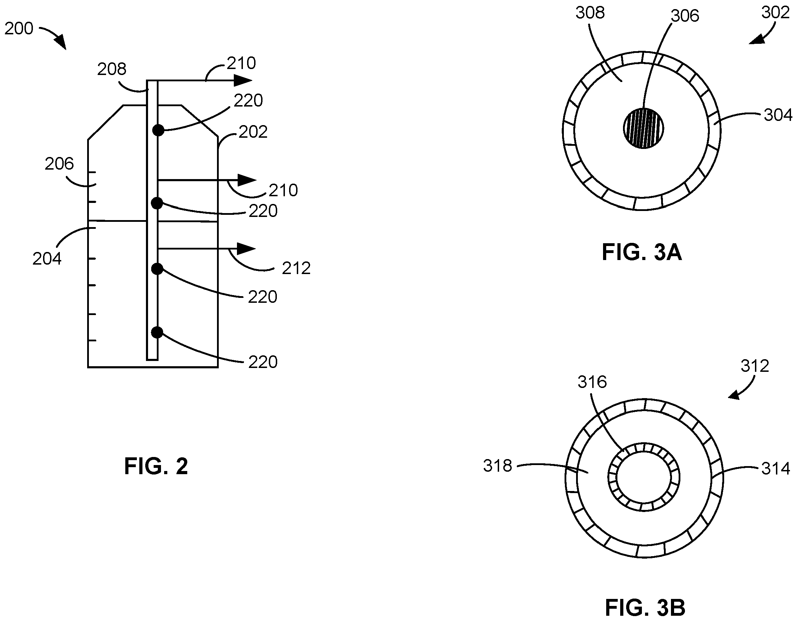

Referring now to FIG. 2, an example cryoablation apparatus 200 is shown. The cryoablation apparatus 200 may be part of the cryoablation system 100 previously described. The other elements of the cryoablation system 100 are not depicted in FIG. 2 but it should be appreciated that the elements of FIG. 1 or other cryoablation systems may be included in other examples.

The cryoablation apparatus 200 may include a Dewar 202, and a cryogen rod 208. The Dewar 202 may be a suitable housing to enclose a volume of cryogen, such as liquid nitrogen. As previously described, the cryogen may separate into liquid cryogen 204 and cryogen gas 206 when the cryoablation apparatus 200 is operated to deliver the pressurized liquid cryogen to the cryoprobe via the cryogen supply 210.

The cryogen rod 208 may operate as both a cryogen liquid level sensor and as a conduit for liquid cryogen. While not shown in FIG. 2, the pump 108 may be positioned at or near an end of the cryogen rod 208 and move liquid cryogen 204 from a base of the Dewar 202 to the cryogen supply 210. The cryogen rod 208 may form a cryogen liquid level capacitance sensor. A measured or determined capacitance of the cryogen rod 208 may be used to determine a liquid level of the liquid cryogen 204 in the Dewar 202.

The cryo-control 104 may be coupled to the cryogen rod 208 by a first control line 210 and a second control line 212. In other examples, the cryo-control 104 may be coupled with more than two control lines 210 or 212. The cryogen rod 208 may include a first capacitor surface and a second capacitor surface that are separated by a dielectric, such as the cryogen in either liquid or gaseous phase. The cryo-control 104 may operate to alternately charge and discharge the first capacitor surface and the second capacitor surface of the cryogen rod 208. The cryo-control 104 may also measure various aspects of the charge and discharge process such as measuring current and voltage. The cryo-control 104 and/or the cryoablation computing device 102 may determine a liquid level of the liquid cryogen 204 in the Dewar by determining the capacitance of the cryogen rod 208.

The cryo-control 104 and/or the cryoablation computing device 102 may determine the level of the liquid cryogen 204 since the overall capacitance (C) of the cryogen rod 208 is a function of the capacitance of the portion of the cryogen rod covered by the liquid cryogen ($C_1$) and the capacitance of the portion of the cryogen rod covered by the gaseous cryogen ($C_2$). The cryogen functions as the capacitor's dielectric that is positioned between the first capacitor surface and the second capacitor surface of the cryogen rod 208. Since the cryogen is in a different phase, the capacitance of the cryogen rod 208 differs between these two different portions. The cryo-control 104 and/or the cryoablation computing device 102 may use this difference to determine where the level of liquid cryogen 204 is positioned in the Dewar 202.

The relationship between the overall capacitance (C), the capacitance at the gaseous portion ($C_1$), and the capacitance at the liquid portion ($C_2$) can be described by equation 1: $C = C_1 + C_2$. In addition, the capacitance (C) may be characterized by equation 2: $C = \varepsilon_r * \varepsilon_0 * A/D$, where $\varepsilon_r$, $\varepsilon_0$ are ratios of the liquid cryogen material contribution to the capacitance, A is the equivalent area of the capacitance rod surfaces/plates, and D is the equivalent distance between the capacitance rod surfaces/plates. The overall capacitance that can be measured is the integrated capacitance and combination value of the two portion capacitance, $C_1$ and $C_2$, which corresponds to the liquid nitrogen portion capacitance plus the gas nitrogen portion capacitance (for systems using nitrogen as the cryogen). By using overall capacitance value, the user and the software and computing device can determine the ratio between $C_1$ and $C_2$ which can be directly related to the material ratio between liquid cryogen vs. gaseous nitrogen. Some experimental or clinical data may be used to determine the relationship between the liquid level in the Dewar and capacitances. For example, an overall capacitance value may be measured when the Dewar is full of gaseous cryogen and when it is full of liquid cryogen. The system may be calibrated to determine the relationship between overall capacitance and the liquid level in the Dewar by measurement of the overall capacitance. The computing device may then be configured to calculate or determine the ratio of $C_1$ to $C_2$, and then determine the level of the liquid nitrogen in the Dewar. For a fixed size Dewar, the capacitance curve (i.e., the relationship between overall capacitance and the liquid level) may be determined prior to operation of the system to link the overall capacitance to the liquid level of the cryogen.

In some examples, the capacitance of the cryogen rod 208 may be mapped for varying ratios and/or varying levels of liquid cryogen and gaseous cryogen using experimental and/or operational data collected during operation of the cryoablation apparatus 200. Such information may be obtained and stored in a database or other repository that may be accessed by the cryo-control 104 and/or the cryoablation computing device 102. In other examples, algorithms, models or other suitable relationships may be determined using such experimental and/or operational data to provide accurate mapping between levels of the liquid cryogen and the capacitance of the cryogen rod 208.

As can be appreciated, other information may be obtained by the cryo-control 104 and/or the cryoablation computing device 102 that may be used to determine the capacitance of the cryogen rod 208. Other information may affect and/or influence the capacitance of the cryogen rod 208. Information such as the temperature and/or pressure of the cryogen in the Dewar may be obtained by the cryo-control 104 and/or the cryoablation computing device 102 from the sensors 220 that may be positioned on the cryogen rod 208. While not shown, sensors may be positioned at other locations in the Dewar. The sensors 220 may include temperature sensors such as thermocouples, thermistors or the like. The sensors 220 may include pressure transducers, thin-film pressure sensors, piezoelectric pressure sensors or the like. The temperature and/or pressure information may be used to, for example, to calibrate, adjust, and/or build highly accurate mapping between the capacitance of the cryogen rod 208 and the liquid level of the liquid cryogen in the Dewar 202. Such accuracy may provide accuracy within $\pm$ 0.5% for the liquid level of the liquid cryogen in the Dewar 202. For capacitance measurement and calculations, there are several factors which may contribute to the capacitance, such as material inside the capacitor, displacement and size of the capacitors, and others. Some other factors may also be able to change the capacitance, such as temperature and pressure around the capacitor. For example, higher pressures of gaseous cryogen will result in high density of the gas which may increase the capacitance. Temperatures may also cause changes to the capacitance. The sensors used in apparatus may be various suitable mechanical transducers which are able to measure the gaseous cryogen pressure, such as a microelectromechanical (MEM) gas pressure sensor. The temperature sensors may be a thermistor or thermocouple, such as type T, which may have an accuracy can be +/-3° C. for the temperature sensor or an Omega PR-10E-3-100-1/8-6 can be used in the apparatus.

As stated above, the cryogen rod 208 may include a first structure that operates as the first capacitor surface and a second structure that operates as the second capacitor surface of the capacitor. The cryogen rod 208 may have various suitable structures to perform this function. As shown in FIG. 3A, an example cryogen rod 302 is shown. In this example, the cryogen rod 302 may formed from an outer tube 304 that is separated by a gap 308 from an inner cylinder 306. The inner cylinder 306 and the outer tube 304 may be concentrically aligned along a common axis with the inner cylinder 306 positioned at a center of the outer tube 304. The gap 308, in this example, may form an annular volume that extends longitudinally along the cryogen rod 302. The cryogen may be moved through the gap 308 when the liquid cryogen is pumped from the Dewar 202 to the cryoprobe 128. In this example, the inner cylinder 306 may form the first capacitor surface and the outer tube 304 may form the second capacitor surface.

Turning now to FIG. 3B, another example cryogen rod 312 is shown. In this example, the cryogen rod 312 includes an outer tube 314 and an inner tube 316 separated by the gap 318. In this example, the outer tube 314 and the inner tube 316 may be axially aligned similarly to the cryogen rod 302 previously described. In this example, the inner tube 316 may form the first capacitor surface and the outer tube 314 may form the second capacitor surface.

In other examples, the Dewar 202 may include other structures that may be used as a capacitance liquid level sensor. The Dewar 202 may include a pair of elongated plates that extend from a top to a bottom of the Dewar. In another example, an inner surface or shell of the Dewar may be used as a first capacitor surface and the outer surface of the cryogen rod 208 may be used as the second capacitor surface. In still other examples, other surfaces of other tubing, insulation, heaters or the like may be used to form one or more capacitance plates in the Dewar 202.

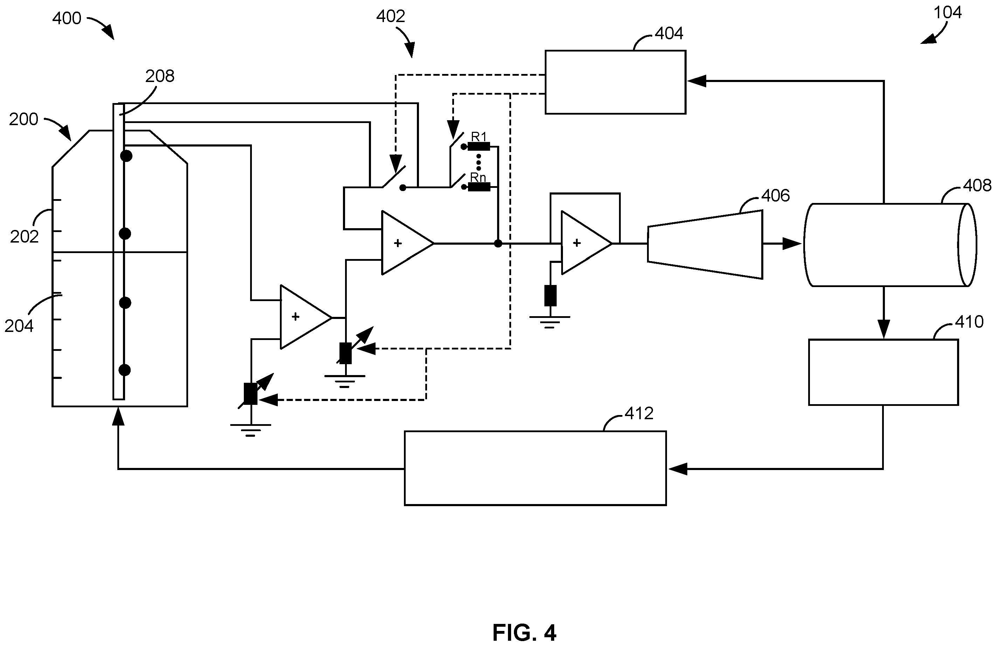

Turning now to FIG. 4, another example cryoablation apparatus 400 is shown. The cryoablation apparatus 400 may operate as previously described using the capacitance of the cryogen rod 208 to determine a liquid level of the liquid cryogen 204 in the Dewar 202. In this example, various aspects of a control circuit 402 and the cryo-control 104 are shown. The control circuit 402 may include one or more operational amplifiers, switches, resistors, and control wires that couple the cryo-control 104 to the cryogen rod 208 and allow the cryo-control to charge and discharge the cryogen rod 208 and determine a capacitance of the cryogen rod 208.

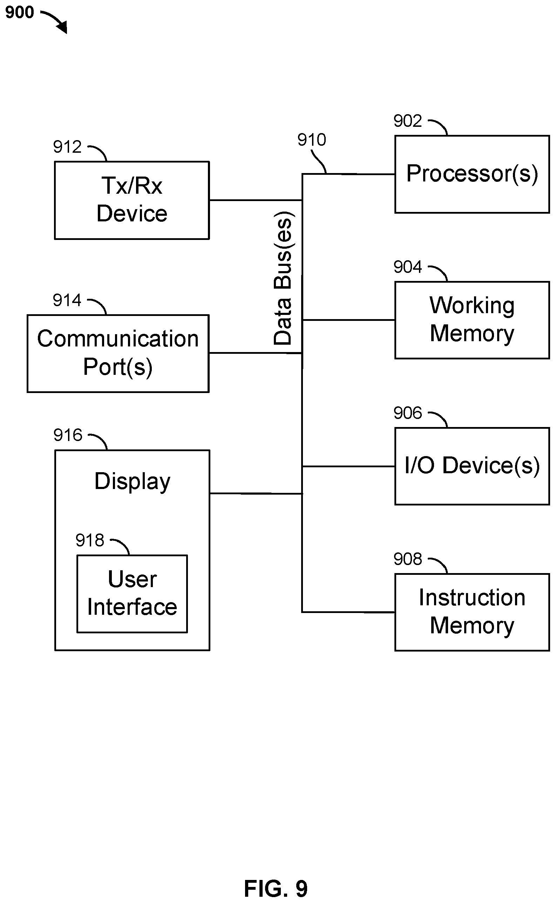

The control circuit 402 may be coupled to the cryo-control 104. The cryo-control 104, in this example, may include a capacitance module 404, a central control and communication module 408, a source level indicator module 410 and an operational feedback module 412. Each of the modules may be implemented as a set of executable instructions that perform the functions as described below. Each of the modules may also include individual processors, memory or other elements as shown in FIG. 9 to implement the functionality. In other examples, the modules may share hardware components (such as those described in FIG. 9).

The capacitance module 404 may be coupled to the switches, variable resistors and the like of the control circuit 402 to allow the cryogen rod 208 be charged, discharged and various parameters be measured such as current and voltage. Such charging, discharging, and measurements may be collected in a predetermined frequency or cadence to allow the capacitance to measured continuously or semi-continuously during operation of the cryoablation apparatus 400. The capacitance module 404 may be coupled to the central control and communication module 408. The central control and communication module 408 may store the information provided by the capacitance module 404 and/or may provide the information to other elements of the cryoablation apparatus 400.

The central control and communication module 408 may also be coupled to the analogue-to-digital (A2D) converter 406. The A2D converter 406 may provide current and/or voltage information to the central control and communication module 408 in a digital signal for further use. The digital signal may also be conditioned, filtered, and/or processed to increase the signal-to-noise ratio.

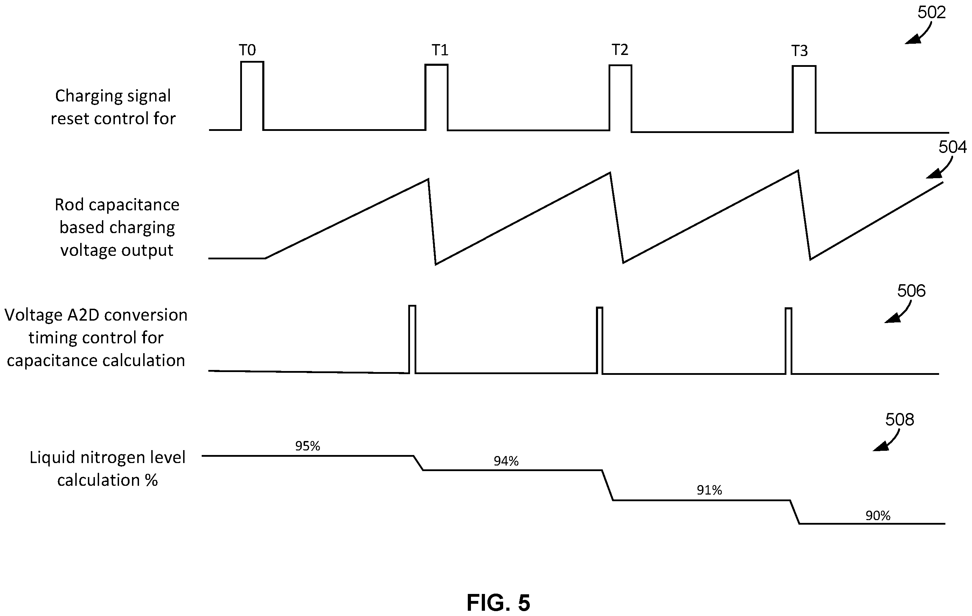

The cryo-control 104 may also include the source level indicator module 410. The source level indicator module 410 may operate to determine a liquid level of the Dewar 202. The source level indicator module 410 may use algorithms, relationships, mappings, machine learning models and the like to determine a liquid level of cryogen liquid in the Dewar 202. The source level indicator module 410 may include or be coupled to databases, servers, or other sources that it may access to determine the liquid level, for example. In some examples, the source level indicator module 410 may determine a liquid level by using the relationships shown in equations 1 and 2. In other examples, a machine learning model may be used such as that described below with reference to FIG. 5. The source level indicator module 410 may compare the liquid level to the predetermined liquid level threshold previously described. The source level indicator module 410 may send an indication, message, or other alert to user to indicate a liquid level. The liquid level may me expressed as a percentage of the total volume of the Dewar 202, for example.

The cryo-control 104 may also include the operational feedback module 412. The operational feedback module 412 may obtain the liquid level information from the source level indicator module 410. The operational feedback module 412 may also obtain pressure, temperature, flow rate, flow volume, and other operational information from various sensors or other information sources. The operational feedback module 412 may be coupled to the Dewar 202 or other elements in the cryogen source. The operational feedback module 412 may control or cause changes to the operating conditions of the cryoablation apparatus 200. The operational feedback module 412 may initiate, change, or stop of the flow of cryogen, for example. The operational feedback module 412 may change the operating conditions to improve the efficiency of the cryoablation apparatus, for example.

Referring now to FIG. 5, example operating profiles of the cryoablation apparatus 200 are shown. The charging signal 502 shows an example signal that may be delivered to the cryogen rod 208. The cryo-control 104 may deliver the charging signal 502 to the cryogen rod 208 via the control circuit 402. The charging signal 502 may deliver a charging current to the capacitor surfaces of the cryogen rod 208 to charge the capacitor surfaces. The charging voltage signal 504 shows an example charging voltage output to the cryogen rod 208. As can be seen, the voltage signal 504 shows that the voltage increases between the capacitor surfaces of the cryogen rod. At a predetermined frequency, the capacitor surfaces of the cryogen charge and the discharge repeatedly.

The voltage signal 504 may be converted to a digital signal 506. The digital signal 506 may be used to determine a capacitance and, in turn, a liquid level as previously described. The liquid level signal 508 may be determined from the digital signal 506. The liquid level signal 508 may correspond to a cryogen liquid level in the Dewar 202. The liquid level signal 508 may indicate a decrease in liquid level in the Dewar during operation of the cryoablation apparatus 200.

A machine learning model may be used to determine relationships between the operational characteristics of the cryoablation apparatus 200 and the performance of the cryoablation apparatus 200. The machine learning model 600, for example, may be used to determine various performance aspects 606 of the cryoablation apparatus 200. The machine learning model 600 may be trained, in some examples, using experimental, laboratory, or historical data from simulated or actual cryoablation treatments. The machine learning model may include inputs 602 that may include various operational characteristics of the cryoablation apparatus 200. The inputs 602 may include, for example, the capacitance of the cryogen rod 208. In other examples, other capacitances may also or alternatively be used as inputs 602. The inputs 602 may also include other operational information such as temperatures, pressures, flows, pump force, pump displacement and the like. The inputs 602 may also include various characteristics of the cryoablation apparatus 200. Such characteristics may include a number, type, temperature, pattern, or mode of the ablation probes. The inputs 602 may also include patient characteristics such a blood pressure, respiration, heart rate, and the like.

The inputs 602 may then be processes by one or more layers 604 of the machine learning model. The layers 604 may include algorithms, formulas, target functions or the like that process the data in the inputs 602 to determine relationships, patterns, or other information from the inputs 602. The layers 604 may use linear regression, decision trees, random forest models, or the like to process the data from the inputs 602. Various proprietary or open source models may be used to process the inputs 602.

In the example shown, the machine learning model 600 may use deep learning that utilizes one or more hidden layers 604 to determine relationships between the inputs 602. The machine learning model 600 may be used to determine the outputs 606 such as the liquid level of cryogen. The machine learning model may also be used to determine optimized operational settings or parameters that can be used in various circumstances or for particular treatments. The outputs 606 may then be used to determine various operational settings or actions 608. The operational settings or actions 608 may include early warning indicators for cryogen liquid levels. The operational settings or action 608 may also include recommended cryogen usage. The operational settings or actions 608 may be used to improve the efficiency and effectiveness of the cryoablation treatments.

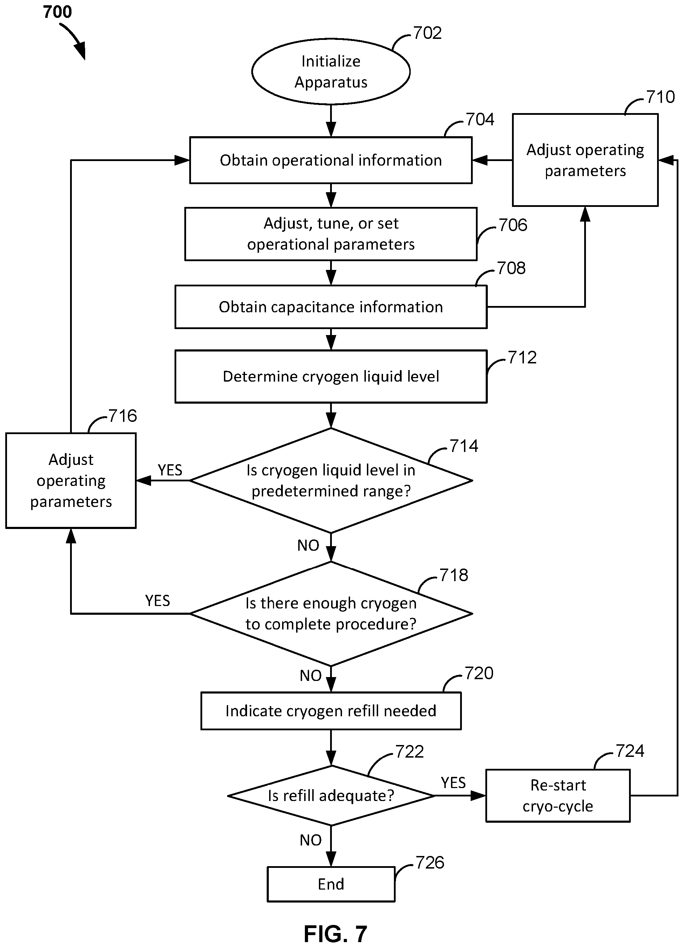

Turning now to FIG. 7, an example method 700 of operating a cryoablation apparatus is shown. The method 700 may be performed using one of the apparatuses described in the present disclosure. In other examples, the method 700 may be performed by other apparatuses. For illustration purposes, the method 700 is described below with reference to cryoablation system 100 and/or cryoablation apparatus 200. It should be appreciated, however, that the method 700 may be performed using other apparatuses or variations of system 100 and apparatus 200.

The method 700 may begin at step 702. At step 702, the cryo-control 104 may start or initialize the cryoablation apparatus 200. Such initialization or start-up may include a booting of the various modules of the cryo-control 104 and/or testing the various systems of the cryoablation apparatus 200 to ensure that the systems are functioning properly and may be connected to exchange or access information as may be desired during the method 700.

At step 704, the cryo-control 104 may begin obtaining operational information. The operational information may include data or information regarding the cryogen in the Dewar 202, such as temperature, pressure, and flow information. The cryo-control 104 may obtain information from the various sensors that may be included in the cryoablation apparatus 200 and the cryoprobe 128. Such information may be compared to a treatment plan or to preferred operational systems as may be indicated in clinical procedures.

At step 706, the cryo-control 104 may adjust, tune, and/or set the various operational parameters of the cryoablation apparatus 200 to desired settings or parameters. The cryo-control 104 may initiate heaters, vacuum pumps, cryogen pumps, or the like to begin a cryoablation cycle. The flow of cryogen may be initiated when the cryogen reaches its desired temperatures and/or pressures as may be required to achieve a desired cryoablation cycle. The settings and/or treatment plan may, for example, define a desired size of an iceball based on a size of the target tissue. This information in the treatment plan may, correspond, to a desired temperature and flow of the cryogen from the Dewar 202 to the cryoprobe 128.

At step 708, the cryo-control 104 may obtain Dewar capacitance and cryogen information. The Dewar capacitance may be information that is obtained from the cryogen rod 208, as previously described. The cryo-control 104 may charge and discharge the cryogen rod 208 using the control circuit 402, for example. During such charging and discharging, the voltage and current of the signals in the control circuit 402 may be obtained by the cryo-control. Other operational information of the Dewar may also be obtained such as temperature, pressure, and flow rates of the cryogen.

During a cryoablation cycle, the cryo-control 104 may continuously or semi-continuously perform the loop of steps 710, 704, 706, and 708. At step 710, the information obtained at step 704 and/or step 706 may be used to adjust operating parameters of the cryoablation apparatus 200 to optimize the cryoablation cycle. The cryo-control 104 may, for example, adjust parameters of the heater, vacuum pump, cryogen pump, or the like to achieve cryoablation conditions as may be defined in the treatment plan or clinical procedures. This loop of steps may be performed until the cryoablation cycle is complete.

While such looping and continuous monitoring and controlled adjustments may be performed, the cryo-control 104 may perform step 712. At step 712, the cryo-control 104 may determine a cryogen liquid level in the Dewar 202. The cryo-control 104 may determine the liquid level based on the Dewar capacitance information that was obtained at step 708. The cryo-control 708 may use equations 1 and/or 2, other algorithms, and/or a machine learning model in various implementations of the present disclosure. In one example, the cryo-control 104 may determine the cryogen liquid level based on a capacitance of the cryogen rod 208. Step 712 may be determined continuously during a cryoablation cycle. Since the cryogen rod 208 is an element in the Dewar 202, the use of the cryogen rod 208 as a liquid level sensor enables a determination of any level of liquid cryogen in the Dewar 202. In existing systems, multiple sensors may exist at discrete locations within the Dewar but such existing system may only determine discrete liquid levels at the location of individual discrete sensors. The information that can be obtained from such existing system is limited by the number of sensors placed in the Dewar. The capacitance-based liquid level sensors of the present disclosure (e.g., cryogen rod 208) allow for continuous measurements at any level in the Dewar 202.

At step 714, cryo-control 104 may determine whether the Dewar level is in a predetermined level range. In other examples, the cryo-control 104 may determine whether the cryogen liquid level is less than a predetermined level threshold. In the example shown, the cryo-control 104 may determine whether the cryogen liquid level is in the range of 15% to 100% of volume of the Dewar 202. In other examples, other predetermined level ranges may be used. The predetermined level ranges and/or the predetermined level threshold may be determined by a treatment plan, by clinical procedures, and may be input to the cryo-control 104 by a user or technician. If the cryogen liquid level is within the predetermined level range, the method 700 may proceed to step 716. If the cryogen liquid level is not in the predetermined level range, the method 700 may move to step 718.

At step 716, the cryo-control may adjust operating parameters to reach a preferred cryoablation status. In a manner similar to the adjustments at step 710, the cryo-control may adjust the flow of cryogen based on the measured cryogen liquid level that was determined at step 712. Such adjustment may be made to optimize an amount of liquid cryogen remaining in the Dewar 202, for example. The cryo-control 104 may also adjust or change various pressures, temperatures or other characteristics of the cryoablation apparatus 200 by causing the settings or operation of the heaters, vacuum pumps, cryogen pumps, or valves to be modified. After step 716, the method may return to step 704 to re-perform steps 704 through 716. Thus, the cryo-control 104 may continuously monitor the liquid level of the cryogen in the Dewar 202 and adjust various operational parameters of the cryoablation apparatus 200 as may be desired to achieve the desired clinical outcome.

At step 718, the cryo-control has determined that the liquid level of the cryogen is not within the predetermined level range. The cryo-control 104 may then determine whether the amount of cryogen that remains in the Dewar is enough to complete the current cryoablation procedure. If the procedure is nearing completion, for example, the amount of remaining liquid cryogen may be enough to complete the procedure. The cryo-control 104 may be able to determine whether the remaining liquid cryogen is enough based on the rate of usage that the cryo-control 104 has recorded during the current or historical cryoablation cycles. If the cryo-control 104 determines that enough liquid cryogen remains to complete the cryoablation procedure, the method 700 moves to step 716. The cryo-control 104 may perform step 716 as previously described and continue operating the cryoablation apparatus 200 to perform the cryoablation cycle.

If the cryo-control 104 determines that the amount of liquid cryogen remaining in the Dewar 202 is not enough to complete the cryoablation procedure, the method moved to step 720. At step 720, the cryo-control 104 may indicate that a refill of the Dewar 202 is required. The cryo-control 104 may alert users of the cryoablation apparatus 200 that a refill is required by sending an alert such as an audible signal, a text message, an email, a visible message, warning light, or other indicator. A refill warning may be indicated on a screen or display that is coupled to the cryo-control 104, in some examples. After the refill indicator is displayed or otherwise sent, the method 700 may move to step 722.

At step 722, the cryo-control 104 may determine whether the Dewar 202 has been refilled with liquid cryogen. The cryo-control 104 may determine whether the Dewar 202 has been refilled by measuring a liquid level of the Dewar 202, as previously described. In some examples, the cryo-control 104 may use the cryogen rod 208 or other capacitance-based liquid level sensor. In other examples, the cryo-control 104 may use a temperature sensor, a pressure sensor or other liquid level sensor to determine that the Dewar 202 has been refilled. If the cryo-control 104 determines that the Dewar 202 has been refilled, the method 700 may move to step 724. If the cryo-control determines that the Dewar has not been refilled, the method may move to step 726.

At step 724, the cryo-control 104 may re-initialize and/or re-start the cryoablation apparatus 200. The cryo-control 104 may need to re-adjust and/or activate elements of the cryoablation apparatus 200 to allow a new cryoablation cycle to begin. After step 724, the method may return to step 710 and the previously described states may be re-performed to perform a cryoablation cycle.

At step 726, the method 700 may end. The cryo-control 104 may determine that the Dewar 202 is not refilled. The cryo-control 104 may end the method after a certain amount of time has passed without the Dewar 202 being refilled. In other instances, a user may indicate or input to the cryo-control 104 that no refill will be performed. In such instances, the cryo-control 104 may halt further action by the cryoablation apparatus 200 and the method 700 may end.

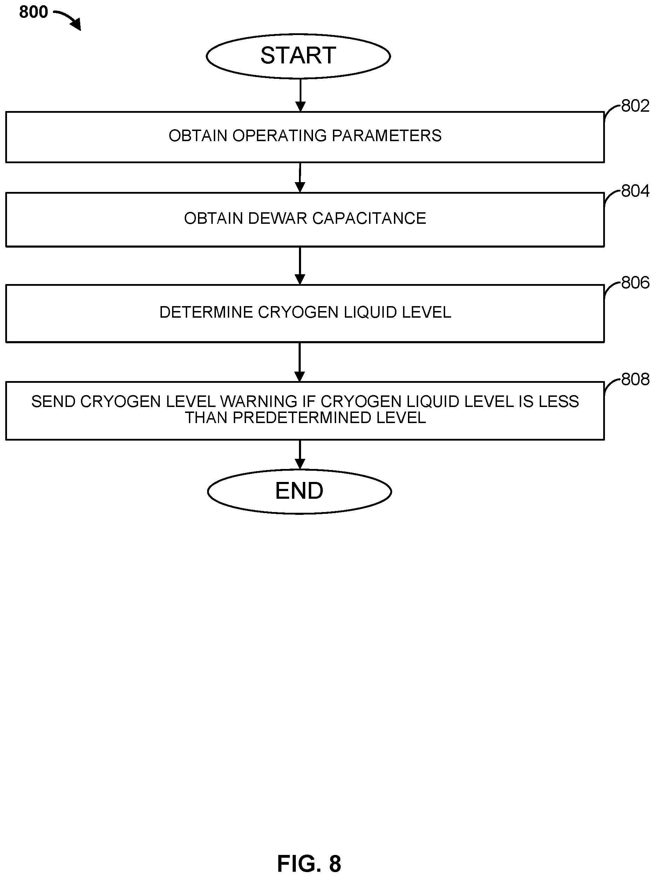

Another example method 800 of performing a cryoablation cycle is provided with reference to FIG. 8. The method 800 may be performed using one of the apparatuses described in the present disclosure. In other examples, the method 800 may be performed by other apparatuses. For illustration purposes, the method 800 is described below with reference to cryoablation system 100 and/or cryoablation apparatus 200. It should be appreciated, however, that the method 800 may be performed using other apparatuses or variations of system 100 and apparatus 200.

The method 800 may begin at step 802. At step 802, the cryo-control 104 may obtain operating parameters of the cryoablation apparatus 200. The operating parameters may include pressures, temperatures and flow information of the cryogen in the Dewar 202. The operating parameters may be obtained using various sensors that may be positioned in the Dewar 202.

At step 804, the cryo-control 104 may obtain an overall Dewar capacitance. The cryo-control 104 may determine the Dewar capacitance using a capacitance-based liquid level sensor such as the cryogen rod 208. The cryo-control 104 may obtain the Dewar capacitance by charging and discharging a capacitor in the Dewar 202. The capacitor may extend continuously from a top of the Dewar 202 to a base of the Dewar 202. The capacitance-based liquid level sensor may be implemented as a cryogen supply tube in the Dewar 202, as a combination of the cryogen supply tube and an inner surface of the Dewar 202, or as other capacitor surfaces in the Dewar 202. In one example, the capacitance-based liquid level sensor is a cryogen rod 208 extending from a base of the Dewar 202 that is fluidly connected to a supply tube and the cryoprobe 128.

At step 806, the cryo-control 104 may determine a cryogen liquid level in the Dewar 202 based on a capacitance of a capacitance-based liquid level sensor in the Dewar 202. The cryo-control 104 may determine the cryogen liquid level based on the capacitance and based on the operating characteristics of the Dewar 202 and/or the cryogen. A temperature, pressure, and flow of the cryogen 204 in the Dewar 202 may be used, in addition to the capacitance, to determine the cryogen liquid level. In some examples, a machine learning model may be used to determine the cryogen liquid level. The machine learning model may be a trained model that is trained using experimental and/or historical cryoablation data.

At step 808, the cryo-control 104 may send a cryogen level warning if the cryogen liquid level is less than a predetermined level. The predetermined level may correspond to a cryogen level that will allow the current cryogen cycle to be performed to completion. The cryo-control 104 may send a message, audible warning, display an alert message or other warning as previously described. The warning may be sent via a communications network to a user's portable device and/or to a display of the cryoablation apparatus 200.

Referring now to FIG. 9, an example computing device 900 is shown. The cryoablation system 100 may include one or more computing devices 900. For example, the cryoablation computing device 102 and/or the cryo-control 104 may have the elements shown in FIG. 9. The methods of the present disclosure, such as methods 700 or 800 may be performed, or steps of such methods may be performed, by a computing device 900.

As shown, the computing device 900 may include one or more processors 902, working memory 904, one or more input/output devices 906, instruction memory 908, a transceiver 912, one or more communication ports 914, and a display 916, all operatively coupled to one or more data buses 910. Data buses 910 allow for communication among the various devices. Data buses 910 can include wired, or wireless, communication channels.

Processors 902 can include one or more distinct processors, each having one or more cores. Each of the distinct processors can have the same or different structure. Processors 902 can include one or more central processing units (CPUs), one or more graphics processing units (GPUs), application specific integrated circuits (ASICs), digital signal processors (DSPs), and the like.

Processors 902 can be configured to perform a certain function or operation by executing code, stored on instruction memory 908, embodying the function or operation. For example, processors 902 can be configured to perform one or more of any function, step, method, or operation disclosed herein.

Instruction memory 908 can store instructions that can be accessed (e.g., read) and executed by processors 902. For example, instruction memory 908 can be a non-transitory, computer-readable storage medium such as a read-only memory (ROM), an electrically erasable programmable read-only memory (EEPROM), flash memory, a removable disk, CD-ROM, any non-volatile memory, or any other suitable memory.

Processors 902 can store data to, and read data from, working memory 904. For example, processors 902 can store a working set of instructions to working memory 904, such as instructions loaded from instruction memory 908. Processors 902 can also use working memory 904 to store dynamic data created during the operation of cryoablation computing device 102. Working memory 904 can be a random access memory (RAM) such as a static random access memory (SRAM) or dynamic random access memory (DRAM), or any other suitable memory.

Input-output devices 906 can include any suitable device that allows for data input or output. For example, input-output devices 906 can include one or more of a keyboard, a touchpad, a mouse, a stylus, a touchscreen, a physical button, a speaker, a microphone, or any other suitable input or output device.

Communication port(s) 914 can include, for example, a serial port such as a universal asynchronous receiver/-transmitter (UART) connection, a Universal Serial Bus (USB) connection, or any other suitable communication port or connection. In some examples, communication port(s) 914 allows for the programming of executable instructions in instruction memory 908. In some examples, communication port(s) 914 allow for the transfer (e.g., uploading or downloading) of data, such as cryogen level data and the like.

Display 916 can display a user interface 918. User interfaces 918 can enable user interaction with the cryoablation computing device 102. For example, user interface 818 can be a user interface that allows an operator to interact, communicate, control and/or modify different messages, settings, or features that may be presented or otherwise displayed to a user. The user interface 918 can include a slider bar, dialogue box, or other input field that allows the user to control, communicate or modify a setting, limitation or input that is used in a cryoablation treatment. In addition, the user interface 918 can include one or more input fields or controls that allow a user to modify or control optional features or customizable aspects of the cryoablation computing device 102 and/or the operating parameters of the cryoablation system 100. In some examples, a user can interact with user interface 918 by engaging input-output devices 906. In some examples, display 916 can be a touchscreen, where user interface 918 is displayed on the touchscreen. In other examples, display 916 can be a computer display that can be interacted with using a mouse or keyboard.

Transceiver 912 allows for communication with a network. In some examples, transceiver 912 is selected based on the type of communication network cryoablation computing device 102 will be operating in. Processor(s) 902 is operable to receive data from, or send data to, a network, such as wired or wireless network that couples the elements of the cryoablation system 100.

ADAPTIVE PRESSURE CONTROL OF CRYOGEN

In some embodiments of the present disclosure, a cryoablation apparatus is provided that enables adaptive pressure control of cryogen. The adaptive pressure control functionality may operate to measure and control a pressure of cryogen in multiple locations in the cryogen storage, supply, and flow from a Dewar or other storage vessel to the cryoprobe. The pressure may be measured and controlled to maintain a desired pressure and/or temperature of the cryogen at various positions in the cryoablation apparatus. The pressure control may operate to more efficiently and effectively utilize the cryogen during cryoablation procedures to reduce cost, improve efficiency, and/or improve effectiveness of the treatment.

In some examples, the cryoablation apparatus may include a smart pressure control, a liquid cryogen heating assembly, a high pressure pump motor assembly, and a Dewar lid assembly. The cryoablation apparatus may be operated by the smart pressure control to independently control a pressure and/or temperature of the cryogen at various locations in the cryoablation apparatus such as in the Dewar, in a cryogen supply conduit, and/or at the cryoprobe. The independent control may allow the cryogen to be used more efficiently that existing systems that may use gravity or single pumps to control the pressure and move cryogen to a cryoprobe.

In some examples, the cryoablation apparatuses and methods of the present disclosure may utilize at least two pumps that may include a low pressure pump and a high pressure pump to independently operate to control a pressure and flow of the cryogen through the system. The pressure control may utilize a closed-loop system whereby operating information may be obtained and then used to continuously control the cryogen pressure and flow to operate the cryoablation apparatus more efficiently and effectively than existing systems. The cryogen that is used in the system may include various suitable cryogens such as nitrogen, helium, argon, or the like. The disclosure below may include a description of operating pressures of liquid nitrogen but it should be appreciated that other cryogens may be used.

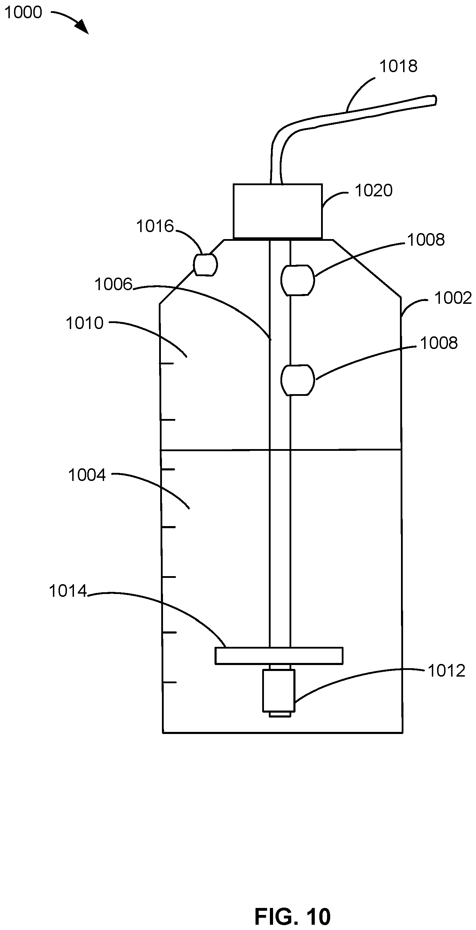

Referring now to FIG. 10, an example cryoablation apparatus 1000 is shown. The cryoablation apparatus 1000 may be incorporated as part of a cryoablation system such as cryoablation system 100 (FIG. 1). The cryoablation apparatus 1000 may be an alternate example of the cryoablation apparatus 116 shown in FIG. 1. The cryoablation apparatus 1000 may be coupled to a cryoprobe 128 to deliver cryogen for a cryoablation treatment. The cryoablation apparatus 1000 may be coupled to a pressure control (not shown in FIG. 10) that may obtain information such as pressure, temperature, and/or flow information from the cryoablation apparatus 1000 and adjust, change, or modify the operation of the cryoablation apparatus 1000.

The cryoablation apparatus 1000 may include a Dewar 1002, a supply tube 1006, one or more sensors 1008, a heating assembly 1012, a high pressure pump assembly 1014, a valve assembly 1016, a cable 1018, and a lid assembly 1020. The Dewar 1002 may be configured as previously described to include a shell of material that may be thermally insulated, double-walled, vacuum-insulated or the like. The Dewar 1002 may define an internal volume to hold a volume of cryogen, such as liquid nitrogen. The cryogen may be deposited into the Dewar 1002 as liquid cryogen 1004. The liquid cryogen may change to cryogen vapor 1010 in the internal volume of the Dewar 1002. In one example, the liquid cryogen 1004 may be pressurized to a desired pressure and/or temperature before it is pumped or otherwise moved from the Dewar 1002 to the cryoprobe 128. The Dewar 1002 may be configured with the liquid level sensors previously described. The liquid level sensors may be capacitance-based liquid level sensors, for example.

The heating assembly 1012 of the cryoablation apparatus 1000 may include a heater such as a resistive heater that can transfer heat to the liquid cryogen 1004. As the liquid cryogen 1004 is heated, the liquid cryogen 1004 may be converted to cryogen vapor (gas) 1010. The accumulation of cryogen vapor 1010 in the Dewar 1002 may cause the pressure of the cryogen in the Dewar 1002 to increase. The heating assembly 1012 may be activated to convert liquid cryogen 1004 to cryogen vapor 1010 until a predetermined or desired pressure is achieved in the Dewar 1002. In this example and as will be further explained, the heating assembly 1012 may be positioned at distal end of the supply tube 1006 that is located in the liquid cryogen 1004 near to or proximate a base of the Dewar 1002. In other examples, the heating assembly 1012 may be located at other locations but is preferably positioned below a liquid level of the cryogen in the Dewar 1002.

The cryoablation apparatus 1000 may also include the high pressure pump assembly 1014. In this example, the high pressure pump assembly 1014 is positioned at or toward the distal end of the supply tube 1006 at or near the heating assembly 1012. The high pressure pump assembly 1014 may be positioned at above the heating assembly 1012. The high pressure pump assembly 1014 may pressurize the liquid cryogen 1004 to a high pressure level to provide highly pressurized liquid cryogen to the cryoprobe. In one example, the pump assembly 1014 may include an internal pressure cylinder design that is operated by the lid assembly 1020 to move and pressure the low pressure liquid cryogen to high pressure liquid cryogen (e.g., about 300 psi to about 600 psi).

While not shown, the lid assembly 1020 may be coupled to a cryogen supply conduit and/or cryogen supply manifold that may be used to fluidly connect the cryogen in the Dewar 1002 to one or more cryoprobes 128. The cryoprobes 128 may be inserted at or near the target tissue in the patient during a cryoablation treatment. The sizing and capacity of the Dewar 1002 and the other elements of the cryoablation apparatus 1000 may be configured to allow a suitable volume and flow of cryogen to be supplied to the desired number of cryoprobes 128.

The sensors 1008 may be positioned at various locations inside the Dewar 1002. In the example shown, multiple sensors 1008 are positioned at various vertical locations along the supply tube 1006. The sensors 1008 may be positioned at various locations in the liquid cryogen 1004 and the cryogen vapor 1010. The sensors 1008 may also be positioned at locations along the surface of the Dewar 1002 and/or on other elements that may be included in the cryoablation apparatus 1000. The sensors 1008 may be configured as suitable sensors to provide information regarding the conditions in the Dewar 1002 and/or conditions of the cryogen that may be located in the Dewar 1002 and/or the supply tube 1006. The sensors 1008 may be coupled to a data acquisition module of the pressure control via wired or wireless connections. In the example shown the sensors 1008 may be coupled to the pressure control via the cable 1018. In other examples, other connections may be used.

The sensors 1008 may be positioned at multiple locations to provide information such as pressure, temperature, or flow information regarding the cryogen in the Dewar and the cryogen being supplied to the cryoprobes. The sensors 1008 may be located at suitable positions to provide, for example, temperature and/or pressure information for the cryogen in the Dewar 1002 that may be maintained at a low pressure condition. The sensors 1008 may also be located at suitable positions to provide, for example, temperature and/or pressure information for cryogen being supplied to the cryoprobe that may be maintained or operated at a high pressure condition. The sensors 1008 may be configured, in some examples, as temperature-pressure sensor transducers. In other examples, other suitable pressure or temperature sensors such as thermistors, thermocouples, piezoelectric pressure sensors, strain gauge pressure sensors, or the like.

The valve assembly 1016 may include one or more controllable valves that may be used to control a pressure of the cryogen in the Dewar 1002. The valve assembly 1016 may include manual or electronically actuated valves that may be used to vent cryogen to the ambient environment. The valve assembly 1016 may be coupled to a pressure control that may open or close the valves of the valve assembly 1016 in response to pressure measurements obtained from the sensors 1008.

The lid assembly 1020 may be positioned at or near a top of the Dewar and may be connected to the supply tube 1006. The lid assembly 1020 may seal the connection location of the supply tube 1006 to the Dewar 1002. The lid assembly 1020 may also seal a location at which the cable 1018 may enter the Dewar 1002 and allow the cable 1018 to be coupled to the various elements of the cryoablation apparatus 1000. The lid assembly may also include one or more of the sensors 1008 to provide information regarding the conditions in the Dewar 1002 and/or in the supply tube 1006. The lid assembly 1020 may also include a pump motor and gear box that operates to move the cylinder in the pump assembly 1014. The pump motor may be controlled to rotate at a desired rotational speed such as 500 rotations per minute (rpm). The pump motor may be coupled to the high pressure pump assembly 1014 previously described.

As previously described, the cable 1018 may be coupled to the various elements of the cryoablation apparatus 1000 to provide control signals that may cause the elements to operate to reach desired operating conditions (e.g., temperature and pressure) in the Dewar 1002. The cable 1018 may include a bundle of wires that may be connected to the sensors 1008, the heating assembly 1012, the high pressure pump assembly 1014, the valve assembly 1016, for example. The cable 1018 may also be coupled to the pressure control that may obtain operating information and then control the operation of the cryoablation apparatus to reach desired operating conditions.

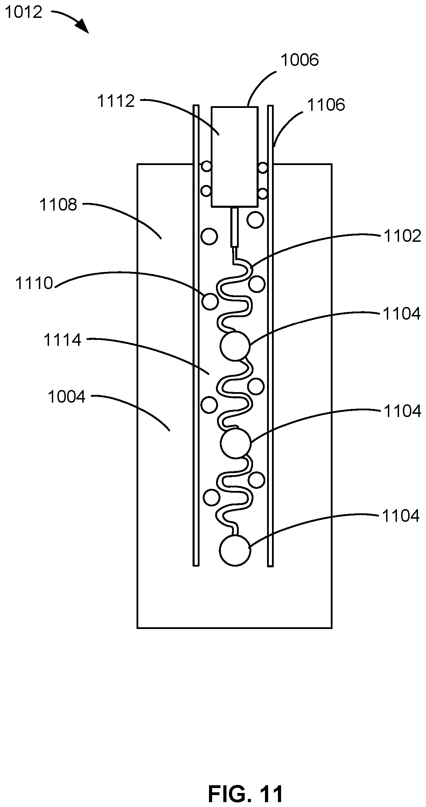

Referring now to FIG. 11, an example heating assembly 1012 is shown. The heating assembly 1012 may be positioned at or near a distal end 1112 of the supply tube 1006. The heating assembly 1012 may include a heater 1102, one or more sensors 1104, and a bubble barrier 1106. The heater 1102 may include one or more heating elements that may extend into the liquid cryogen 1004. The heater 1102 may be a resistive heater, a laser heater, infrared heater or other heaters. Other heaters may include a radiation heater, wireless emission heater, microwave heater, and RF heater. The heater 1102 may be positioned within a heating boundary 1114 defined by the bubble barrier 1106. The heating boundary 1114 may be a volume that surrounds the heater 1102. The heating boundary 1114 may be separated from the interior volume of the Dewar 1002 by the bubble barrier 1106. The bubble barrier 1106 may be a wall that may surround the heater 1102. In the example shown, the bubble barrier 1106 may have a cylindrical shape to define a cylindrically shaped heating boundary 1114. The bubble barrier 1106 may be open at its top and bottom to allow liquid cryogen 1004 to enter the heating boundary 1114 and allow cryogen vapor to exit the heating boundary 1114. The bubble barrier 1106 may be made of a double-walled or insulated stainless steel, for example, and be shaped as a vertically oriented tubular member with an axis that is aligned with an axis of the supply tube 1006. The bubble barrier 1106 may alternatively be made of a non-heat conducting material. In some examples, the bubble barrier 1106 may be formed from a rubber, plastic, polymer, or composite material. In other examples, the bubble barrier 1106 may have other shapes or configurations.

When the heater 1102 is activated, the liquid cryogen 1004 that is located inside the bubble barrier 1106 may begin to be converted to cryogen vapor. The cryogen vapor may form as vapor bubbles 1110. The vapor bubbles 1110 may travel upwards after formation before reaching a surface of the liquid cryogen 1004. The bubble barrier 1106 retains the cryogen vapor locally in the heating boundary 1114 inside the bubble barrier 1106. In other systems without the heating boundary 1114, the cryogen vapor bubbles 1110 may travel without restriction and may move in various directions throughout the Dewar 1002. The restriction of the cryogen vapor bubbles 1110 to the heating boundary 1114, allows a region of the liquid cryogen 1004 that is located in the bubble barrier 1116 to reach a localized pressure that is different from a pressure of the liquid cryogen that may be located outside the bubble barrier 1116. This localized pressure differential may allow the pressure of the liquid cryogen 1004 to be greater than the pressure of the liquid cryogen 1004 that is located outside the bubble barrier 1116.

The heating assembly 1012 with the bubble barrier 1106 may allow the liquid cryogen 1004 to achieve a desired pressure condition more quickly than in other systems that do not include this structure. In other systems, a heater may heat liquid cryogen but the liquid cryogen may not be moved to the cryoprobe until the entire Dewar 1002 is pressurized to a desired pressure condition. In the systems and apparatuses of the present disclosure, the liquid cryogen 1004 may be pressurized to desired pressure locally inside the bubble barrier 1116 rather than pressurizing the entire Dewar 1002 to the desired pressure condition. The systems and apparatuses of the present disclosure may allow cryoablation cycles to be performed more quickly than other systems. In systems that require the entire Dewar 1002 to be pressurized to a desired pressure condition, such pressure condition may be achieved in periods of time that may be in the range of about 45 minutes to about 60 minutes. The systems and apparatuses of the present disclosure may achieve cryogen in the desired pressure condition in about 10 to about 15 minutes. In other examples, the systems and apparatuses of the present disclosure may achieve cryogen in the desired pressure condition in less than about 10 minutes.

As used in the present disclosure, a low pressure condition of the cryoablation apparatus 1000 may refer to a condition of the cryogen in the Dewar which is maintained before the cryogen is moved to the cryoprobe. The low pressure condition may correspond to a pressure in a range of about 10 pounds per square inch (psi) to about 15 psi. This low pressure condition has been determined to result in efficient conversion of the liquid cryogen to the high pressure condition. The low pressure condition may be maintained in the Dewar 1002 as will be further described below. The heating assembly 1012 may use a closed-loop process to maintain the pressure in the Dewar 1002 in the low pressure condition. The high pressure condition may refer to an pressure that is higher than the low pressure condition and may refer to the pressure of the cryogen that is moved from the Dewar 1002 to the cryoprobe to perform a cryoablation cycle. The high pressure condition may refer to a pressure of the cryogen sufficient to remove sufficient amount of heat from the cryoprobe and surrounding tissue to form an iceball at a distal end of the cryoprobe. The high pressure condition may correspond to a super-critical, critical, or near-critical state of the liquid cryogen. The high pressure condition may correspond to a pressure in a range of about 450 psi to about 550 psi or greater. The pump assembly 1014 and/or the lid assembly 1020 may operate to increase the liquid cryogen from the low pressure condition in the Dewar 1002 to the high pressure condition for use in the cryoprobe 128.

The heating assembly 1012 may also include one or more sensors 1104. The sensors 1104 may a temperature and/or pressure sensor such as those previously described with respect to sensors 1008 (FIG. 10). The sensors 1104 may be positioned inside the bubble barrier 1106. The sensors 1104 may also be positioned at different vertical locations within the heating boundary 1114. The sensors 1104 may provide temperature and/or pressure information to the pressure control that may activate the heater 1102 as needed to achieve a predetermined high pressure condition of the liquid cryogen 1004 inside the heating boundary 1114. When such a suitable or predetermined high pressure condition is achieved, the pressure control may also activate a pump to move the liquid cryogen from inside the bubble barrier 1106 to the cryoprobe through the supply tube 1006.

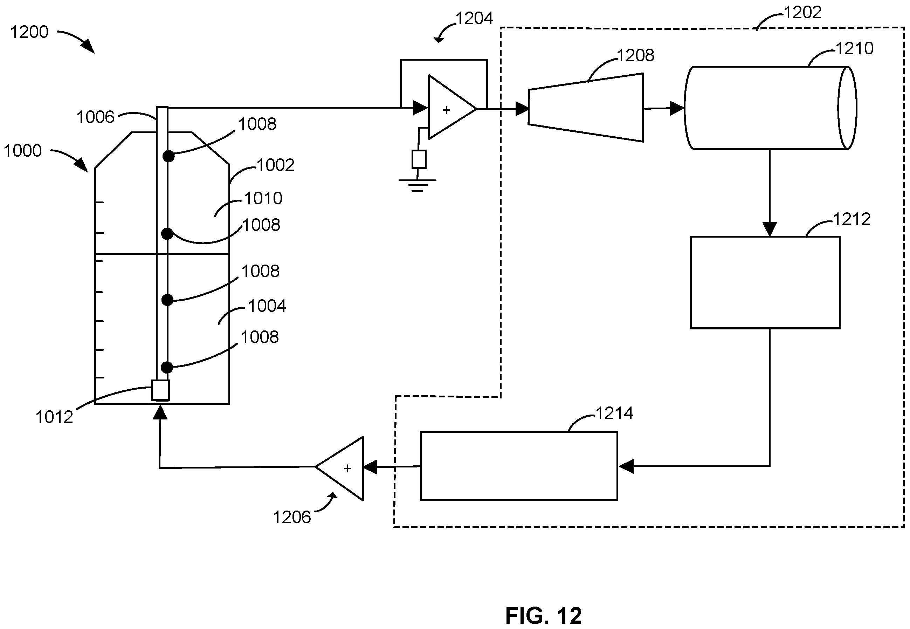

Referring now to FIG. 12, an example cryoablation system 1200 is shown. The cryoablation system 1200 may include the cryoablation apparatus 1000 previously described. The cryoablation apparatus 1000 may include the heating assembly 1012 that includes the heating boundary 1114 and heater 1102. The cryoablation apparatus 1000 may be coupled to one or more control circuits 1204, 1206. The cryoablation apparatus 1000 may also be coupled to a pressure control 1202. The pressure control 1202 may be configured as a computing device, PLC, controller or other suitable device. The pressure control 1202 may have the functionality and may be combined with or implemented as the control 104 previously described. The pressure control 1202 may have the elements of the computing device 900 described above.

The pressure control 1202 may operate to obtain information from the cryoablation apparatus 1000 and/or the cryoprobe 128 to determine operating parameters of the cryoablation apparatus 1000. The pressure control 1202 may include a converter 1208, a communication module 1210, a cryogen pressure module 1212, and a heating module 1214. Each of the modules may be implemented in various manners such as by executable instructions, application specific circuits or the like.

The converter 1208 may be coupled to the control circuit 1204. The control circuit 1204 may include an amplifier or other elements to provide signals or data regarding operation of the cryoablation apparatus 1000. The sensors 1008 and 1104 may be coupled to the control circuit 1204 and, in turn, to the converter 1208. The converter 1208 may be configured as an analog-to-digital (A2D) converter to provide a digital signal. The converter 1208 may be coupled to the communication module 1210. The communication module 1210 may be a transceiver, data acquisition unit or other suitable device or module to obtain data from the cryoablation apparatus 1000. The communication module 1210 may be coupled to the other elements of the cryoablation system 1200 using wired or wireless connections to provide information and/or feedback regarding operation of the cryoablation system 1200.

The communication module 1210 may be coupled to the pressure module 1212. The pressure module 1212 may determine various operating parameters of the cryoablation system 1200. The pressure module 1212 may determine what action should be taken to achieve a desired result or outcome during a cryoablation procedure or treatment. In some examples, a treatment plan may be generated prior to a cryoablation treatment. The treatment plan may describe various details of the cryoablation treatment such a number or duration of cryoablation cycles, a size of an iceball, and other aspects. The pressure module 1212 may determine temperatures, pressures, and flow rates of the cryogen that may be desired to achieve the details described in the treatment plan. The pressure module 1212 may also determine when and how particular elements of the cryoablation system 1200 will be activated to achieve the details of the treatment plan. The pressure module 1212 may, for example, determine when the heating assembly 1012 will be activate and for how long to achieve a desired temperature and pressure of the liquid cryogen. The pressure module 121 may determine when and if the valve assembly 1016 should be activated to release pressure from the Dewar 1002. The pressure module 1212 may determine when and how the pump assembly 1014 is activated to achieve a temperature, pressure, and flow of cryogen to the cryoprobe 128. The pressure module 1212 may determine other operating aspects of the cryoablation system 1000 as well.

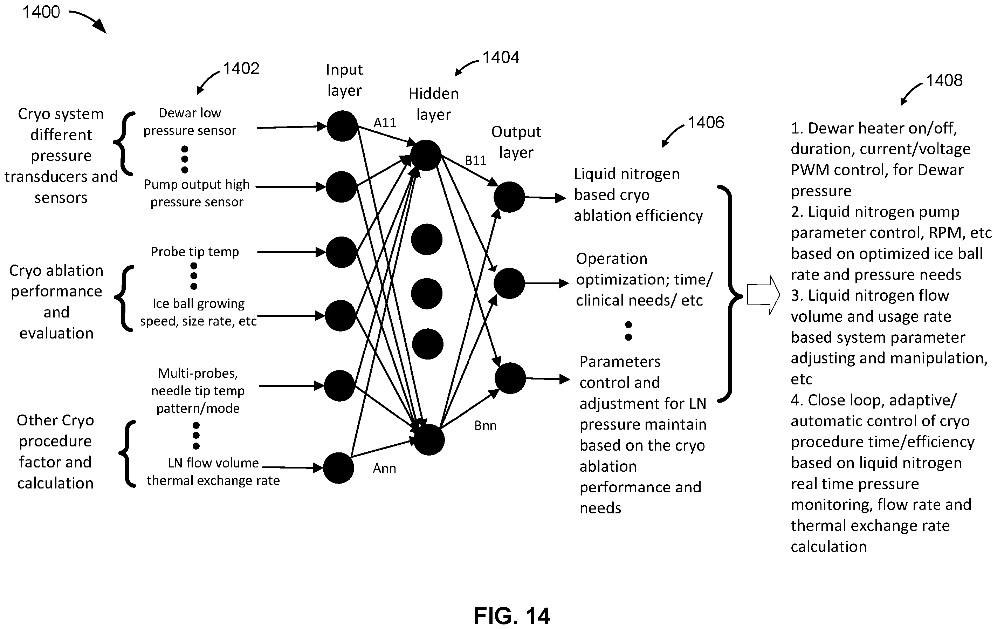

The pressure module 1212 may use algorithms, look-up tables, machine learning models, artificial neural networks (ANN), regressions, and other tools to determine the operation of the cryoablation system 1200. If machine-learning models or ANNs are used historical or experimental data may be used to train such models and then updated with treatment data to improve their performance. An example machine learning model or ANN is described below with respect to FIG. 14.

The pressure module 1212 may be coupled either directly or indirectly to other elements of the cryoablation system 1200 to control the function and operation. As shown, the pressure module 1212 may be coupled to the heating module 1214. The heating module 1214 may activate and control the function of the heating assembly 1012. The heating module 1214 may be coupled to the control circuit 1206. The heating module 1214 may include a suitable power supply that may be amplified or conditioned by the control circuit 1206 to provide a power signal to the heating assembly 1012. The power signal may energize the heater 1102 of the heating assembly 1012 to cause the liquid cryogen to be converted to cryogen vapor. The heating module 1214 may provide the power signal to maintain or create desired operating conditions (e.g., pressure, temperature, etc.) in the heating assembly 1012. Pulse width modulation (PWM), frequency, amplitude or other controls may be provided by the heating module 1214.

The cryoablation system 1000 may operate to maintain a low pressure condition in the Dewar 1002. The pressure module 1212 may obtain temperature and/or pressure information from the sensors 1008 in the cryoablation apparatus 1000. The pressure module 1212 may determine that the pressure in the Dewar 1002 is less than the desired temperature such as the low pressure operating condition (e.g., 10 to 15 psi). The pressure module 1212 may communicate with the heating module 1214 to energize the heating assembly 1012 to heat the liquid cryogen 1004 in the Dewar 1002. The pressure module 1212 may continue to monitor a pressure in the Dewar 1002 using a closed-loop feedback process to continue heating the liquid cryogen 1004 in the Dewar 1002 until the desired pressure is achieved in the Dewar 1002. The pressure module 1212 may then deactivate and modify a power signal that is delivered to the heating assembly 1012 to maintain the desired pressure in the Dewar 1002.

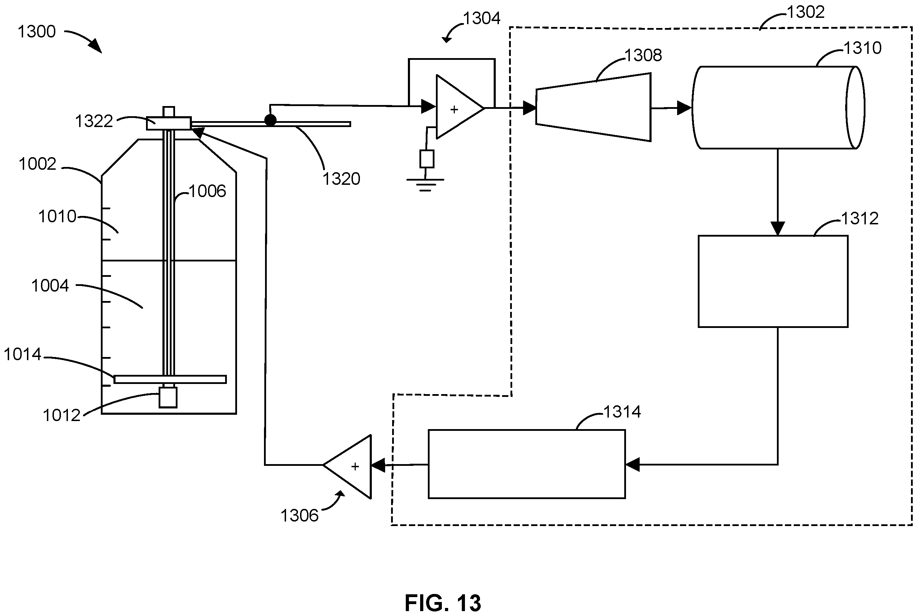

Another example cryoablation system 1300 is provided in FIG. 13. The cryoablation system 1300 is similar in many respects to the cryoablation system 1200 previously described. The cryoablation system 1300 may include a pressure control 1302 that includes an A2D converter 1308, a communication module 1310, a pressure module 1312, and a heating module 1314. The cryoablation system 1300 may also include control circuits 1304, 1306. These elements may have a structure and function similar to that described above for the same elements of cryoablation system 1200. For the sake of brevity, the descriptions of such elements are not repeated.

In the example shown, the cryoablation system 1300 may include cryoablation system 1300 that includes at least two assemblies to change or modify the pressure in the system. As shown, the cryoablation system 1300 may include a first assembly, such as the heating assembly 1012 and a second assembly such as the pump assembly 1014. The two assemblies may be controlled via the pressure control 1302 to efficiently and effectively deliver high pressure cryogen to the cryoprobe as need for the cryoablation treatment.

The two assemblies may be controlled and/or adjusted by the pressure control 1302 to raise the pressure of the liquid cryogen to a high pressure condition. The high pressure condition may correspond to a critical, super-critical, or near-critical pressure for the cryogen. In an example in which liquid nitrogen is used as the cryogen, the high pressure condition may correspond to a pressure in the range of about 400 psi to about 600 psi. In some examples, the heating assembly 1012 may be used to raise the pressure of the liquid cryogen to a first pressure range and the pump assembly 1014 may raise the pressure of the liquid cryogen from the first pressure range to a second high pressure range.

The cryoablation system 1300 may include a sensor 1320 positioned at or near an outlet of the lid assembly 1322. The sensor 1320 may be a temperature and/or pressure sensor (such as those previously described). The sensor 1320 may be configured to provide information regarding the pressure of the cryogen that is supplied by the lid assembly 1322 to the cryoprobe 128. The pressure may preferably be in the high pressure condition for use to perform a cryoablation cycle at the cryoprobe 128. The sensor 1320 may be configured to measure high pressures (e.g., in a range of about 400 psi to about 600 psi). In other examples, the cryoablation system 1300 may include additional sensors positioned at various location along the cryogen supply line and/or the cryoprobe 128 to provide information regarding a temperature, pressure, and/or flow of the cryogen.

Referring now to FIG. 14, an example machine learning or ANN model 1400 is illustrated. The model 1400 may use machine learning to determine relationships between inputs 1402 to determine outputs 1406 and operational actions 1408. The inputs may be processed using various layers 1404. The term model, such as model 1400, may refer to data models created using machine learning and/or artificial intelligence. Machine learning may involve training a mathematical model in a supervised or unsupervised setting. Machine learning models may be trained to learn relationships between various groups of data. The models may be based on a set of algorithms that are designed to model abstractions in data by using a number of processing layers. The processing layers may be made up of non-linear transformations. Machine learning models may include, for example, neural networks, convolutional neural networks and deep neural networks. Such neural networks may be made of up of levels of trainable filters, transformations, projections, hashing, and pooling. The models may be used in large-scale relationship-recognition tasks. The models can be created by using various open-source and proprietary machine learning tools and/or libraries known to those of ordinary skill in the art.

In the example shown, the inputs 1402 may include data and information such as information obtained from the Dewar sensors, including low pressure sensors and high pressure sensors. The inputs 1402 may also include temperature data such as probe temperature data. The inputs 1402 may also include iceball information such as iceball growth rates, iceball size, and/or iceball isotherm locations. The inputs 1402 may also include other information such as cryogen flow rates, volumes and the like.

The model 1400 may determine outputs 1406. The outputs 1406 may include an efficiency or other treatment optimization measures. The outputs 1406 may also include operational parameters of the cryoablation apparatus such as desired pressures, and adjustments to the operational parameters to improve efficiency and/or effectiveness.

The outputs 1406 may be used to determine operational actions 1408. The operational actions 1408 may be implemented by the pressure control, for example. The operational actions 1408 may include an activation or timing of the heater and/or a configuration of the power signal to be delivered to the heater including PWM, current, voltage, or other power signal characteristics. The operational actions 1408 may also include cryogen pump parameters, cryogen flow parameters, and cryoablation cycle timing, duration, and others.

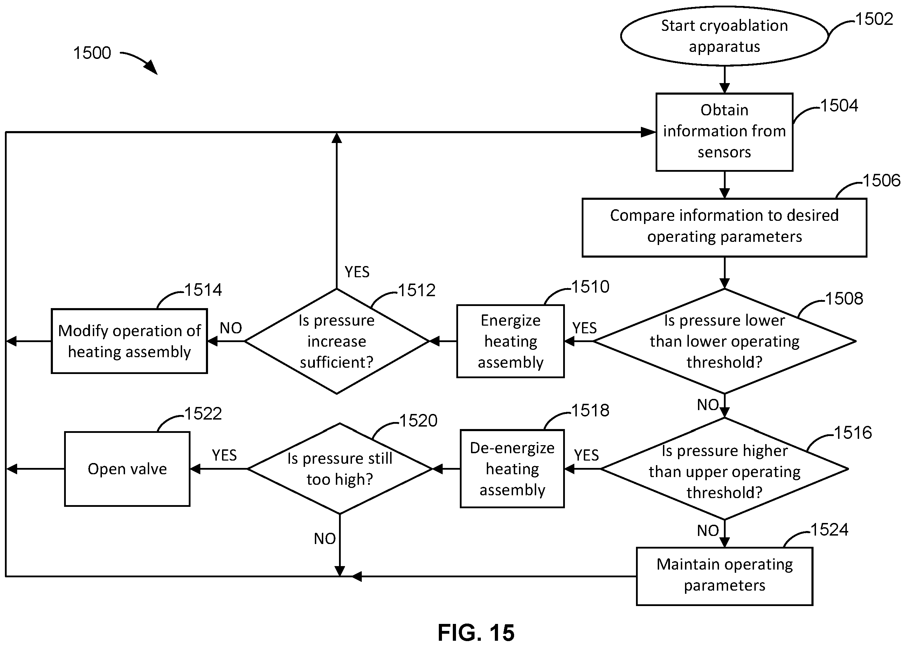

Referring now to FIG. 15, a method 1500 for maintaining a Dewar low pressure condition is provided. The method 1500 may be performed using the cryoablation apparatuses of the present disclosure but it should be appreciated that other apparatuses may be used. For example, the cryoablation system 1200 may be used to perform the method 1500. The description of method 1500 below describes operations as performed by the cryoablation system 1200 but it should not be interpreted as limiting the method to this particular apparatus.

The method 1500 may be used to achieve a low pressure condition in the Dewar 1002. As previously described, it has been determined that a low pressure condition of about 10 psi to about 15 psi is desired for a cryoablation apparatus that utilizes liquid nitrogen as the cryogen. The low pressure condition may have other ranges or desired levels for other cryogens or for other cryoablation apparatuses. This low pressure condition is desirable because it allows the other high pressure pumps of the cryoablation apparatus to efficiently and effectively pressurize the cryogen to a temperature and pressure for use in the cryoprobe 128.

At step 1502, the method 1500 may start. At step 1502, the cryoablation apparatus 1000 and the pressure control 1202 may be turned on and/or the Dewar 1002 may be filled with cryogen to desired level. At step 1504, the pressure control 1202 may begin obtaining information from the various sensors 1008. The sensors 1008 may provide, for example, temperature and pressure information of the Dewar 1002.

At step 1506, the pressure control 1202 may compare the information from the sensors 1008 to desired operating parameters. The desired operating parameters may be described in a treatment plan, operating or clinical procedures, or may be experimental or clinically determined. The desired operating parameters may correspond to operating parameters that achieve treatment results and/or achieve optimized and efficient performance of the cryoablation apparatus 1000. The desired operating parameters may be determined by algorithms, models, machine learning or other methods as previously described. The desired operating parameters may include a lower pressure threshold and an upper pressure threshold of a low pressure range of the Dewar 1002. The low pressure range may correspond to a desired low pressure range of the Dewar 1002 that may allow efficient and effective conversion of the liquid cryogen to high pressure cryogen. It may be desirable, therefore, to maintain the Dewar 1002 within the low pressure range for efficient, effective operation of the cryoablation apparatus 1000.

At step 1508, the pressure control 1202 may determine whether the Dewar pressure is lower than the lower operating threshold defined in the desired operating parameters described above. In some examples, the lower operating threshold may correspond to about 10 psi for an apparatus using liquid nitrogen as the cryogen. In other examples, the lower operating threshold may correspond to other pressure values. If the pressure control 1202 determines that the pressure in the Dewar 1002 is lower than the lower operating threshold, the method may move to step 1510. If the pressure control 1202 determines that the pressure in the Dewar 1002 is not lower than the lower operating threshold, the method 1500 may move to step 1516.

At step 1510, the pressure control 1202 may energize or turn on the heater of the heating assembly 1012. The pressure control 1202 may cause a power signal with a suitable power level, PWM, voltage, or current is delivered to the heater. After the heater is energized, the liquid cryogen 1004 may be converted to cryogen vapor 1010 and the pressure in the Dewar 1002 may increase.

At step 1512, the pressure control 1202 may determine whether the rate of increase of the pressure in the Dewar 1002 is sufficient for the cryoablation cycle. For example, if the cryoablation apparatus 1000 has just been started, the user may want to begin a cryoablation cycle as soon as possible. The pressure control 1202 may desire a rate of pressure increase as quickly as possible. If the cryoablation process is underway and an iceball is being formed, and/or the cryoablation cycle is almost complete, it may be desirable for minimal pressure increase since the cycle is almost complete. These different circumstances may dictate a sufficient rate of increase of pressure in the Dewar 1002. If the pressure control 1202 determines that the rate of increase is sufficient, the method 1500 may return to step 1504 where information is continued to be obtained from the sensors and the pressure in the Dewar is continually monitored. If the pressure control 1202 determines that the rate of pressure increase is not sufficient, the method 1500 may proceed to step 1514.

At step 1514, the pressure control 1202 may modify the power delivered to the heating assembly 1012. The pressure control 1202 may increase the power delivered to the heating assembly 1012 to increase the generation of cryogen vapor and increase the rate of pressure increase. The pressure control 1202 may otherwise modify the power signal to increase the rate of pressure increase. The pressure control 1202 may modify the current, voltage, PWM, or other variable of the power signal, for example. After such modification is made, the method 1500 may return to step 1504 to continue monitoring and control of the pressure in the Dewar 1002.

Returning now to step 1516, the pressure control 1202 may determine if the pressure in the Dewar 1002 is higher than the upper operating threshold. The pressure control previously determined that the pressure in the Dewar 1002 was not lower than the lower operating threshold (at step 1508) and now determines if the pressure is higher than the upper operating threshold. The upper operating threshold may correspond to about 15 psi for a cryoablation apparatus 1000 that uses liquid nitrogen as the cryogen. The upper operating threshold may have other values in other examples and for other cryogens. If the pressure control 1202 determines that the pressure in the Dewar 1002 is higher than the upper operating threshold, the method 1500 moves to step 1518. If the pressure control 1202 determines that the pressure in the Dewar is not higher than the upper operating threshold, the pressure control 1202 has determined that the pressure in the Dewar is in the desired operating range and no changes are required. In this circumstance, the method 1500 returns to step 1504 to continue to obtain information from the sensors 1008 and monitor the pressure in the Dewar 1002.

If the method 1500 moves to step 1518, the pressure control 1202 can de-energize the heating assembly 1012. Since the pressure in the Dewar 1002 is too high, the heater no longer needs to convert liquid cryogen to cryogen vapor. The heater can be de-energized to stop this conversion. The method 1500 may then proceed to step 1520.

At step 1520, the pressure control 1202 may determine whether the pressure in the Dewar is still too high (i.e., above the upper pressure threshold) or is increasing. If the pressure is still too high or the pressure is increasing, the method 1500 may move to step 1522. If the pressure is not too high and the pressure is not increasing, the method may proceed to step 1504 to continue to obtain information from the sensors 1008 and monitor the pressure in the Dewar 1002.

At step 1522, the pressure control 1202 may lower pressure in the Dewar. The pressure control 1202 may actuate or open the valve assembly 1016. The pressure control 1202 may cause pressure to be vented from the Dewar to decrease the pressure and/or stop the pressure in the Dewar from increasing. After the pressure control 1202 takes action to decrease pressure or decrease pressure increase, the method 1500 may return to step 1504 to continue to obtain information from the sensors 1008 and monitor the pressure in the Dewar 1002.

As can be appreciated, the method 1500 may be performed continuously or semi-continuously during operation of the cryoablation apparatus 1000 to maintain the Dewar temperature in the desired operating range. This closed-loop feedback process can be used so that the cryoablation apparatus 1000 can be efficiently used to provide cryogen to the cryoprobe 128.

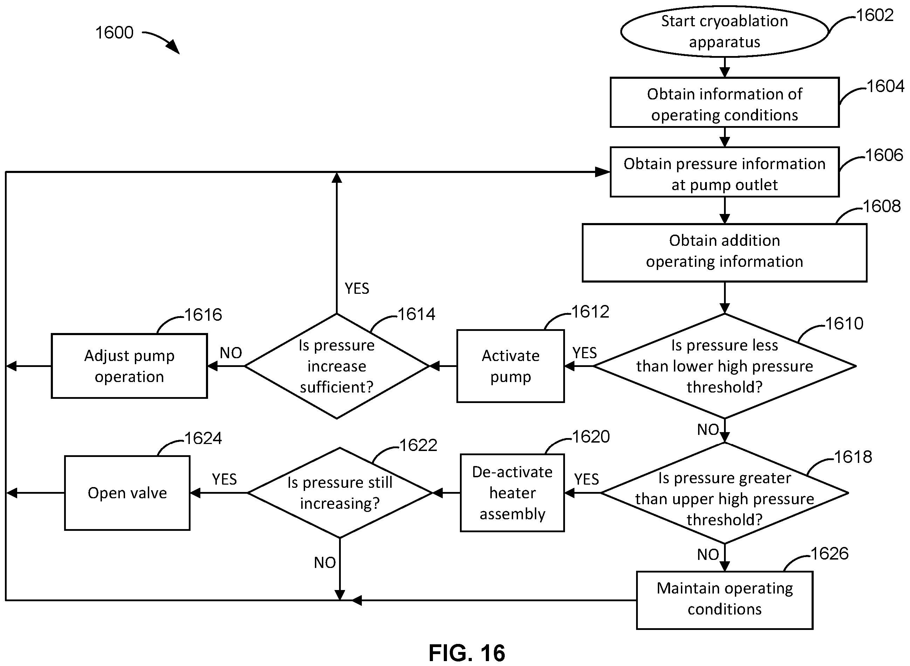

Turning now to FIG. 16, a method 1600 of providing cryogen to a cryoprobe is provided. The method 1600 may be performed simultaneously or in parallel with the method 1500. The method 1600 may be performed, for example, by the cryoablation apparatus 1000 and/or the cryoablation system 1300. The method 1600 is described below with reference to the cryoablation apparatus 1000 and the cryoablation system 1300 but it should be appreciated that other apparatuses and systems may be used.

The method 1600 may begin at step 1602. At step 1602, the cryoablation system 1300 may be ready to begin a cryoablation cycle. The cryoablation system 1300 may already have been activated or started operation. For example, the pressure control 1302 may already have been preparing the cryogen in the Dewar 1002 to maintain the cryogen at the low pressure condition in the Dewar 1002. The method 1500 may have been performed, for example, to pressurize the Dewar to the low pressure condition. When the Dewar 1002 is ready or the system has otherwise been primed, the method 1600 may proceed to step 1604. The pressure control 1302 may verify that the Dewar 1002 is operating as needed before moving to step 1604.

At step 1604, the pressure control 1302 may begin obtaining information regarding the operating conditions of the cryoablation system 1300. The pressure control 1302 may obtain information regarding a desired iceball size, growth rate, cycle duration or other information. Such information may be obtained from a treatment plan or may be received by the pressure control 1302 from another source such as user input, database, clinical procedures or the like. The method 1600 may then proceed to step 1606.

At step 1606, the pressure control may receive pressure information regarding a pressure of cryogen at the outlet of the pump assembly 1014 or at the outlet of the lid assembly 1322. The pressure control 1302 may receive pressure information from the sensor 1320 positioned at an outlet of the lid assembly 1322. The method 1600 may proceed to step 1608.

At step 1608, the pressure control 1302 may obtain other information regarding a performance of the cryoablation system 1300. The information may include the information previously described at step 1604. The information may include temperature and pressure information at the cryoprobe 128 that may indicate a growth of the iceball at the cryoprobe 128. This information may be compared to optimized or desired operating parameters. The optimized or desired operating parameters may be determined using algorithms, look-up tables, models or the like that were previously described. In some examples, the operating information is input to a trained machine learning model that may output the optimized or desired operating parameters of the cryoablation system 1300. The pressure control 1302 may obtain or determine a lower high pressure threshold and/or an upper high pressure threshold. The lower high pressure threshold and the upper high pressure threshold may define a desired high pressure range that is desired for the cryogen when the cryogen flows through the cryoprobe to produce an iceball of desired size, growth rate, or other characteristics.

At step 1610, the pressure control 1302 may determine if the pressure of the cryogen at the pump outlet is less than the lower high pressure threshold. If the pressure at the pump outlet, as measured by the sensor 1320 for example, is less than the lower high pressure threshold, the method 1600 may move to step 1612. If the pressure control 1302 determines that the pressure at the pump outlet is not less than the lower high pressure threshold, the method 1600 may move to step 1618.

At step 1612, the pressure control 1302 may activate or turn on the pump. The pressure control 1302 may, for example, turn on the pump in the pump assembly 1014. The pump assembly 1014 may pressurize the cryogen in the supply tube 1006 that is supplying the cryogen to the cryoprobe 128. The method 1600 may then move to step 1614.

At step 1614, the pressure control 1302 may determine whether the pressure of the cryogen is increasing at rate sufficient for purposes of the cryogen treatment. For example, if the pressure is increasing at a rate sufficient to allow the cryoablation treatment to successfully complete within the desired time, the pressure control 1302 may determine that the pressure increase is sufficient. If the pressure control 1302 determines that the pressure increase is too slow such that the iceball will not reach a desired size within the specified time, the pressure control 1302 may deem the pressure increase insufficient. Such determination may be made using the treatment plan, algorithms, machine learning models, or the like. If the pressure increase is determined to be sufficient, the method 1600 moves back to step 1606, whereby the pressure control 1302 continues to monitor the operation of the cryoablation system 1300. If the pressure increase is determined to be insufficient, the method 1600 may move to step 1616.

At step 1616, the pressure control 1302 may adjust or modify the operating parameters of the cryoablation system 1300 to change the pressure of the cryogen. For example, the pressure control 1302 may change, increase, or adjust the speed, or duty cycle of power signal delivered to the pump. Such modification may increase the rate of pressure increase of the cryogen. The nature, size, amplitude, or details of the change to the operation of the pump may be determined by the pressure control 1302 using an algorithm, look-up table, database, known relationship, trained model or the like. The method 1600 may then proceed back to step 1606 where the steps are re-performed and the pressure control 1302 continues to monitor the operation of the cryoablation system 1300.

At step 1618, the pressure control 1302 may determine if the pressure of the cryogen at the outlet of the pump is greater than the upper high pressure threshold. The pressure control 1302 may make this determination by comparing the pressure of the cryogen received from the sensor 1320 to the upper high pressure threshold. If the pressure is greater than the upper high pressure threshold, the method 1600 may move to step 1620. If the pressure is not greater than the upper high pressure threshold, the method 1600 may move to step 1626.

At step 1620, the pressure control 1302 may deactivate or reduce a duty cycle delivered to the heater assembly. The pressure control 1302 may have been pressurizing the cryogen and now the feedback from the system indicates that the pressure is too high. The pressure control 1302 may take action at step 1620 to reduce the pressure or reduce the pressure increase. The method 1600 may then move to step 1622.

At step 1622, the pressure control 1302 may determine if the pressure is still increasing at the pump outlet. The pressure control 1302 may make this determination from the pressure information received from the sensor 1320, for example. If the pressure at the pump outlet is still increasing, the method 1600 may move to step 1624. If the pressure at the pump outlet is not increasing, the method 1600 may return to step 1606 where the steps are re-performed and the pressure control 1302 continues to monitor the operation of the cryoablation system 1300.

At step 1624, the pressure control 1302 may take further action to reduce the pressure of the cryogen. The pressure control 1302 may, for example, open the valve in the valve assembly 1016. The pressure control 1302 may take such action to vent the Dewar 1002 to reduce the pressure of the cryogen. In other instances, the pressure control may take other action such as further reducing a duty cycle to the pump and/or opening other valves or vents that may be included in the cryoablation system 1300. The method 1600 may then return to step 1606 where the steps of method 1600 are re-performed and the pressure control 1302 continues to monitor the operation of the cryoablation system 1300.

At step 1626, the pressure control 1302 has determined that the pressure at the pump outlet is within a desired operating range (e.g., between the lower and upper high pressure thresholds). The method 1600 may return to step 1606 where the steps of method 1600 are re-performed and the pressure control 1302 continues to monitor the operation of the cryoablation system 1300. As can be seen, the method 1600 provides a closed-loop feedback system whereby the pressure of the cryogen that is delivered to a cryoprobe can be continuously or semi-continuously monitored and adjusted. The adjustments to the operation of the cryoablation system 1300 may be performed automatically based on experimental data and/or historical data. In some examples, the trained machine learning models can be continually updated using treatment data obtained during cryoablation treatments. Method 1600 allows for the optimization of the efficiency and effectivity of the cryoablation system 1300 to deliver improved performance and reduced costs over existing systems.

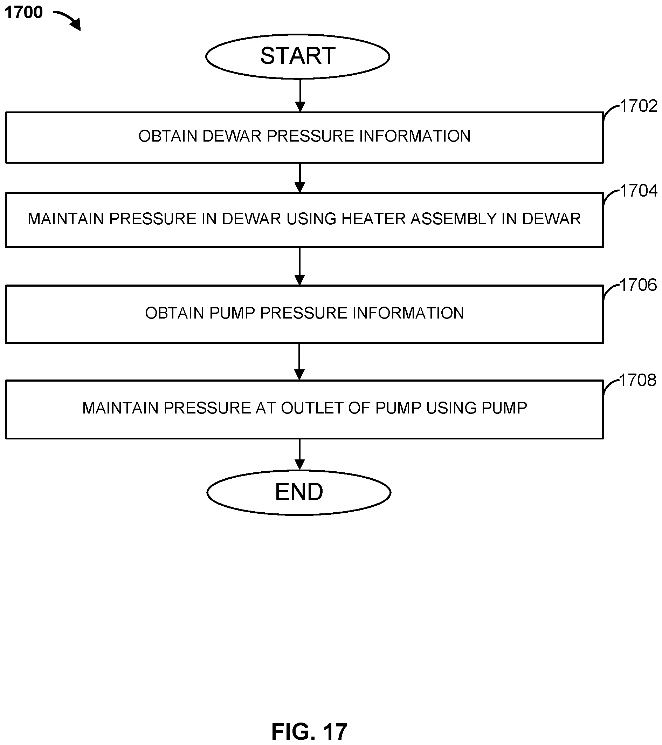

Referring now to FIG. 17, an example method 1700 of providing cryogen to a cryoprobe is provided. The example method 1700 may be performed using the apparatuses and systems of the present disclosure. For example, the cryoablation system 1300 may be used to perform the method 1700. The cryoablation system 1300 is referred to in the description below for illustration purposes only and the description should not be interpreted as limiting the performance of the method 1700 with respect to a particular system of apparatus.

The method 1700 may begin at step 1702. At step 1702, the pressure control 1302 may obtain Dewar pressure information. The Dewar pressure information may characterize a pressure of the cryogen in the Dewar 1002. The pressure information may be obtained by one or more pressure sensors such as sensors 1008 that may be positioned on the supply tube 1006 and/or in the Dewar 1002. The pressure information may be used to determine whether the pressure in the Dewar is within a first predetermined operating range. Such predetermined operating range in the Dewar may correspond to a low pressure range such as about 10 psi to about 15 psi for liquid nitrogen.

At step 1704, the pressure control 1302 may maintain the pressure of the cryogen in the Dewar 1002 within the first predetermined operating range. The pressure control 1302 may use the heating assembly 1012 as previously described. The pressure control 1302 may activate or deactivate the heater in the heating assembly 1012 to convert liquid cryogen to cryogen vapor in the Dewar 1002. The pressure control 1302 may use various methods such as algorithms, predetermined relationships, databases, look-up tables, mathematical relationships, machine learning models or the like to determine what actions should be taken to maintain the pressure in the Dewar to within the first predetermined operating range.

At step 1706, the pressure control 1302 may obtain pump pressure information. The pump pressure information may characterize a pressure of the cryogen at an outlet of the pump. The pump pressure information may be obtained from the sensor 1320, for example. The pump pressure information may be used to determine whether the pressure at the outlet is within a second predetermined operating range. Such predetermined operating range at the pump outlet may correspond to a high pressure range such as about 400 psi to about 600 psi for liquid nitrogen.

At step 1708, the pressure control 1302 may maintain the pressure of the cryogen at the outlet of the pump to a pressure within the second predetermined operating range. The pressure control 1302 may activate or deactivate a pump of the pump assembly 1014 or the lid assembly 1322 to increase the pressure of the cryogen at the outlet of the pump. The pressure control 1302 may modify pump speed, pump pulse frequency, amplitude or other characteristic of the flow to modify the pressure. The pressure control 1302 may use various methods such as algorithms, predetermined relationships, databases, look-up tables, mathematical relationships, machine learning models or the like to determine what actions should be taken to maintain the pressure at the outlet to a pressure within the second predetermined operating range.

As can be appreciated, the steps 1702 to step 1708 may be continuously or semi-continuously be performed so that the pressure control 1302 may monitor and adjust the operating parameters of the cryoablation system 1300. Furthermore, the steps of maintaining the pressure in the Dewar and maintaining the pressure at the outlet of the pump may be performed simultaneously or in parallel during operation of the cryoablation system 1300. By maintaining the pressure of the cryogen in the Dewar at a desired operating pressure, the pressurization of the cryogen to the high pressure condition may be performed more efficiently and effectively than existing systems. Such operation results in improved performance over existing systems.

AUTOMATIC GAS CONVERSION AND PURGE

In some embodiments of the present disclosure, apparatuses and methods are provided that may be used to automatically control cryogen gas pressure, convert liquid cryogen to cryogen vapor, and may use such cryogen vapor for purposes other than producing the iceball at the cryoprobe. Such other purposes may include system cleaning, purging, thawing and pressure pushing.

Prior to the start of a cryoablation cycle to produce the iceball at the target tissue, it may be desirable to move a heated fluid, such as cryogen vapor, through the cryogen flow path to clean, purge, or otherwise prepare the cryogen flow path for the cryoablation cycle. Such movement of cryogen vapor through the flow path may also be used for a thaw process in the cryoablation cycle. The thaw process may raise the temperature of the cryoprobe to allow the cryoprobe to remove from the iceball that may have formed during the cryoablation cycle.

The apparatuses and methods of the present disclosure may also be used in a pre-chill process. During such a process, cryogen vapor may be passed through the cryogen flow path. The cryogen vapor used in the pre-chill process is not elevated to a temperature to thaw ice and instead, may be chilled cryogen vapor. The chilled cryogen vapor may be at a temperature lower than ambient temperature but may not be as low as the temperature of the cryogen that is used during the cryoablation cycle. This pre-chill process may lower the temperature of the cryogen supply lines and/or the cryoprobe prior to the cryoablation cycle. This pre-chill may allow the temperature of the cryogen to be lower when it reaches the cryoprobe than in other systems that begin the cryoablation cycle with the flow path (e.g., supply lines and cryoprobe) being at ambient temperature. With the pre-chill, the cryogen is able to remove heat from the target more quickly during early period of the cryoablation cycle so that the iceball may form more quickly than traditional cryoablation systems.

The pre-chill process may lower the temperature of the entire cryogen flow path and elements of the cryoablation apparatus. The console, cryogen flow path, valves, cryoprobes and other elements may be chilled to lower the temperature from an initial or ambient condition. The flow path is cooled to make the cryoablation treatment time less than may be otherwise required and can be operated more efficiently. In existing systems, liquid cryogen (e.g., liquid nitrogen) may be used to cool the cryoablation apparatus, using low pressure (50 psi) or high pressure (500 psi) liquid nitrogen. Such use of liquid cryogen, however, creates the risk of micro ice ball creation inside the cryogen flow path, particularly if there is moisture inside the cryogen flow path and/or probe. The pre-chilling processes of the present disclosure may use cryogen gas, such as nitrogen gas at about -20°C to about -80°C, to purge and cool the cryoablation apparatus. The pre-chill process reduces the risk of micro ice ball formation in the cryogen flow path resulting improvement over existing systems and methods.

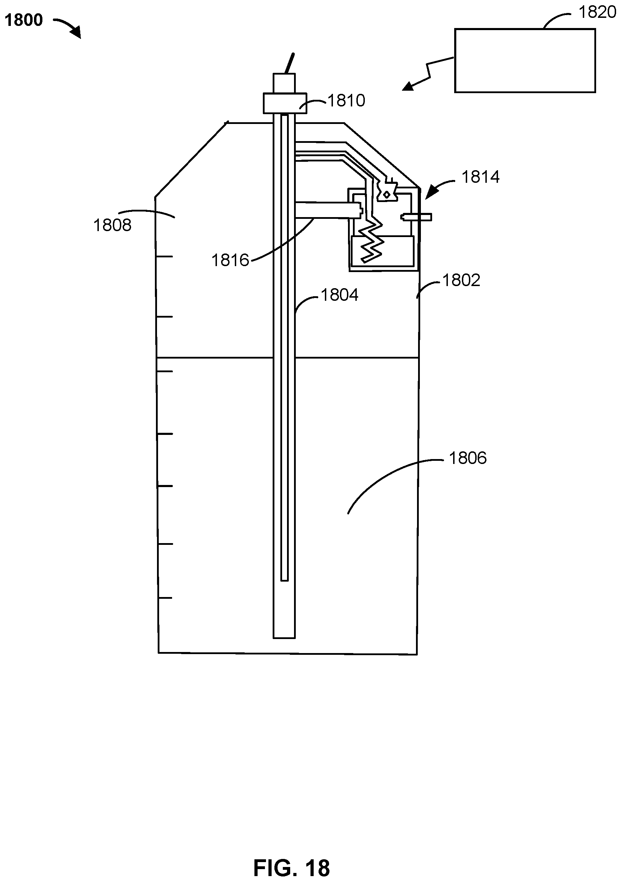

Turning now to FIG. 18, an example cryoablation apparatus 1800 is shown. The cryoablation apparatus 1800 may be included in a cryoablation system such as the cryoablation system 100 described above. The cryoablation apparatus 1800 may be coupled to a cryogen supply 114 and a cryoprobe 128 to provide cryogen to the cryoprobe 128 during a cryoablation treatment.

The cryoablation apparatus 1800 may include a Dewar 1802, a supply rod 1804, a lid assembly 1810 and a cryogen gas assembly 1814. The Dewar 1802 may be similar to the Dewars described above and may be a chamber defining an interior volume to retain a volume of cryogen. The cryogen may various suitable cryogenic fluids such as nitrogen, helium, argon, and the like. The Dewar 1802 may include a volume of liquid cryogen 1806 and a volume of cryogen vapor 1808.

The supply rod 1804 may extend into the interior volume of the Dewar 1802. A distal end of the supply rod 1804 may extend toward a base of the Dewar 1802 and extend into the liquid cryogen 1806. The supply rod 1804 may be a hollow member or include an inner cavity or tubing to allow liquid cryogen to be pumped from the Dewar 1802 to the cryoprobe 128. Lid assembly 1810 may include a controllable pump motor and/or pump assembly to pump the liquid cryogen 1806 through the supply rod 1804. The lid assembly 1810 may pressurize the cryogen when the cryogen is supplied to the cryoprobe 128 to create the iceball at the cryoprobe 128 during a cryoablation cycle. The supply rod 1804 may also be fluidly connected to the cryogen gas assembly 1814. The connector 1816 may be a tube, conduit or other member to allow liquid cryogen 1806 to be dispensed into the cryogen gas assembly 1814. A suitable pump may be used to automatically dispense liquid cryogen 1806 into the cryogen gas assembly 1814. A liquid level sensor may be used to determine when liquid cryogen 1806 needs to be dispensed into the cryogen gas assembly 1814.

As shown, the cryogen gas assembly 1814 may be positioned in the internal volume of the Dewar 1802. The cryogen gas assembly 1814 may be positioned toward a top of the Dewar 1802 and/or in the portion of the Dewar 1802 that is typically filled with the cryogen vapor 1808. In other examples, the cryogen gas assembly 1814 may be positioned outside the Dewar 1802. In one example, the cryogen gas assembly 1814 may be positioned in the cryogen flow path, such as downstream of the lid assembly 1810 along or fluidly connected to the cryogen supply 114 (FIG. 1) between the Dewar 1802 and the cryoprobe 128.

As further shown, the cryoablation apparatus 1800 may include a gas control 1820. The gas control 1820 may be coupled to the lid assembly 1810, the cryogen gas assembly 1814, and to other aspects of the cryoablation apparatus 1800. The gas control 1820 may be a computing device, PLC, controller or other suitable device. The gas control 1820 may include the elements included in the computing device 900, for example. The gas control 1820 may be coupled to the elements of the cryoablation apparatus 1800 via wired or wireless connections to send and receive information and/or signals from the elements. The gas control 1820 may operate to control the operating parameters of the cryoablation apparatus 1800 and/or to obtain data or information regarding the operation of the cryoablation apparatus 1800. While not shown, the cryoablation apparatus may include one or more sensors position in the Dewar 1802, along the supply rod 1804, in the lid assembly 1810 and/or along the flow path of the cryogen. The sensors may obtain information such as temperature, pressure, flow rate, flow volume, and other information.

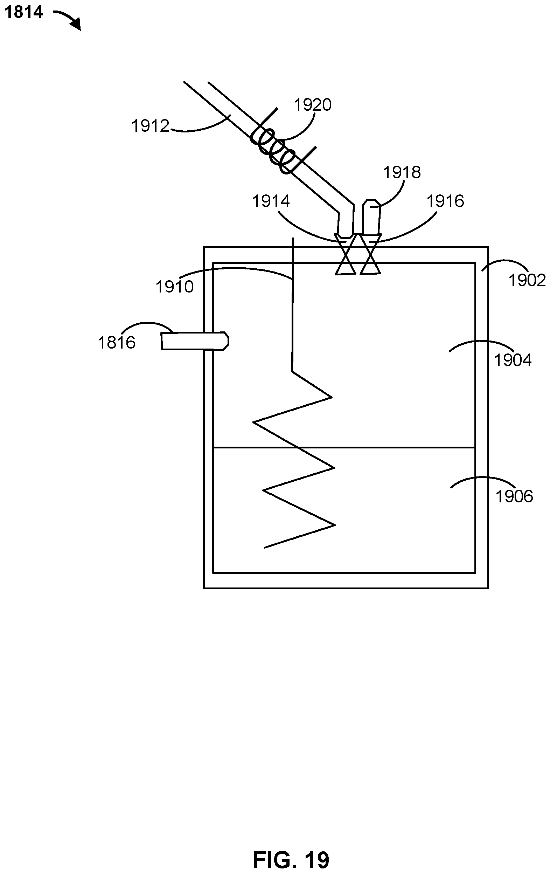

Further aspects of the example cryogen gas assembly 1814 are shown in FIG. 19. The cryogen gas assembly 1814 may include a housing 1902. The housing 1902 may define a cavity that may be used to retain liquid cryogen 1906 and cryogen gas 1904. The housing 1902 may be a thermally insulated heating and pressure temperature. The housing 1902 is preferably insulated to allow the conditions of the cryogen in the housing 1902 to be set to preferred operating ranges as may be needed to perform the particular action, such as cleaning, purging, pre-chilling, thawing, etc. The housing 1902 may be insulated using a double-walled structure, insulating materials, a vacuum layer, or the like. By positioning the housing 1902 in the Dewar 1802, the cryoablation apparatus 1800 may operate more efficiently than if positioned at other positions outside the Dewar 1802.

The cavity of the housing 1902 may be fluidly connected to a flow conduit 1912 and a valve assembly 1918. The flow conduit 1912 may fluidly connect the interior cavity of the housing 1902 with the cryogen supply 114. The flow conduit 1912 may include a conduit heater 1920. The conduit heater 1920 may be positioned in the flow conduit 1912 and allow cryogen gas that may be dispensed to the cryogen flow path to be heated to desired temperature. It may be desired, for example, to use the cryogen gas for a thaw cycle. In such an instance, the conduit heater 1920 may be controlled/activated by the gas control 1820 to heat the cryogen gas to a desired temperature. The conduit heater 1920 may be a resistive heater, laser heater, radio frequency (RF) heater, or other suitable heaters.

The cryogen gas may flow from the housing 1902 through the flow conduit and through the cryogen flow path and the cryoprobe 128. A controllable flow valve 1914 may be opened or closed to variably control the flow of the cryogen gas 1904 that exits the housing 1902. The valve assembly 1918 may include a vent valve 1916. The vent valve 1916 may also be a controllable valve that may be opened or closed to variably control a venting of cryogen gas from the interior cavity of the housing 1902. The flow valve 1914 and the vent valve 1916 may each be coupled to the gas control 1820. The gas control 1820 may operate and/or adjust a flow of cryogen gas in order to supply a desired flow of cryogen gas or to vent cryogen gas to control a pressure in the housing 1902.

The cryogen gas assembly 1814 may also include a heater 1910. The heater 1910 may extend in the housing 1902 and may extend from a top of the housing toward a bottom of the housing 1902 to contact or thermally engage the liquid cryogen 1906. The heater 1910 may include a controllable heating element such as a resistive heater, laser heater, radio frequency (RF) heater or the like. The heater 1910 may heat the liquid cryogen to convert the liquid cryogen 1906 to cryogen gas 1904. The heater 1910 may be used in such a manner to produce cryogen gas 1904 and to raise the pressure of the housing 1902 to desired pressure. The heater 1910 may also heat the cryogen gas to a desired temperature. The heater 1910 may be coupled to the gas control 1820. The gas control 1820 may interface with the heater 1910 to activate, deactivate or otherwise variably deliver a power signal to the heater 1910 to case the heater to energize. The gas control 1820 may provide various duty cycles to the heater 1910 such as variable frequencies, amplitudes, pulse width modulation (PWM), and the like.

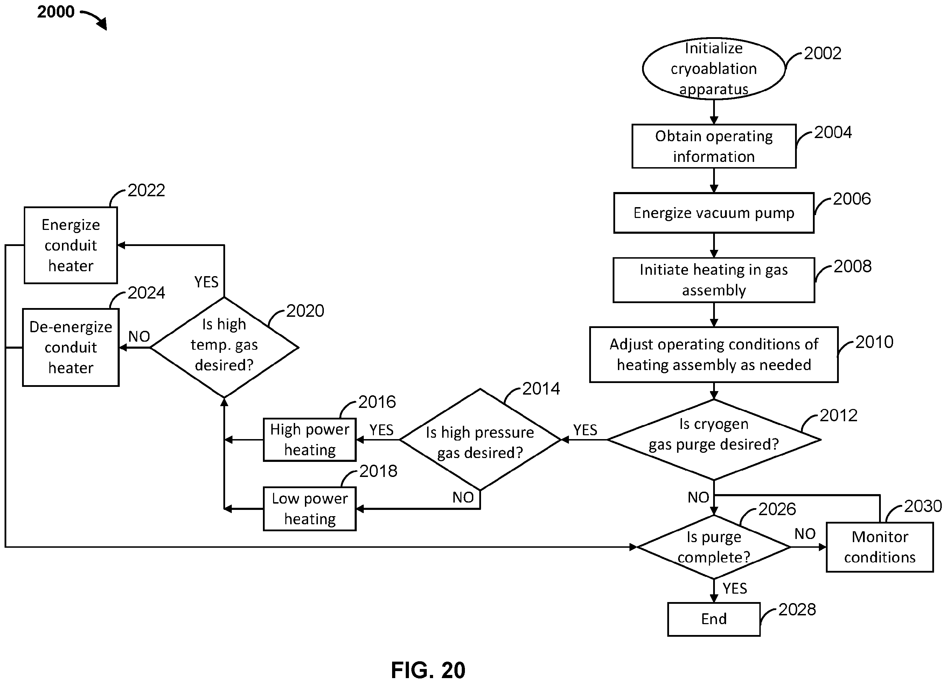

Referring now to FIG. 20, a method 2000 of providing cryogen gas to a cryoablation flow path is provided. The method 2000 may be performed using the cryoablation apparatus 1800, for example. The description below described the method 2000 performed by the cryoablation apparatus 1800. Reference may also be made to the cryoablation system 100. It should be appreciated, however, that the method 2000 is not limited to being performed by these apparatuses and systems. Other systems and apparatuses may be used, including other systems and apparatuses of the present disclosure.

The method 2000 may begin at step 2002. At step 2002, the cryoablation apparatus 1800 may initialize and the gas control 1820 may determine and/or check whether all elements are functioning as intended. Step 2002 (and the method 2000) may be performed, for example, prior to the start of a cryoablation cycle. Step 2002 and method 2000 may also be performed after a cryoablation cycle, when a thaw cycle is desired or when maintenance is needed for the cryoablation apparatus 1800.

The method 2000 may proceed to step 2004. At step 2004, the gas control 1820 may obtain operating information. The operating information may include information or data that characterizes operating conditions at various locations of the cryoablation apparatus 1800. The operating conditions may include, for example, pressures, temperatures, flow rates, etc. The operating conditions may include a pressure and temperature of the Dewar 1802, a pressure and temperature of cryogen gas assembly 1814, and/or a pressure and temperature of in the supply rod 1804 or at an outlet of the lid assembly 1810. The gas control 1820 may additionally obtain operating information for other locations in the flow path of the cryoablation system.

The method 2000 may proceed to step 2006. At step 2006, a vacuum pump may be energized. While not shown in FIG. 18, the cryoablation apparatus 1800 may include a vacuum pump and the vacuum pump may be coupled to the gas control 1820. The vacuum pump may operate to provide an insulated layer between the housing 1902 of the cryogen gas assembly 1814 and the Dewar 1802. The gas control 1820 may energize the vacuum pump to evacuate an insulation layer of the housing 1902.

The method 2000 may proceed to step 2008. At step 2008, the gas control 1820 may initiate heating in the cryogen gas assembly 1814. The gas control 1820 may cause a power signal with a desired duty cycle to be delivered to the heater 1910 of the cryogen gas assembly 1814. The heater 1910 may begin heating of the liquid cryogen 1906 to convert the liquid cryogen 1906 to cryogen has 1904.

The method 2000 may proceed to step 2010. At step 2010, the gas control 1820 may monitor the operating conditions of the cryogen gas assembly 1814 and continue to produce cryogen gas and/or to vent cryogen gas to achieve desired operating conditions in the housing 1902 of the cryogen gas assembly 1814. The process for performing this maintenance may be a closed-loop feedback process whereby the gas control 1820 receives information regarding the cryogen gas and the conditions of the cryogen gas assembly 1814, such as temperature, pressure, power signal to the heater 1910, etc. This information may be used to adjust the operation of the heater 1910 and/or the flow valve 1914 and/or the vent valve 1916. The gas control 1820 may maintain the conditions of the cryogen gas within predetermined operating levels or ranges.

The method 2000 may proceed to step 2012. At step 2012, the gas control 1820 may determine whether a cryogen gas purge is needed. The gas control 1820 may determine whether a purge, clean, thaw, pre-chill, or other cryogen gas flow process is needed. This information may be obtained by the gas control 1820 according to a treatment plan or from clinical treatment procedures. For example, a flush or purge may be indicated before every cryoablation cycle or upon start-up or after conclusion before storage of the system. The information regarding the need or desire for a purge may also be obtained via a user interface. In still other examples, the gas control 1820 may obtain the information as a result of one or more sensors that may indicate that a purge or cleaning is needed. As detailed below, the gas control 1820 or other computing device may execute a diagnostics step whereby the need for a purge process may automatically be determined. If the gas control 1820 determines that purge process is desired, the method 2000 moves to step 2014. If the gas control 1820 determines that purge process is not desired, the method 2000 moves to step 2026.

At step 2014, the gas control 1820 may determine if a high pressure cryogen gas is required for the purge process. The gas control 1820 may automatically determine if high pressure gas is required according to the information described above including the treatment plan, clinical procedures, input from a user or elsewhere. High pressure gas may be needed for particular types of purge process such as instances in which a blockage is detected or determined to exist in the cryogen flow path. The high pressure gas may be used to clean and remove blockages. A high pressure gas may be a gas at a pressure in a range of about 100 psi to about 1000 psi. A low pressure gas may be a gas in a range of about 10 psi to about 50 psi. A low pressure gas may be used in a purge process for cleaning and no blockage is detected or determined to exist. If the gas control 1820 determines that high pressure gas is desired, the method moves to step 2016. If the gas control 1820 determines that high pressure gas is not desired, the method 2000 proceeds to step 2018. At step 2016, the gas control 1820 may energize the heater 1910 using a high power mode to increase the conversion of liquid cryogen to cryogen gas to increase the pressure in housing 1902. At step 2018, the gas control may energize the heater 1910 using a lower power mode to convert the liquid cryogen to cryogen gas at a lower rate than the high power mode. The low power mode versus the high power mode may use different duty cycles, voltages, currents, or PWMs that may be controlled by the gas control 1820.

After steps 2016 and 2018, the method 2000 may proceed to step 2020. At step 2020, the gas control 1820 may determine whether high temperature gas is desired. The gas control 1820 may determine whether high temperature gas is desired similarly to whether high pressure gas is desired. The information may be input by a user or may be detailed in a treatment plan or clinical procedures. In other instances, the gas control 1820 may automatically determine whether high temperature gas is desired based on a diagnostic procedure or information obtained from one or more sensors of the cryoablation apparatus 1800. If the gas control 1820 determines that high temperature gas is desired, the method 2000 proceeds to step 2022. If the gas control 1820 determines that high temperature gas is not desired, the method proceeds to step 2024.

A desired temperature of the gas may be based on the type of procedure being performed. A thaw procedure may use a temperature of about 40°C to about 60°C. A coagulation and/or a cauterization procedure may use a temperature of about 80°C to about 120°C. The temperature may also vary with a pressure of the cryogen gas. The pressure and temperature used during a procedure may also vary with clinical needs. In some examples, a cryogen gas may be used in a pressure range of about 10 psi to about 50 psi with a temperature of about 10°C to about 40°C for a thaw procedure. These conditions may be utilized to provide a quick time to remove the needle from the iceball. In some circumstances, cryogen gas may be used in a pressure range of about 10 psi to about 50 psi with a temperature of greater than about 50°C for a coagulation or cauterization procedure. Such operation may reduce the procedure to a time of about 10 to about 15 seconds. This may be particularly useful if the needle needs to be repositioned during a treatment. This reduced time may reduce a total treatment time from about 2 hours to about 30 minutes.

At step 2022, the gas control 1820 may turn on the conduit heater 1920 . The conduit heater 1920, as described above, may be located in the flow conduit 1912. The flow conduit 1912 may fluidly connect the cryogen gas apparatus 1814 to the cryogen flow pathway. The gas control 1820 may activate the conduit heater 1920 to heat the cryogen gas as it is passed to the cryogen flow pathway during a purge cycle. The gas control 1820 may activate the heater with various power signals, duty cycles and the like to heat the cryogen gas to a desired temperature. The gas control 1820 may obtain temperature information from one or more sensors positioned in the flow conduit 1912 and modify or adjust the power supplied to the conduit heater 1920 to adjust the cryogen gas temperature to a desired temperature or desired temperature range.

As step 2024, the gas control 1820 may deactivate the conduit heater 1920 and/or deliver a low level power signal to the conduit heater 1920. The gas control 1820, similarly to that described above, may obtain temperature information from one or more sensors positioned in the flow conduit 1912 and modify or adjust the power supplied to the conduit heater 1920 to adjust the cryogen gas temperature to a desired temperature or desired temperature range. The temperature of the cryogen gas at step 2024 is a lower temperature cryogen gas than the high temperature gas provided by step 2022.

At step 2026, the gas control 1820 may determine whether the purge process is complete. The purge process may be for a certain duration or for a certain volume of cryogen gas. In other instances, the gas control 1820 may determine whether the purge process is complete by obtaining such information from the treatment plan, clinical procedures, input from a user, or automatically by measuring operating or diagnostic information from one or more sensors. If the gas control 1820 determines that the purge process is complete, the method moves to step 2028. If the gas control determines that the purge process in not complete, the method moves to step 2030.

At step 2030, the gas control 1820 may determine whether the conditions in the housing 1902 of the cryogen gas assembly 1814 meet predetermined cryogen gas conditions. The particular purge process that is being performed (e.g., clean, thaw, pre-chill, etc.) may indicate a particular set of cryogen gas conditions such as temperature, pressure, volume, etc. The gas control 1820 may obtain information regarding such conditions and determine whether the cryogen gas conditions meet or are sufficient according the particular purge process that is to be performed.

At step 2030, the gas control 1820 may also take action to modify or adjust the conditions of the cryogen gas. The gas control 1820 may determine, for example, that the pressure in the housing 1902 is too high. The gas control 1820 may vent pressure from the housing 1902 using the vent valve 1916. In another example, the gas control 1820 may determine that the cryogen gas pressure is too low. The gas control 1820 may increase the power delivered to the heater 1910 to increase the production of cryogen gas to increase the pressure. In other instances, the gas control 1820 may take other action. In this manner, the gas control 1820 may monitor the operation of the cryoablation apparatus 1800 and continually adjust the parameters of the system, as needed, to perform an efficient and effective purge process.

At step 2028, the gas control 1820 may stop heating the gas in the cryogen gas assembly 1814 and may deactivate or de-energize the conduit heater 1920. The cryoablation may continue if further cryoablation cycles are prescribed. If no further cycles are needed, the method 2000 may end.

As can be appreciated, the method 2000 may be performed multiple times during a cryoablation treatment. For example, the method 2000 may be performed before the first cycle is performed to purge or clean the system. The method 2000 may be performed again to perform a pre-chill process. The method 2000 may be performed again at the end of the cryoablation treatment to purge the system.

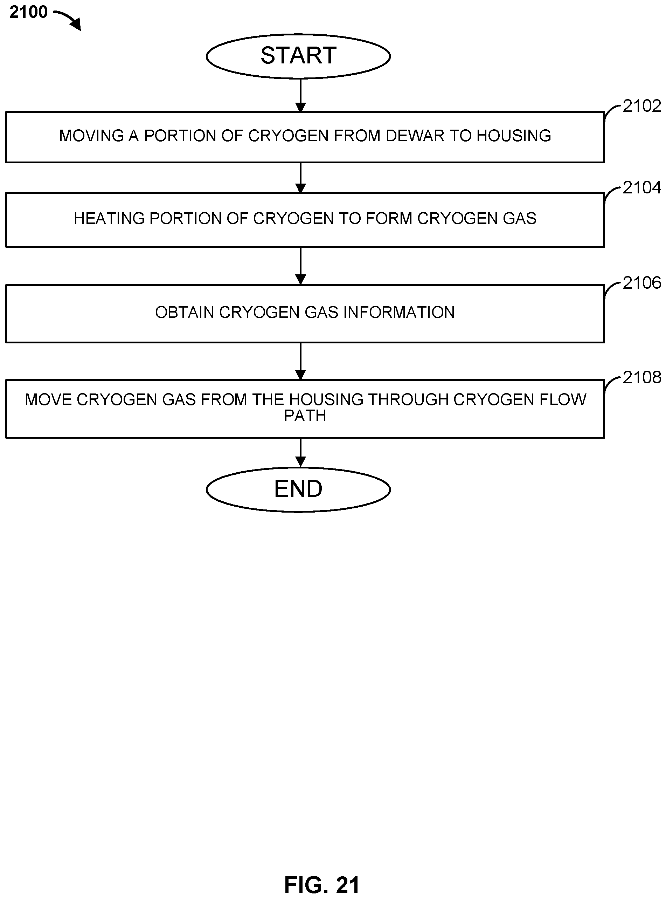

Referring now to FIG. 21, another example method 2100 of performing a purge process is provided. The method 2100 may be performed by the apparatuses and/or systems of the present disclosure. The method 2100 may also be performed by other apparatuses and/or systems. The method 2100 is described below as being performed by the cryoablation apparatus 1800 but it should be appreciated that the steps and/or operations may be performed by other apparatuses and/or systems.

The method 2100 may begin at step 2102. At step 2102, the gas control 1820 may move a portion of liquid cryogen from the Dewar 1802 to the housing 1902 of the cryogen gas assembly 1814. The liquid cryogen may be moved into the housing 1902 using a pump, for example. A conduit may fluidly connect the internal volume of the Dewar 1802 with the housing 1902. The supply rod 1804 may be part of the pathway as shown in FIG. 18. The connector 1816 may fluidly connect the supply rod 1804 to the cryogen gas assembly 1814. The gas control 1820 may cause the liquid cryogen to move from the Dewar 1802 into the housing 1902. A liquid level sensor or other measurement device may indicate when the liquid cryogen needs to be moved into the housing 1902. The gas control 1820 may automatically maintain a predetermined cryogen liquid level in the housing 1902, for example.

At step 2104, the liquid cryogen in the housing 1902 may be heated. The gas control 1820 may, for example, activate the heater 1910 by causing a suitable power signal to be delivered to the heater 1910. The heater 1910 may be in contact with the liquid cryogen or otherwise thermally engage the liquid cryogen to cause liquid cryogen to be converted to cryogen gas in the housing 1902.

At step 2106, the gas control 1820 may obtain cryogen gas information. The gas control 1820 may obtain cryogen gas information that characterized the conditions of the gas cryogen in the housing 1902. The cryogen gas information may include a temperature and pressure of the cryogen gas. The gas control 1820 may compare such cryogen gas information to predetermined thresholds, ranges, or other desired characteristics of the cryogen gas. The desired characteristics of the cryogen gas may be dictated by the treatment plan, clinical procedures, user preferences, and/or automatically by the gas control 1820. The desired characteristics may be predetermined based on a type of purge process to be provided such as a cleaning, purge, thaw, pre-chill, or other.

The gas control 1820 may determine whether the measured cryogen gas information corresponds or is sufficient for the purge process. If not, the gas control may take action to increase/decrease pressure or temperature, for example. If the gas control 1820 determines that the cryogen gas is suitable for the desired purge process, the method may continue to step 2108.

At step 2108, the gas control 1820 may move the cryogen gas from the housing 1902 into the cryogen flow path. The cryogen flow path may include the cryogen supply 114 and/or the cryoprobe 128. The cryogen gas may clean, clear, thaw, pre-chill, or otherwise purge this cryogen flow path. The gas control 1820 may open the flow valve 1914, for example, to allow cryogen gas to flow to the cryogen flow path. In some instances, the gas control 1820 may also heat the cryogen gas when it flows the cryogen flow path. The gas control 1820 may heat the cryogen gas using the conduit heater 1920, for example.

The apparatuses and methods described above may provide a cryogen gas apparatus or assembly that can provide improvements over known or existing devices. The cryogen gas assembly 1814, for example, may integrated into the cryoablation system and provide automatic control and closed-loop feedback processes to provide a source of cryogen gas suitable for a particular purge process. The purge process may be provided to prepare the cryoablation system for a cryogen ablation treatment and/or to prepare the cryoablation system for storage following a cryoablation treatment. Still further, the cryoablation gas assembly 1814 may be used during the cryoablation treatment to provide a thaw process or to address a blockage or other issue that may occur.

AUTOMATIC CRYOABLATION SYSTEM PERFORMANCE DETECTION AND DIAGNOSIS

In some embodiments of the present disclosure, the cryoablation systems, apparatuses, and methods of the present disclosure may provide automatic real-time performance detection and diagnosis. Such embodiments may include a diagnostics control that may be coupled to the cryoablation system. The cryoablation system may use various valves and cryogen sensors positioned at locations along a cryogen flow path. The diagnostics control may open and close the valves at positions along the cryogen flow path and obtain measurements regarding the operating parameters of the cryogen at various locations. This information (e.g., temperature and pressure) may be used to determine if the cryoablation system is performing properly and/or at desired levels for efficient and effective operation. The diagnostics control may be able to determine and/or anticipate if operating issues may be occurring such as detecting a location of a blockage or leak. The diagnostics control may also be able to determine or anticipate a possible issue (e.g., blockage or leak) by monitoring a rate of change of the operating characteristics of the cryoablation system.

The apparatuses and methods of the present disclosure are improvement over existing systems in that a location of a blockage or leak may be easily and readily determined. Existing system suffer from problems in that a use or operator may not be able to determine a location of a blockage, a leak or other issue. Existing system may require an entire system to be taken offline and removed from a treatment environment to determine a location or cause of an operating issue. The systems, apparatuses and methods of the present disclosure may allow an issue such as a blockage or leak to detected and corrected without the need for expensive, time-consuming, and possibly ineffective repairs.

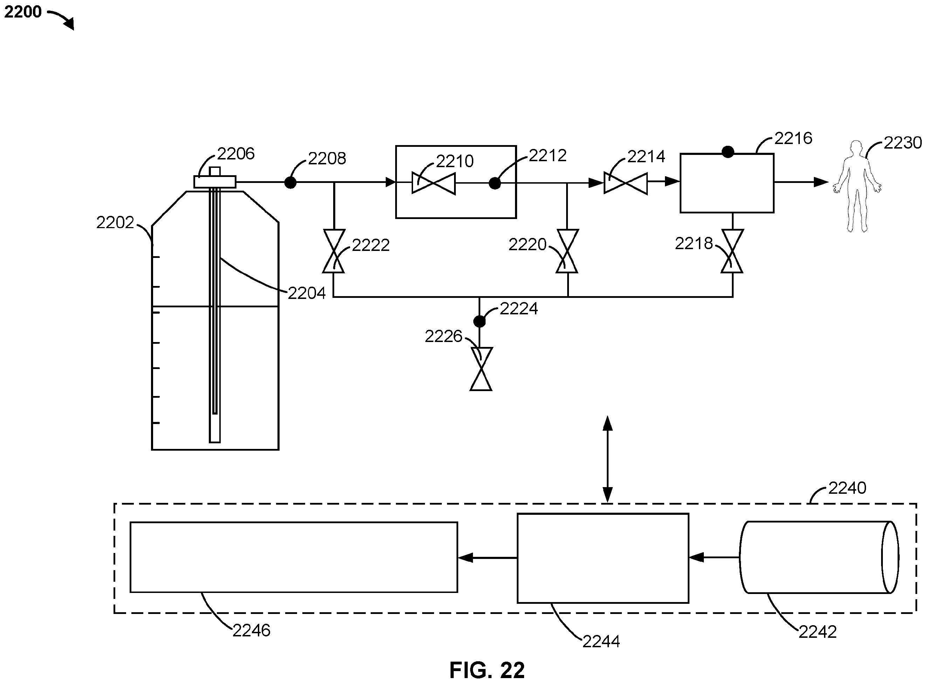

An example cryoablation system 2200 is shown in FIG. 22. The cryoablation system 2220 may include a Dewar 2202. The Dewar 2202 may include the structure and features previously described such as cryogen liquid level sensor, a cryogen rod 2204, and a lid assembly 2206. The Dewar 2202 may be suited to retain a volume of cryogen and move the liquid cryogen to a cryogen flow path to perform a cryoablation treatment. The liquid cryogen, for example, may be pumped from the Dewar 2202 to the cryogen supply 2230. The cryogen may flow through the cryogen supply 2230 to the cryoprobe 2216. The cryoprobe 2216 may positioned at or near a target tissue in the patient 2232.

The cryoablation system 2200 may also include multiple sensors and valves positioned at various locations along the cryogen flow path. The cryoablation system 2200 may include, for example, an outlet sensor 2208, a vacuum chamber sensor 2212, a probe sensor 2216, and a return sensor 2224. Each of the outlet sensor 2208, the vacuum chamber sensor 2212, the probe sensor 2216, and the return sensor 2224 may be configured to provide information regarding a flow of cryogen in the cryogen flow path. The outlet sensor 2208, the vacuum chamber sensor 2212, the probe sensor 2216, and the return sensor 2224 may provide pressure, temperature, flow, or other information. In one example, the outlet sensor 2208, the vacuum chamber sensor 2212, the probe sensor 2216, and the return sensor 2224 are temperature or pressure transducers. In other examples, the outlet sensor 2208, the vacuum chamber sensor 2212, the probe sensor 2216, and the return sensor 2224 may be other types of sensors. In other examples, the outlet sensor 2208, the vacuum chamber sensor 2212, the probe sensor 2216, and the return sensor 2224 may be thin-film pressure sensors, piezoelectric pressure sensors or the like.

The outlet sensor 2208 may be positioned at an outlet of the pump. The vacuum chamber sensor 2212 may be positioned at or near a vacuum chamber of the cryoablation system or may be positioned after a supply valve 2210 that is used to supply the cryogen to the cryogen flow path. The probe sensor 2216 may be positioned at or in the cryoprobe 2216. The return sensor 2224 may be positioned in a return line 2232 that may allow cryogen to return to the Dewar 2202 or be vented to atmosphere. The return line 2232 may also be connected to the supply 2230. The return line may allow a line to be fluidly connected at or between various elements of the cryoablation system for diagnostic purposes as will be further described below.

It should be appreciated that the cryoablation system may include more or other sensors than the outlet sensor 2208, the vacuum chamber sensor 2212, the probe sensor 2216, and the return sensor 2224 shown. In addition, each of the outlet sensor 2208, the vacuum chamber sensor 2212, the probe sensor 2216, and the return sensor 2224 may include multiple sensors. For example, there may be multiple probe sensors 2216 located at various locations along the cryoprobe 2216.

As further shown, the cryoablation system 2200 may include a supply valve 2210, a probe valve 2214, a first connector valve 2218, a second connector valve 2220, a third connector valve 2222, and a return valve 2226. The supply valve 2210 may positioned at or near an outlet of the pump. The supply valve 2210 may be used, for example, to allow cryogen to flow from the Dewar 2202 to the cryogen flow path. The probe valve 2214 may be used to allow cryogen to flow to the cryoprobe 2216. The first connector valve 2218 may positioned at a return of the cryoprobe 2216 and allow cryogen to flow from the cryoprobe 2216 to the return line 2232. The second connector valve 2220 may be positioned to fluidly connect the supply 2230 to the return line 2232 at a position between the Dewar 2202 and the cryoprobe 2216. The second connector valve 2220 may allow the cryoprobe 2216 to be excluded from the cryogen flow path for diagnostic purposes. The third connector valve 2222 may be positioned to fluidly connect the supply 2230 and the return line 2232 at a position between the Dewar and the return line upstream from the second connector valve. The third connector valve 222 may allow further portions to be excluded from the cryogen flow path such as the vacuum chamber. The return valve 2226 may be positioned between the return line 2232 and Dewar 2202. In some examples, the return valve 2226 may allow cryogen to be returned to the Dewar 2202. In other examples, the return valve 2226 may allow cryogen to be vented to the atmosphere after it returns from the cryoprobe 2216.

The supply valve 2210, the probe valve 2214, the first connector valve 2218, the second connector valve 2220, the third connector valve 2222, and the return valve 2226 may each be configured as a controllable flow valve that may be electronically controlled by a diagnostics control 2240. The supply valve 2210, the probe valve 2214, the first connector valve 2218, the second connector valve 2220, the third connector valve 2222, and the return valve 2226 may each be coupled to the diagnostics control 2240 via wired or wireless connections that can cause the supply valve 2210, the probe valve 2214, the first connector valve 2218, the second connector valve 2220, the third connector valve 2222, and/or the return valve 2226 to each be independently opened or closed as may be desired to perform diagnostics procedures.

The diagnostics control 2240 may be a suitable computing device, PLC, controller, or other device configured to perform the operations of present disclosure. In some examples, the diagnostics control 2240 may be a device such as the computing device 900 described above. The diagnostics control 2240 may be incorporated into and/or may be the same device as the other controls described above.

The diagnostics control 2240 may include a data acquisition module 2242 that may operate to obtain information regarding the operating conditions of the cryoablation system 2200. The diagnostics control 2240 may obtain data or signals from the outlet sensor 2208, the vacuum chamber sensor 2212, the probe sensor 2216, and/or the return sensor 2224, as well as from other sensors of the cryoablation system 2200. The data acquisition module 2242 may process the information and data and may further communicate and/or send such information to other aspects of the diagnostics control.

The diagnostics control 2240 may also include a diagnostics module 2244. The diagnostics module 2244 may operate to determine whether the cryoablation system 2200 is operating properly and/or as is expected. The diagnostics may include algorithms, models, trained machine-learning models, artificial neural networks (ANNs), look-up tables, or the like that may be used to determine where or what elements of the cryoablation system 2200 is operating as intended. Information such as clinical procedures, look-up tables, predetermined relationships, models, or the like may be obtained by the diagnostics module 2244.

The diagnostics control 2240 may also include the operations module 2246. The operations module 2246 may send and/or receive signals from the various elements of the cryoablation apparatus 2200 to perform functions to adjust, modify, and/or otherwise control the operation of the cryoablation system 2200. The operations module 2246 may cause various valves to open or close, may adjust or modify a pressure or temperature of cryogen, and/or may adjust a flow or flow rate of cryogen.

The diagnostics module 2244 may use various suitable methods to determine whether the cryoablation system 2200 is operating as intended. The diagnostics module 2244 may obtain pressure information, for example, from one or more locations in the cryoablation system 2200 and compare the pressures and/or rate of change of the pressures to determine the performance of the cryoablation system 2200. The diagnostics module 2244 may receive the pressure information from the data acquisition module 2242. The operations module 2246 may open and/or close various valves of the cryoablation and then compare the pressures at various locations along the cryogen flow path to determine if the cryoablation system 2200 is operating as intended.

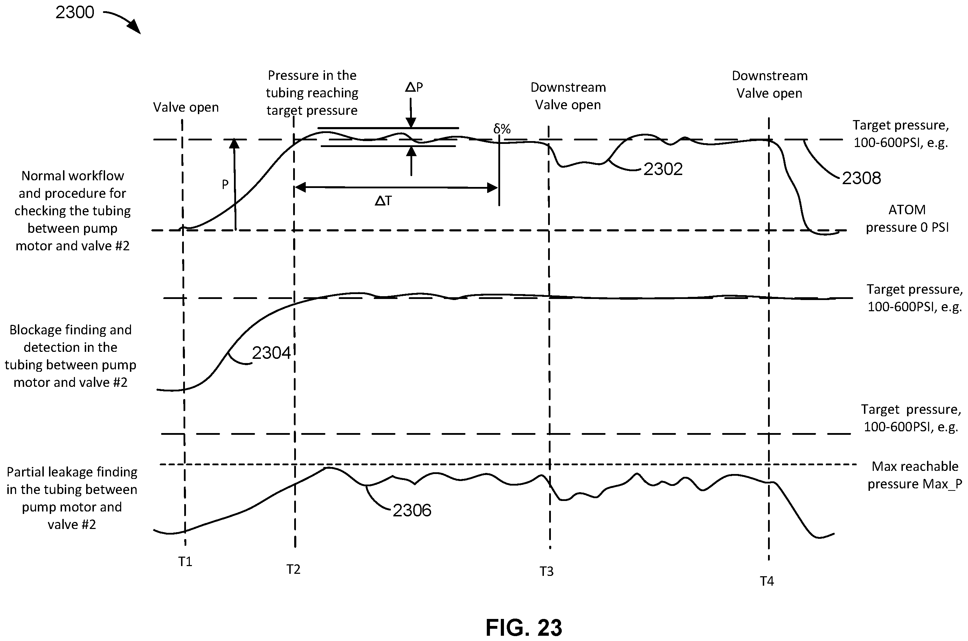

Referring now to FIG. 23, an example pressure output 2300 is illustrated as observed during an example diagnostics procedure. The pressure output 2300 may be output on a user interface of the diagnostics control 2240 in some examples. The diagnostics module 2244 may monitor the pressures shown in the pressure output 2300. In this example, pressure curves 2302, 2304, and 2306 are shown. The pressure curves are shown as a function of time during a diagnostics procedure. The first curve 2302 displays pressure data for a cryogen flow path that is operating without any issues. The second curve 2304 displays pressure data for a cryogen flow path that has a blockage in the cryogen flow path between the Dewar 2202 and the supply valve 2210. The third curve 2306 displays pressure data for a cryogen flow path that has a partial leak in the cryogen flow path between the Dewar 2202 and the supply valve 2210.

In this example, the cryoablation system 2200 may be ready to perform a cryoablation treatment. The diagnostics control 2240 may perform a diagnostics procedure to determine if there are any issues with the system such as leaks, blockages, or other issues that may interfere with the performance of the cryoablation treatment. In this example procedure, the diagnostics control 2240 may obtain pressure information from the supply sensor 2208. The cryogen may be initiated and flow from the Dewar toward the supply valve 2210. As shown, the pressure in the cryogen flow path between the Dewar 2202 and the supply valve 2210 increases over time and may reach a target pressure 2308. The target pressure 2308 may be defined as a threshold and/or a target pressure range $\Delta P$. The diagnostics control 2240 may monitor an amount of time that it takes the pressure in the cryogen flow path to reach the target temperature. The required time is shown as time period $\Delta T$. The time period time period $\Delta T$ may be determined using experimental or actual data and/or may be determined using a model. the diagnostics control 2240 may determine whether the time period $\Delta T$ is within a predetermined control tolerance range R%. If the time period $\Delta T$ is within R% of the time period $\Delta T$, the diagnostics control may determine that the cryoablation system 2200 is operating as intended. If there is a blockage, the pressure may reach the target pressure more quickly that it normally should. If there is a leak, the pressure may take longer or never reach the target pressure. In either instance, the diagnostics control 2240 may indicate to the user or otherwise communicate the potential problem for correction.

As shown in curve 2304, the pressure reaches the target pressure much more quickly than intended. In this case, the diagnostics control 2240 would see that the time period $\Delta T$ is much shorter than the target time period $\Delta T$ and outside the tolerance range R%. The blockage would be communicated to the user or corrective action would be taken such as a purge process or the like.

As shown in curve 2306, the pressure never reaches the target pressure. After the time period $\Delta T$ elapses, the diagnostics control 2240 would recognize that there is a problem and communicate the probe to the user.

If the diagnostics control determines that the cryoablation system 2200 is operating as intended. The diagnostics control 2240 may move on to another portion of the cryogen flow path and take similar steps as that previously described to determine if the pressure in the next portion of the cryogen flow achieves a target pressure in a target time period $\Delta T$. In this manner, the diagnostics control 2240 may determine whether each portion of the cryogen flow has a leak, blockage, or other issue. For example, the 2240 may open the supply valve 2210 to determine if the next downstream portion of the cryogen flow path is operating properly. The diagnostics control 2240 may then move on by opening the probe valve 2214 to check the next portion of the cryogen flow path. If at any time the diagnostics control 2240 determines that a blockage or leakage exists, the diagnostics control 2240 may open one or more of the connector valve 2218, 2220, 2222 to vent the cryogen from the system by opening return valve 2226.

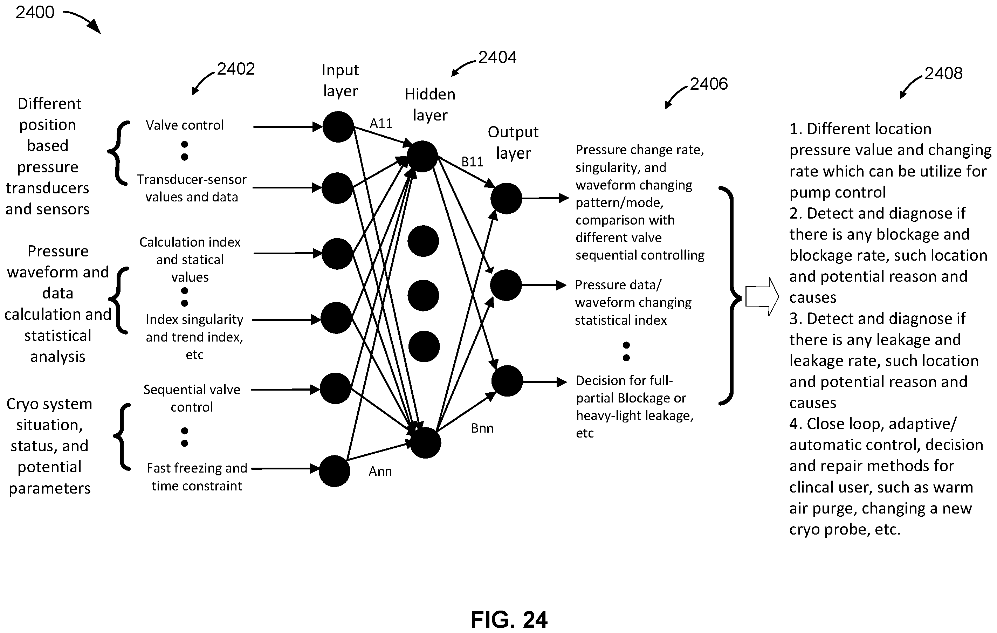

Referring now to FIG. 24, an example diagnostics model 2400 is illustrated. The model 2400 may be a machine learning model and may involve training a mathematical model in a supervised or unsupervised setting. Machine learning models may be trained to learn relationships between various groups of data. The models may be based on a set of algorithms that are designed to model abstractions in data by using a number of processing layers. The processing layers may be made up of non-linear transformations. Machine learning models may include, for example, neural networks, convolutional neural networks and deep neural networks. Such neural networks may be made of up of levels of trainable filters, transformations, projections, hashing, and pooling. The models may be used in large-scale relationship-recognition tasks. The models can be created by using various open-source and proprietary machine learning tools and/or libraries known to those of ordinary skill in the art.

The model 2400 may include inputs 2402, layers 2404, outputs 2406, and results 2408. The inputs 2402 for the model 2400 may include various items of data or information regarding the cryoablation system 2200. The inputs may include data regarding a status or setting of the valves of the cryoablation system 2200. The inputs 2402 may include one or more indexes or statistical values. The inputs may include various calculations or other numerical indexes, ratios or the like that may be determined from the data regarding operation of the cryoablation system 2200. The indexes may include pressure indexes and/or ratios such as the following.

Pressure index and ratio

$$Flucuation\ index \quad X = \frac{\Delta P}{P_{target}}$$

$$Flucuation\ ratio \quad X = \frac{\frac{\Delta P}{P_{target}}}{\partial\%}$$

$$Flucuation\ pressure\ time\ ratio \quad X = \frac{\Delta P}{\Delta T}$$

$$Pressure\ Singularity\ index\_1\ \ X = \frac{Max(P)}{\Delta P}$$

$$Pressure\ Singularity\ index\_2\ \ X = \frac{Target(P)}{\Delta P}$$

$$Pressure\ trend\ index\ \ X\_1 = \frac{Max(P)}{Target(P)}$$

$$Pressure\ trend\ index\ \ X\_2 = \frac{Max(P)\_before - release - valve}{Max(P)\_after - release - valve}$$

$$Pressure\ trend\ index\ \ X\_2 = \frac{\Delta(P)\_before - release - valve}{\Delta(P)\_after - release - valve}$$

where P is the pressure in the cryogen flow path.

The statistical indexes may include the following.

**Statistical analysis of the index**

$$\text{Index Mean or averaging value (expectation): } mean(X) = \frac{1}{W}\sum_{i \in W} X(i);$$

$$\text{Index Standard deviation: } STD(X) = \frac{1}{W-1}\sum_{i \in W-1}(X(i) - mean(X))$$

$$\text{Index Variation } \underline{Var\_a}(X) = \frac{mean(X)}{STD(X)}$$

$$\text{Index Variability } \underline{Var\_b}(X) = \frac{max(X - mean(X))}{mean(X)}$$

where X is the index, W is a window size used in the calculation.

The pressure indexes and ratios and the statistical indexes may be used as inputs 2402 in the model 2400. The model 2400 may determine relationships between the indexes and ratios and the outputs 2406 in order to accurately determine and/or predict a potential issue (e.g., blockage or leak) that may exist or be anticipated to occur in the cryoablation system 2200.

The model 2400 may use one or more layers 2404 to determine the outputs 2406. The outputs 2406 may be used as predictors to determine the performance of the cryoablation system. The outputs 2406 may include, for example, a pressure change rate, a singularity in the pressure change during pressurization of the cryogen flow path, shapes and slopes of the pressure and/or pressure change rate. These outputs may be determined for the various portions of the cryogen flow path so that problems can be determined and predicted in the cryoablation system 2200. The outputs 2406 may also determine an extent of the problem. For example, the outputs 2406 may allow the model 2400 to determine if a blockage is a complete blockage, partial blockage, is a growing blockage, and/or is clearing in the cryogen flow path. The model 2400 may similarly be able to determine an output 2406 that determines an extend of a leak.

The outputs 2406 may be used to determine a result 2408. The results 2408 may be actions that the model enables the diagnostic control 2240 to perform. The results 2408 may allow the diagnostics control 2240 to perform an expected or allowable pressure changing rates in the various portions of the cryogen flow path. This may allow the diagnostics control 2240 to control the operation of the cryoablation system 2200 to perform more efficiently and effectively, including reducing a time to perform a cryoablation treatment. The results 2408 may also include allowing the diagnostics control 2240 to determine whether the cryoablation system 2200 is performing as intended and where and extent of a problem such as a blockage or leak. Overall, the model 2400 may allow the diagnostics control 2240 to perform real-time and automatically controlled pressure diagnostics and control of the cryoablation system 2200.

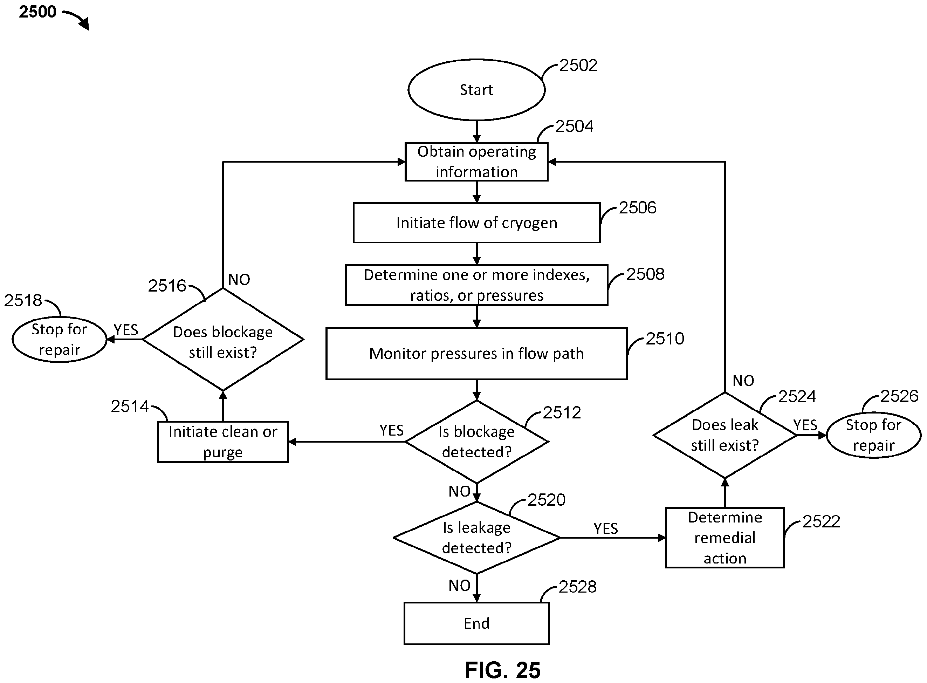

Referring now to FIG. 25, an example method 2500 of performing a diagnostics procedure is provided. The method 2500 may be performed using the cryoablation system 2200 previously described. In other examples, the method 2500 may be performed using other apparatuses or systems. The description below describes the method 2500 being performed using the cryoablation system 2200. It should be appreciated, however, that other apparatuses and/or systems may be used.

The method 2500 may start at step 2502. The method 2500 may be used to perform a diagnostics procedure on a cryoablation system. At step 2504, the diagnostics control 2240 may obtain information regarding the operating conditions of the cryoablation system 2200. Such information may be obtained, for example, from the pressure sensors positioned at various locations along the cryogen flow path.

At step 2506, the diagnostics control 2240 may cause cryogen to flow to the cryogen flow path and may begin recording the operating information (e.g., pressure information) over time. Step 2506 may be performed sequentially for a desired portion of the cryogen flow path so that each portion is verified to be operating properly before the next downstream valve is opened to check the next downstream portion.

At step 2508, the diagnostics control may be calculating one or more indexes, ratios and or otherwise extracting and calculating the operating pressures at different times during the pressurization and sequential verification of the cryogen flow path.

At step 2510, the diagnostics control 2240 may be monitoring the pressures in the cryogen flow path in real-time. The diagnostics control 2240 may also be determining statistical indexes as the pressure in the cryogen flow path is monitored and verified for each sequential portion of the cryogen flow path.

At step 2512, the diagnostics control 2240 may determine whether any blockages are detected at any portion along the cryogen flow path. The diagnostics control 2240 may compare the pressure change and/or pressure level and a time period to achieve the pressure level with a predetermined target pressure, target time period and tolerance ranges. If the diagnostics control 2240 determines that a blockage is present, the method 2500 may proceed to step 2514. If the diagnostics control 2240 determines that a blockage is not present, the method 2500 may proceed to step 2520.

At step 2514, the diagnostics control 2240 may initiate a cleaning or purge cycle to try and remove the blockage. The blockage may be due to moisture or ice in the cryogen flow path. The diagnostics control 2240 may cause a flow of heated gas or other fluid to be moved through the cryogen flow path in an attempt to clear the blockage. The cryoablation system 2200 may include a gas cryogen assembly 1814, such as that previously described, that may be used to purge or clean the cryogen flow path. In other examples, other purge or cleaning processes may be used.

At step 2516, the diagnostics control 2240 may re-perform a diagnostics procedure to determine whether the blockage still exists. If the diagnostics control 2240 determines that no blockage exists, the method 2500 may return to step 2504 to re-perform the steps as previously described to verify and check the cryogen flow path. If the diagnostics control 2240 determines that a blockage remains in the cryogen flow path, the method 2500 may proceed to step 2518 where the cryoablation system may be halted for further repair.

At step 2520, the diagnostics control 2240 may determine whether leakage exists in the cryogen flow path. The diagnostics control 2240 may determine whether a leak exists by comparing the pressure information to the pressure thresholds, pressure ranges, and/or time periods and tolerance ranges as previously described. The diagnostics control 2240 may use a machine learning model, such as model 2400, in some examples. If the diagnostics control 2240 determines that leak exists, the method 2500 may proceed to step 2522. If the diagnostics control 2240 determines that no leak exists, the method may proceed to step 2528.

At step 2522, the diagnostics control may determine a remedial action to try and correct the leak found at step 2520. The diagnostics control 2240 may recommend to check a particular connection, to replace the cryoprobe, and/or to re-connect a particular fitting or conduit. Such action may be recommended using the model 2400 that may be trained to identify potential fixes for leaks at various portions of the cryogen flow path.

The method 2500 may then proceed to step 2524 where the diagnostics control 2240 may re-check the particular portion of the cryogen flow path where it detected a leak. The diagnostics control 2240 may re-perform one or more steps of the method 2500 to pressurize and test the identified portion of the cryogen flow path. If a leak is still detected, the method 2500 may proceed to step 2526 where the system is halted for further repair or maintenance. If no leak is detected, the method 2500 may return to step 2504 where the method may be re-performed to check and verify a proper operation of the cryoablation system 2200.

At step 2518, the diagnostic control 2240 may have determined that there are not leaks or blockages. The method 2500 may end and the system may perform the cryoablation treatment as intended. If a problem arises or the cryoablation system 2200 is not performing, the method 2500 may be pre-performed to diagnose and possible repair or correct an issue that may exist with the cryoablation flow path.

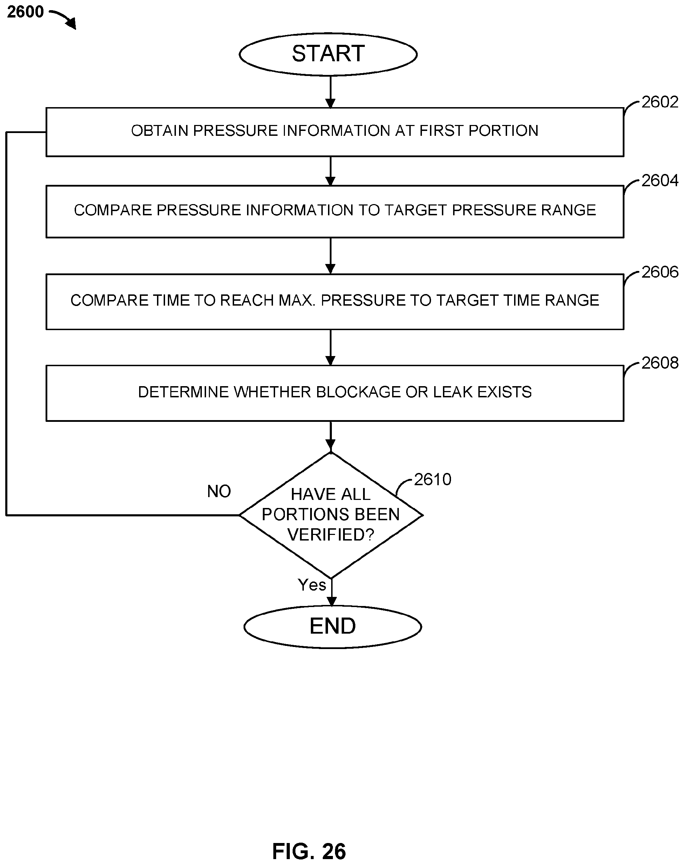

Referring now to FIG. 26, another method 2600 of performing a diagnostic procedure is provided. The method 2600 may be performed by one or more of the cryoablation systems and apparatuses of the present disclosure or variations thereof. In one example, the method 2600 may be performed by the cryoablation apparatus 2200. The description below described the method 2600 being performed by the cryoablation system 2200. It should be appreciated that the method 2600 is not limited to being performed by the cryoablation system 2200 and other systems and apparatuses may be used.

The method may begin at step 2602. At step 2602, the diagnostic control 2240 may obtain pressure information for a pressure of cryogen in a first portion of a cryogen flow path. The diagnostic control 2240 may obtain pressure information from the flow sensor 2208 that characterizes a pressure in the supply 2230. The flow valve 2210 may be closed at this point in the method 2600 so that the diagnostic control 2240 can obtain the pressure information in an isolated portion of the cryogen flow path, such as that portion of the supply 2230 upstream of the flow valve 2210. The diagnostics control 2240 may obtain and record the pressure information over time since the supply 2230 is pressurized and/or track or record a rate of change of the pressure over time. This information may be stored and/or displayed for later use. The method may proceed to step 2604.

At step 2604, the diagnostics control 2240 may compare the pressure in the first portion of the cryogen flow path to a target pressure range. The diagnostic control 2240 may obtain the target pressure range from a database, from experimental or clinical data, or as the target pressure may be recommended by a model, algorithm or other source. The method 2600 may then proceed to step 2606.

At step 2606, the diagnostics control 2240 may compare a time to reach a maximum pressure in the first portion of the cryogen flow path to a target time range. As previously discussed, the diagnostics control 2240 may record the pressure over time. When the pressure in the first portion reaches a maximum value, the time to reach such maximum pressure may be recorded. This time period may be compared to a target time range. The target time range may be obtained from a database, from experimental or clinical data, or as the target pressure may be recommended by a model, an algorithm or other source. The method 2600 may then proceed to step 2608.

At step 2608, the diagnostics control 2240 may send a flow alert if the diagnostics control 2240 determines that a flow issue exists. The diagnostics control 2240 may determine that a flow issue exists if the pressure in the first portion of the cryogen flow path is outside the target pressure range. The diagnostics control 2240 may determine that a flow issue exists if the time to reach a maximum pressure in the first portion of the cryogen flow path takes longer or shorter than the time indicated by the target time range. If the time takes too long, a leak may exist, for example. If the time takes too short, a blockage may exist, for example. In order for proper or intended operation to be confirmed, the pressure should be in the target pressure range and the time should be in the target time range.

The diagnostics control 2240 may send a flow alert if an issue is detected. The flow alert may be a message, audible signal, warning light, email, text message, alert display on a screen of the cryoablation system 2200, or other suitable indicator or a combination of the above.

While not shown in FIG. 26, if a flow issue is detected, the diagnostics control 2240 may perform a remedial action in the attempt to correct or fix the flow issue. The diagnostics control 2240 may, for example, cause a purge of the cryogen flow system to be performed. In other instances, the diagnostics control 2240 may send a recommended action to a user such as to re-connect an element or replace an element of the cryoablation apparatus 2200.

The method 2600 may proceed to step 2610. At step 2610, the diagnostics control 2240 may determine whether all portions of the cryogen flow path have been verified. If all portions have the verified, the method 2600 may end. If all portions have not been verified, the method 2600 may return to step 2602 and re-perform the steps 2602 to 2610 as previously described.

For example, the method 2600 may be re-performed sequentially to text second, third, fourth, fifth, or other portions of the cryogen flow path. The method 2600 may be performed sequentially so that subsequent downstream portions of the cryogen flow path can be tested to verify the proper operation and integrity of the cryogen flow path. To perform verification of subsequent portions, the diagnostics control 2240 may open a valve downstream of the previously verified portion and pressurize the downstream portion. This process may be repeated sequentially to pressurize adjacent downstream portions and test the portions for flow issues.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The following is a list of non-limiting illustrative embodiments disclosed herein:

Illustrative embodiment 1: A cryoablation apparatus comprising: a Dewar configured to retain a volume of cryogen; a cryogen flow path fluidly coupled to the Dewar to supply the cryogen to a cryoprobe; a first pressure sensor and a second pressure sensor positioned at a first portion of the cryogen flow path and at a second portion of the cryogen flow path, respectively; a first valve and a second valve each positioned along the cryogen flow path, each configured to operate in an open position to allow the flow of cryogen and in a closed position to prevent the flow cryogen; and a diagnostics control operatively coupled to the first pressure sensor, the second pressure sensor, the first valve, and the second valve, the diagnostics control configured to: determine whether a blockage or leak exists in the first portion and the second portion of the cryogen flow path.

Illustrative embodiment 2: The cryoablation apparatus of illustrative embodiment 1, wherein: the second portion of the cryogen flow path is positioned downstream of the first portion; and the diagnostics control is configured to determine whether a blockage or leak exists in the first portion before the second portion.

Illustrative embodiment 3: The cryoablation apparatus of any one of the preceding illustrative embodiments, wherein the first portion and the second portion are positioned along a supply line of the cryogen flow path.

Illustrative embodiment 4: The cryoablation apparatus of illustrative embodiment 3, further comprising a return line fluidly coupled to the cryoprobe and to the supply line.

Illustrative embodiment 5: The cryoablation apparatus of any one of the illustrative embodiment 4, further comprising a first connector valve fluidly connecting the supply line to the return line at a position upstream of the first valve.

Illustrative embodiment 6: The cryoablation apparatus of illustrative embodiment 5, further comprising a second connector valve fluidly connecting the supply line to the return line at a position downstream of the first valve and upstream of the second valve.

Illustrative embodiment 7: The cryoablation apparatus of any one of illustrative embodiments 4 to 6, wherein the return line is fluidly coupled to a vent allowing pressure in the return to be vented from the cryogen flow path.

Illustrative embodiment 8: The cryoablation apparatus of any one of the preceding illustrative embodiments, wherein the diagnostics control determines whether a blockage or leak exists by comparing a pressure to a target pressure range and comparing a time to reach maximum pressure to target time range.

Illustrative embodiment 9: The cryoablation apparatus of any one of the preceding illustrative embodiments, wherein the diagnostics control determines whether a blockage or leak exists using a trained machine learning model.

Illustrative embodiment 10: The cryoablation apparatus of any one of the preceding illustrative embodiments, wherein the cryogen is Nitrogen.

Illustrative embodiment 11: The cryoablation apparatus of any one of the preceding illustrative embodiments, wherein the diagnostics control is configured to operate the first valve in the open position and the second valve in the closed position to determine whether a blockage or leak exists in the first portion before the diagnostics control operates the first valve in the open position and the second valve in the open position to determine whether a blockage or leak exists in the second portion.

Illustrative embodiment 12: The cryoablation apparatus of any one of the preceding illustrative embodiments, wherein the diagnostics control is further configured to cause a purge of the cryogen flow path to be performed when it determines a blockage exists.

Illustrative embodiment 13: The cryoablation apparatus of any one of the preceding illustrative embodiments, further comprising: a housing configured to retain a second volume of liquid cryogen; a heater positioned in the housing and configured to heat the liquid cryogen to convert the liquid cryogen to cryogen gas; and a flow conduit comprising a conduit heater, the flow conduit fluidly connected to the housing and configured to fluidly connect the housing to the cryogen flow.

Illustrative embodiment 14: The cryoablation apparatus of illustrative embodiment 13, further comprising a connector configured to fluidly connect the housing to the Dewar.

Illustrative embodiment 15: The cryoablation apparatus of any one of the illustrative embodiments 13 or 14, wherein the housing is positioned inside the Dewar.

Illustrative embodiment 16: The cryoablation apparatus of any one of the illustrative embodiments 13 to 15, wherein the flow conduit is positioned at a top of the housing to dispense cryogen gas from the housing to the cryogen flow path.

Illustrative embodiment 17: The cryoablation apparatus any one of the illustrative embodiments 13 to 16, further comprising a gas control coupled to the heater and the conduit heater, the gas control configured to: activate the heater to achieve a predetermined pressure in the housing; and activate the conduit heater to achieve a predetermined temperature of cryogen gas.

Illustrative embodiment 18: The cryoablation apparatus of any one of the illustrative embodiments 13 to 17, wherein the gas control is further configured to dispense the cryogen gas to the cryogen flow path to perform the purge of the cryogen flow path.

Illustrative embodiment 19: A method of diagnosing cryogen flow in a cryogen flow path comprising: obtaining pressure information for a pressure of cryogen in a first portion of the cryogen flow path; comparing the pressure information to a target pressure range; and comparing a time to reach a maximum pressure in the first portion of the cryogen flow path to a target time range; and sending a flow alert if a flow issue is detected based on the pressure information and the time to reach the maximum pressure.

Illustrative embodiment 20: The method of illustrative embodiment 19, further comprising opening a first valve to allow cryogen to flow to the first portion of the cryogen flow path.

Illustrative embodiment 21: The method of any one of the illustrative embodiments 19 or 20, further comprising: opening a second valve to allow cryogen to flow to a second portion of the cryogen flow path, wherein the second portion of the cryogen flow path is downstream of the first portion; obtaining pressure information for a pressure of cryogen in the second portion of the cryogen flow path; comparing the pressure information to a second target pressure range; and comparing a second time to reach a maximum pressure in the second portion of the cryogen flow path to a second target time range; and sending a flow alert if a flow issue is detected based on the pressure information in the second portion of the cryogen flow path and the second time to reach the maximum pressure.

Illustrative embodiment 22: The method of any one of the illustrative embodiments 19 to 21, further comprising venting pressure from the cryogen flow path if a flow issue is detected.

Illustrative embodiment 23: The method of illustrative embodiment 22, wherein pressure is vented by opening a connector valve that fluidly connects the first portion of the cryogen flow path to a return line of the cryogen flow path.

Illustrative embodiment 24: The method of any one of the illustrative embodiments 19 to 23, further comprising: moving a portion of a volume of liquid cryogen from a Dewar to a housing of a cryogen gas apparatus; heating the portion of the volume of liquid cryogen to form cryogen gas in the housing; and moving the cryogen gas from the housing into the cryogen flow path to purge the cryogen flow path.

Illustrative embodiment 25: The method of illustrative embodiment 24, wherein the housing defines an internal cavity inside the Dewar.

Illustrative embodiment 26: The method of any one of the illustrative embodiments 24 or 25, wherein the housing is positioned in the Dewar.

## Claims

1. A cryoablation apparatus (2200) comprising:

   a Dewar (2202) configured to retain a volume of cryogen, preferably nitrogen;
   a cryogen flow path fluidly coupled to the Dewar to supply the cryogen to a cryoprobe;
   a first pressure sensor (2208) and a second pressure sensor (2212) positioned at a first portion of the cryogen flow path and at a second portion of the cryogen flow path, respectively;
   a first valve (2218) and a second valve (2220) each positioned along the cryogen flow path, each configured to operate in an open position to allow the flow of cryogen and in a closed position to prevent the flow cryogen; and
   a diagnostics control (2240) operatively coupled to the first pressure sensor, the second pressure sensor, the first valve, and the second valve, the diagnostics control configured to:
   determine whether a blockage or leak exists in the first portion and the second portion of the cryogen flow path, optionally wherein:

      the second portion of the cryogen flow path is positioned downstream of the first portion; and
      the diagnostics control is configured to determine whether a blockage or leak exists in the first portion before the second portion; wherein preferably the diagnostics control is further comfigured to cuae a purge of the cryogen flow path to be performed when it determines a blockage exists.

2. The cryoablation apparatus of claim 1, wherein the first portion and the second portion are positioned along a supply line of the cryogen flow path, optionally wherein the apparatus further comprises a return line fluidly coupled to the cryoprobe and to the supply line.

3. The cryoablation apparatus of claim 2, further comprising a first connector valve fluidly connecting the supply line to the return line at a position upstream of the first valve, and optionally further comprising a second connector valve fluidly connecting the supply line to the return line at a position downstream of the first valve and upstream of the second valve, preferably wherein the return line is fluidly coupled to a vent allowing pressure in the return to be vented from the cryogen flow path.

4. The cryoablation apparatus of any preceding claim, wherein the diagnostics control determines whether a blockage or leak exists by comparing a pressure to a target pressure range and comparing a time to reach maximum pressure to target time range, preferably by using a trained machine learning model.

5. The cryoablation apparatus of any preceding claim, wherein the diagnostics control is configured to operate the first valve in the open position and the second valve in the closed position to determine whether a blockage or leak exists in the first portion before the diagnostics control operates the first valve in the open position and the second valve in the open position to determine whether a blockage or leak exists in the second portion.

6. The cryoablation apparatus of any preceding claim, further comprising:

   a housing configured to retain a second volume of liquid cryogen;
   a heater positioned in the housing and configured to heat the liquid cryogen to convert the liquid cryogen to cryogen gas; and
   a flow conduit comprising a conduit heater, the flow conduit fluidly connected to the housing and configured to fluidly connect the housing to the cryogen flow.

7. The cryoablation apparatus of claim 6, further comprising a connector configured to fluidly connect the housing to the Dewar, optionally wherein the housing is positioned inside the Dewar.

8. The cryoablation apparatus of claim 6 or claim 7, wherein the flow conduit is positioned at a top of the housing to dispense cryogen gas from the housing to the cryogen flow path.

9. The cryoablation apparatus of any of claims 6 to 8, further comprising a gas control coupled to the heater and the conduit heater, the gas control configured to:

   activate the heater to achieve a predetermined pressure in the housing; and
   activate the conduit heater to achieve a predetermined temperature of cryogen gas, preferably wherein the gas control is further configured to dispense the cryogen gas to the cryogen flow path to perform the purge of the cryogen flow path.

10. A method of diagnosing cryogen flow in a cryogen flow path comprising:

    obtaining pressure information for a pressure of cryogen in a first portion of the cryogen flow path;
    comparing the pressure information to a target pressure range; and
    comparing a time to reach a maximum pressure in the first portion of the cryogen flow path to a target time range; and
    sending a flow alert if a flow issue is detected based on the pressure information and the time to reach the maximum pressure, optionally further comprising opening a first valve to allow cryogen to flow to the first portion of the cryogen flow path.

11. The method of claim 10, further comprising:

    opening a second valve to allow cryogen to flow to a second portion of the cryogen flow path, wherein the second portion of the cryogen flow path is downstream of the first portion;
    obtaining pressure information for a pressure of cryogen in the second portion of the cryogen flow path;
    comparing the pressure information to a second target pressure range; and
    comparing a second time to reach a maximum pressure in the second portion of the cryogen flow path to a second target time range; and
    sending a flow alert if a flow issue is detected based on the pressure information in the second portion of the cryogen flow path and the second time to reach the maximum pressure.

12. The method of claim 9 or claim 10, further comprising venting pressure from the cryogen flow path if a flow issue is detected.

13. The method of claim 12, wherein pressure is vented by opening a connector valve that fluidly connects the first portion of the cryogen flow path to a return line of the cryogen flow path.

14. The method of any of claims 10 to 13, further comprising:

    moving a portion of a volume of liquid cryogen from a Dewar to a housing of a cryogen gas apparatus;
    heating the portion of the volume of liquid cryogen to form cryogen gas in the housing; and
    moving the cryogen gas from the housing into the cryogen flow path to purge the cryogen flow path.

**15.** The method of claim 14, wherein the housing defines an internal cavity inside the Dewar, and preferably wherein the housing is positioned in the Dewar.

**FIG. 1**

EP 4 527 326 A1

FIG. 3A

FIG. 3B

FIG. 2

**FIG. 4**

EP 4 527 326 A1

502

T0          T1          T2          T3

Charging signal
reset control for

504

Rod capacitance
based charging
voltage output

506

Voltage A2D conversion
timing control for
capacitance calculation

508

Liquid nitrogen level
calculation %

95%
94%
91%
90%

**FIG. 5**

FIG. 6

EP 4 527 326 A1

**700**

Initialize
Apparatus 702

Obtain operational information 704

Adjust, tune, or set
operational parameters 706

Obtain capacitance information 708

Adjust operating
parameters 710

Determine cryogen liquid level 712

Is cryogen liquid level in
predetermined range? 714

Adjust
operating
parameters 716

YES

NO

Is there enough cryogen
to complete procedure? 718

YES

NO

Indicate cryogen refill needed 720

Is refill adequate? 722

YES

Re-start
cryo-cycle 724

NO

End 726

**FIG. 7**

800 ⤵

```
        ┌─────────────┐
        │    START    │
        └──────┬──────┘
               │
               ▼
┌──────────────────────────────────────┐ ⌐802
│      OBTAIN OPERATING PARAMETERS       │
└──────────────────┬───────────────────┘
                   │
                   ▼
┌──────────────────────────────────────┐ ⌐804
│       OBTAIN DEWAR CAPACITANCE         │
└──────────────────┬───────────────────┘
                   │
                   ▼
┌──────────────────────────────────────┐ ⌐806
│      DETERMINE CRYOGEN LIQUID LEVEL    │
└──────────────────┬───────────────────┘
                   │
                   ▼
┌──────────────────────────────────────┐ ⌐808
│  SEND CRYOGEN LEVEL WARNING IF CRYOGEN │
│   LIQUID LEVEL IS LESS THAN            │
│       PREDETERMINED LEVEL              │
└──────────────────┬───────────────────┘
                   │
                   ▼
        ┌─────────────┐
        │     END     │
        └─────────────┘
```

FIG. 8

EP 4 527 326 A1

**900**

```
912                          910            902
┌──────────┐                 │        ┌──────────────┐
│  Tx/Rx   │                 │        │ Processor(s) │
│  Device  │─────────────────┤        └──────────────┘
└──────────┘                 │
                             │        904
914                 Data Bus(es)      ┌──────────────┐
┌──────────────┐             │        │   Working    │
│Communication │─────────────┤        │   Memory     │
│   Port(s)    │             │        └──────────────┘
└──────────────┘             │
                             │        906
916                          │        ┌──────────────┐
┌──────────────┐             │        │ I/O Device(s)│
│   Display    │─────────────┤        └──────────────┘
│              │             │
│  918         │             │        908
│  ┌────────┐  │             │        ┌──────────────┐
│  │  User  │  │             └────────│ Instruction  │
│  │Interface│ │                      │   Memory     │
│  └────────┘  │                      └──────────────┘
└──────────────┘
```

**FIG. 9**

**FIG. 10**

**FIG. 11**

**FIG. 12**

FIG. 13

**FIG. 14**

**FIG. 15**

EP 4 527 326 A1

**FIG. 16**

**1700**

FIG. 17

1800

1820

1810

1814

1808

1816

1804

1802

1806

**FIG. 18**

FIG. 19

**FIG. 20**

2100 ⟍

START

MOVING A PORTION OF CRYOGEN FROM DEWAR TO HOUSING — 2102

HEATING PORTION OF CRYOGEN TO FORM CRYOGEN GAS — 2104

OBTAIN CRYOGEN GAS INFORMATION — 2106

MOVE CRYOGEN GAS FROM THE HOUSING THROUGH CRYOGEN FLOW PATH — 2108

END

FIG. 21

FIG. 22

**FIG. 23**

FIG. 24

**2500**

Start — 2502

Obtain operating information — 2504

Initiate flow of cryogen — 2506

Determine one or more indexes, ratios, or pressures — 2508

Monitor pressures in flow path — 2510

2512 — Is blockage detected?

YES → Initiate clean or purge — 2514

2516 — Does blockage still exist? — NO → Obtain operating information

YES → Stop for repair — 2518

NO → 2520 — Is leakage detected?

YES → Determine remedial action — 2522

2524 — Does leak still exist? — NO → Obtain operating information

YES → Stop for repair — 2526

NO → End — 2528

**FIG. 25**

EP 4 527 326 A1

61

**2600**

```
            ┌─────────────┐
            │    START    │
            └─────────────┘
                   │
                   ▼
```
OBTAIN PRESSURE INFORMATION AT FIRST PORTION    —2602

COMPARE PRESSURE INFORMATION TO TARGET PRESSURE RANGE    —2604

COMPARE TIME TO REACH MAX. PRESSURE TO TARGET TIME RANGE    —2606

DETERMINE WHETHER BLOCKAGE OR LEAK EXISTS    —2608

NO ◄── HAVE ALL PORTIONS BEEN VERIFIED?    —2610

Yes

```
            ┌─────────────┐
            │     END     │
            └─────────────┘
```

**FIG. 26**

# EP 4 527 326 A1

**EUROPEAN SEARCH REPORT**

Application Number

EP 24 19 9491

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/207426 A1 (BIOCOMPATIBLES UK LTD [GB]) 31 October 2019 (2019-10-31) | 1-5, 10-15 | INV.<br>A61B18/02 |
| Y | * paragraphs [0010], [0039] - [0041], [0050], [0052] - [0054], [0080], [0084], [0106], [0107], [0112], [0122]; figures 1-5, 7, 9 * | 6-9 | A61B18/00<br>A61B1/015<br>A61B90/00 |
| Y | US 2012/059364 A1 (BAUST JOHN M [US] ET AL) 8 March 2012 (2012-03-08) * paragraphs [0057], [0058], [0091], [0098] - [0103]; figures 1, 17 * | 6-9 | |
| X | US 2023/190355 A1 (WANG SHI [CN] ET AL) 22 June 2023 (2023-06-22) * paragraphs [0065], [0067], [0075], [0083], [0171], [0194], [0198]; figures 2, 3a * | 1-5, 10-15 | |
| X | US 2023/165619 A1 (MA WING-CHOI [US]) 1 June 2023 (2023-06-01) * paragraphs [0039], [0040], [0041], [0045], [0046]; figures 1-5 * | 1-5, 10-15 | TECHNICAL FIELDS SEARCHED (IPC) |
| X | US 2013/211394 A1 (FISCHER GERALD [AT] ET AL) 15 August 2013 (2013-08-15) * paragraphs [0039], [0040], [0041], [0090], [0095], [0100], [0108], [0109], [0110], [0120]; figure 1 * | 1-5, 10-15 | A61B |
| X | EP 2 218 418 A2 (MEDTRONIC CRYOCATH LP [CA]) 18 August 2010 (2010-08-18) * paragraphs [0019], [0021]; figure 2 * | 1-5, 10-15 | |
| A | WO 2021/108894 A1 (MEDTRONIC CRYOCATH LP [CA]) 10 June 2021 (2021-06-10) * paragraphs [0044], [0047], [0048], [0052] - [0054]; figures 2, 3 * | 1-15 | |

The present search report has been drawn up for all claims

| Place of search | Date of completion of the search | Examiner |
|---|---|---|
| The Hague | 6 February 2025 | Bois, Nadège |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

63

## EP 4 527 326 A1

### ANNEX TO THE EUROPEAN SEARCH REPORT
### ON EUROPEAN PATENT APPLICATION NO.

EP 24 19 9491

This annex lists the patent family members relating to the patent documents cited in  the above-mentioned European search report.
The members are as contained in the European Patent Office EDP file on
The European Patent Office is in no way liable for these particulars which are merely given for the purpose of information.

06-02-2025

| Patent document cited in search report | | Publication date | Patent family member(s) | | Publication date |
|---|---|---|---|---|---|
| WO 2019207426 | A1 | 31-10-2019 | AU | 2019259501 A1 | 24-09-2020 |
| | | | AU | 2022202915 A1 | 26-05-2022 |
| | | | CN | 112020337 A | 01-12-2020 |
| | | | EP | 3784156 A1 | 03-03-2021 |
| | | | JP | 7146935 B2 | 04-10-2022 |
| | | | JP | 7441910 B2 | 01-03-2024 |
| | | | JP | 2021517050 A | 15-07-2021 |
| | | | JP | 2022171938 A | 11-11-2022 |
| | | | TW | 201944967 A | 01-12-2019 |
| | | | US | 2019328437 A1 | 31-10-2019 |
| | | | US | 2022183737 A1 | 16-06-2022 |
| | | | WO | 2019207426 A1 | 31-10-2019 |
| US 2012059364 | A1 | 08-03-2012 | BR | 112013022378 A2 | 06-12-2016 |
| | | | CA | 2829058 A1 | 07-09-2012 |
| | | | CL | 2013002518 A1 | 25-07-2014 |
| | | | CN | 103501719 A | 08-01-2014 |
| | | | CN | 106214244 A | 14-12-2016 |
| | | | EP | 2680775 A1 | 08-01-2014 |
| | | | EP | 3443919 A1 | 20-02-2019 |
| | | | IL | 228223 A | 29-10-2015 |
| | | | KR | 20140022819 A | 25-02-2014 |
| | | | MX | 340642 B | 19-07-2016 |
| | | | SG | 193263 A1 | 30-10-2013 |
| | | | US | 2012059364 A1 | 08-03-2012 |
| | | | US | 2016022345 A1 | 28-01-2016 |
| | | | WO | 2012119088 A1 | 07-09-2012 |
| US 2023190355 | A1 | 22-06-2023 | CN | 113576649 A | 02-11-2021 |
| | | | CN | 115363737 A | 22-11-2022 |
| | | | CN | 115363738 A | 22-11-2022 |
| | | | CN | 115568932 A | 06-01-2023 |
| | | | EP | 4144314 A1 | 08-03-2023 |
| | | | US | 2023190355 A1 | 22-06-2023 |
| | | | WO | 2023001215 A1 | 26-01-2023 |
| US 2023165619 | A1 | 01-06-2023 | EP | 4190255 A1 | 07-06-2023 |
| | | | US | 2023165619 A1 | 01-06-2023 |
| US 2013211394 | A1 | 15-08-2013 | EP | 2575657 A2 | 10-04-2013 |
| | | | EP | 3281594 A1 | 14-02-2018 |
| | | | US | 2013211394 A1 | 15-08-2013 |
| | | | US | 2017196616 A1 | 13-07-2017 |
| | | | WO | 2011151354 A2 | 08-12-2011 |
| EP 2218418 | A2 | 18-08-2010 | AT | E478620 T1 | 15-09-2010 |

EPO FORM P0459

For more details about this annex : see Official Journal of the European Patent Office, No. 12/82

**ANNEX TO THE EUROPEAN SEARCH REPORT**
**ON EUROPEAN PATENT APPLICATION NO.**                EP 24 19 9491

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2025

| Patent document cited in search report | Publication date | Patent family member(s) | | Publication date |
|---|---|---|---|---|
| | | AT | E537773 T1 | 15-01-2012 |
| | | CA | 2598248 A1 | 14-09-2006 |
| | | EP | 1871258 A1 | 02-01-2008 |
| | | EP | 2218418 A2 | 18-08-2010 |
| | | US | 2006212026 A1 | 21-09-2006 |
| | | US | 2008221508 A1 | 11-09-2008 |
| | | US | 2012271291 A1 | 25-10-2012 |
| | | US | 2015223859 A1 | 13-08-2015 |
| | | US | 2016367306 A1 | 22-12-2016 |
| | | WO | 2006096272 A1 | 14-09-2006 |
| WO 2021108894 A1 | 10-06-2021 | CN | 114746037 A | 12-07-2022 |
| | | EP | 4069111 A1 | 12-10-2022 |
| | | US | 2021169545 A1 | 10-06-2021 |
| | | WO | 2021108894 A1 | 10-06-2021 |

EPO FORM P0459

For more details about this annex : see Official Journal of the European Patent Office, No. 12/82